# FASCICULE DE BREVET EUROPEEN

(11) **EP 1 259 541 B1**
(45) Date de publication et mention de la délivrance du brevet: **05.09.2007**
(21) Numéro de dépôt: 01911821.5
(22) Date de dépôt: 01.03.2001
(51) Int. Cl.: C07K 14/47

(54) **SEQUENCES D'ACIDES AMINES DERIVEES DES PROTEINES HUMAINES REAGISSANT AVEC L'HEPARINE FACILITANT LE TRANSFERT DE SUBSTANCES D'INTERET A L'INTERIEUR DES CELLULES ET/OU DES NOYAUX CELLULAIRES**
VON HUMANEN PROTEINEN, WELCHE MIT HEPARIN REAGIEREN, ABGELEITETE AMINOSÄURESEQUENZEN, DIE DEN TRANSFER VON SUBSTANZEN VON INTERESSE IN DAS INNERE VON ZELLEN UND/ODER ZELLKERNEN ERLEICHTERN
AMINO ACID SEQUENCES FACILITATING PENETRATION OF A SUBSTANCE OF INTEREST INTO CELLS AND/OR CELL NUCLEI

(30) Priorité: 01.03.2000 FR 0002621
(43) Date de publication de la demande: 27.11.2002
(60) Demande divisionnaire: 04292771.5
(73) Titulaire: Diatos S.A., 75014 Paris (FR)
(72) Inventeur: AVRAMEAS, Eustrate, F-75015 Paris (FR); TERNYNCK, Thérèse, F-75015 Paris (FR)
(74) Mandataire: Breese, Pierre
(86) Numéro de dépôt international: PCT/FR2001/000613
(87) Numéro de publication internationale: WO 2001/064738

(56) Documents cités:
- WO-A-91/04315
- WO-A-96/08274
- WO-A-98/56938
- WO-A-99/07414
- WEISGRABER, K.H. & RALL, S.C. JR.: "Human Apolipoprotein B-100 Heparin-binding Sites" JOURNAL OF BIOLOGICAL CHEMISTRY, vol. 262, no. 23, 15 août 1987 (1987-08-15), pages 11097-11103, XP002186291
- DATABASE MEDLINE [en ligne] avril 1999 (1999-04) OHTA, H. ET AL.: "Internalization of human extracellular-superoxide dismutase by bovine aortic endothelial cells" Database accession no. 94274140 XP002186296
- MESRI, E.A. ET AL.: "The heparin-binding domain of heparin-binding EGF-like growth factor can target Pseudomonas exotoxin to kill cells exclusively through heparan sulfate proteoglycans" JOURNAL OF CELL SCIENCE, vol. 107, no. 9, septembre 1994 (1994-09), pages 2599-2608, XP002186292
- XU, G. ET AL.: "Interaction of heparin with synthetic peptides corresponding to the C-terminal domain of intestinal mucins" GLYCOCONJUGATE JOURNAL, vol. 13, no. 1, février 1996 (1996-02), pages 81-90, XP001042193 cité dans la demande
- AMARA, A. ET AL.: "Stromal Cell-derived Factor-1 alpha Associates with Heparan Sulfates through the First beta-Strand of the Chemokine" JOURNAL OF BIOLOGICAL CHEMISTRY, vol. 274, no. 34, 20 août 1999 (1999-08-20), pages 23916-23925, XP002186293 cité dans la demande
- OLOFSSON, A.M. ET AL.: "Heparin-binding protein targeted to mitochondrial compartments protects endothelial cells from apoptosis" JOURNAL OF CLINICAL INVESTIGATION, vol. 104, no. 7, octobre 1999 (1999-10), pages 885-894, XP002186294 -& DATABASE EMBL, HEIDELBERG, RFA [en ligne] 1 février 1991 (1991-02-01) POHL, J. ET AL.: "AZUROCIDIN PRECURSOR (CATIONIC ANTIMICROBIAL PROTEIN CAP37) (HEPARIN-BINDING PROTEIN) (HBP)" retrieved from HOMO SAPIENS Database accession no. P20160 XP002186297
- MARGALIT, H. ET AL.: "Comparative Analysis of Structurally Defined Heparin Binding Sequences Reveals a Distinct Spatial Distribution of Basic Residues" JOURNAL OF BIOLOGICAL CHEMISTRY, vol. 268, no. 26, 15 septembre 1993 (1993-09-15), pages 19228-19231, XP002186295

## Description

La présente invention a pour objet une séquence d'acides aminés présentant la capacité de faciliter la pénétration d'une substance d'intérêt à l'intérieur des cellules et/ou des noyaux cellulaires.

Disposer d'outils permettant de transférer efficacement des substances d'intérêt du milieu extérieur vers l'intérieur des cellules, et plus particulièrement des noyaux cellulaires, est un atout considérable dans le domaine des biotechnologies, et tout particulièrement pour produire des protéines, ou des peptides, pour réguler l'expression de gènes, ou pour analyser et cribler les voies de signalisation intracellulaires, ou encore pour analyser des propriétés d'une substance donnée sur ladite cellule.

Une autre application importante de tels outils touche au domaine de la thérapie génique puisque, jusqu'à présent, les divers procédés de thérapie génique se heurtent au même besoin, non satisfait de manière optimale, qui est de pouvoir disposer de vecteurs capables de transférer dans le cytoplasme et/ou le noyau des cellules de l'organisme de l'hôte à traiter des principes biologiquement actifs, sans pour autant altérer ni le génome de l'hôte, ni les propriétés biologiques desdits principes actifs transférés.

Plusieurs techniques ont jusqu'à présent été développées pour transférer de l'ADN dans les cellules, mais aucune n'est réellement satisfaisante. L'une d'entre elles, dérivant de la technique de Mandel et Higa, basée sur l'acquisition par E. coli de la susceptibilité à la transformation par traitement au CaCl₂ consiste à coprécipiter l'ADN avec le phosphate de calcium ou le DEA-dextran, et à introduire le précipité obtenu directement dans la cellule ou le noyau. Cette méthode, outre le fait qu'elle soit toxique, n'est pas sélective.

Une autre méthode d'introduction directe de l'ADN, l'électroporation, consiste à soumettre les cellules à un bref choc électrique de quelques milliers de volts, ce qui permet à l'ADN de passer la membrane cytoplasmique et de s'introduire dans la cellule. Cette méthode est très toxique pour les cellules, entraînant une forte mortalité et une grande variabilité selon les cellules utilisées.

D'autres méthodes utilisent un criblage de l'entrée du gène dans les cellules par des récepteurs présents sur leur membrane. L'ADN peut alors pénétrer dans la cellule par l'intermédiaire soit d'un ligand spécifique de ces récepteurs, soit d'anticorps spécifiques de constituants membranaires. Le complexe ADN-ligand pénètre ainsi dans la cellule par un processus d'endocytose. Ce procédé se trouve limité du fait qu'il y a une destruction importante du complexe utilisé dans les vésicules lysosomales. Plusieurs procédés ont été développés pour pallier ces inconvénients, mais aucun ne donne entière satisfaction.

Le brevet US n° 5 635 383 décrit quant à lui un autre type de vecteur complexe, à base de polylysine, pour le transfert d'acides nucléiques dans les cellules.

Le brevet US n° 5 521 291 décrit une autre méthode basée sur l'utilisation d'un conjugué formé d'un virus lié à une substance ayant une forte affinité pour l'ADN par l'intermédiaire d'un anticorps. De tels conjugués sont d'une utilisation lourde, et certains risques sont liés à l'utilisation des virus.

Pour tenter de pallier ces inconvénients, il a été décrit, dans la demande de brevet n° WO 97/02840, un procédé mis en oeuvre *in vitro* qui consiste à utiliser des anticorps anti-ADN murins ou leurs fragments F(ab')₂ et Fab' capables de pénétrer à l'intérieur des cellules vivantes, comme immunovecteurs pour le transfert intracytoplasmique, et/ou intranucléaire de substances biologiquement actives. Bien que ces vecteurs présentent une efficacité importante, leur utilisation peut être complexe dans certaines applications. En outre, l'utilisation de molécules de la taille et de la complexité des anticorps peut représenter un inconvénient important dans leur manipulation et mise en oeuvre.

Dans la demande de brevet n° WO 99/07414, il a été décrit qu'il était possible d'utiliser *in vitro* des peptides dérivés des anticorps murins anti-ADN, divulgués dans la demande WO 97/02840 précitée, comme vecteurs d'internalisation intracytoplasmique et intranucléaire de substances biologiquement actives.

Bien que ces vecteurs peptidiques d'origine murine soient codés par la lignée germinale et ne portent pas de mutation, et par conséquent, devraient être antigéniquement proches de ceux rencontrés chez l'homme, le risque d'une réaction immunitaire chez l'homme ne peut être exclu.

La demande WO 98/56938 décrit l'utilisation de lipoproteines, en particulier la lipoprotéine B, dans le transfert in vivo d'acides nucléiques.

Il existe donc un réel besoin en peptides et séquences d'acides aminés palliant les inconvénients décrits plus haut, à savoir susceptibles d'être utilisés comme, ou dans un, vecteur d'internalisation cellulaire chez l'homme et qui ne présenteraient aucun des risques mentionnés plus haut.

Il est connu qu'un très grand nombre de régulations cellulaires dépendent des interactions entre les protéines et les glycosaminoglycanes (GAG) de la surface des cellules (**1-6**) [les chiffres en gras, entre parenthèses, renvoient à la liste de références bibliographiques annexée]. De telles interactions interviennent, par exemple, dans le contrôle de l'hémostase (**7**), dans la prolifération des cellules musculaires lisses (**8**), dans l'expression de l'activité des facteurs de croissance (**9**), dans l'expression de l'activité lipolytique des enzymes (**10**), dans l'intégrité de la matrice extracellulaire (**11**) et autres. Dans un système donné, ces effets biologiques sont dus à l'interaction d'une ou d'un nombre restreint de protéines avec les GAG de la surface cellulaire. Les GAG sont des constituants très conservés au cours de l'évolution, présents sur la surface de toutes les cellules eucaryotes. Les GAG sont des polymères de saccharide ; par exemple l'héparine est un polymère du disaccharide (α-1, 4-2-iduronique acide → D-glucosamine et les chondroïtines sulfates sont des polymères du disaccharide N-acétyl chondrosine, contenant un grand nombre de groupements sulfate et donc négativement chargés. Les protéines qui réagissent spécifiquement avec les GAG contiennent toutes, dans leur séquence, un ou plusieurs segments peptidiques qui sont responsables de l'interaction de ces protéines avec les GAG. La longueur de ces segments varie d'une protéine à l'autre allant de quatre à trente acides aminés. Presque tous ces peptides sont positivement chargés et contiennent un nombre élevé d'acides aminés basiques, surtout de la lysine et de l'arginine. Il a été montré que ces acides aminés participent de façon prépondérante à l'interaction de ces protéines avec les GAG (**1-6**).

Les peptides se liant aux GAG ou plus généralement aux aminoglycanes, et en particulier à l'héparine, à l'héparane sulfate et aux chondroïtines sulfates (peptides globalement désignés par PLH, pour "peptide se liant à l'héparine", même si l'héparine n'est qu'un exemple d'aminoglycane) peuvent être d'origine naturelle, comme les peptides décrits plus haut, ou artificielle. Ils peuvent être utilisés sous leur forme native ou polymère (dimère, trimère, etc...).

Il a été constaté, de manière inattendue, selon la présente invention, que ces peptides peuvent être utilisés aussi bien *in vivo* qu'*in vitro* comme agents d'internalisation de substances d'intérêt dans les cellules.

En effet, mettre en oeuvre *in vivo* une technique connue en *in vitro* implique de déterminer plusieurs paramètres (biodisponibilité, réactions immunitaires, etc.) qui n'interviennent pas lors des essais *in vitro,* en espace clos. En outre, un tel transfert de l*'in vitro* à l'*in vivo* reste somme toute assez hasardeux, du fait des nombreuses interactions potentielles susceptibles d'interférer avec la manipulation *in vivo* (réaction immunitaire, effets secondaires, blocage ou inhibition des principes actifs, rejet, etc...) ; par conséquent il y a peu d'espérance de réussite raisonnable dans une application *in vivo* d'une technique *in vitro.*

Ainsi, selon un premier aspect, la présente invention a pour objet une séquence d'acides aminés qui présente la capacité de faciliter la pénétration d'une substance d'intérêt à l'intérieur des cellules et/ou des noyaux cellulaires, et qui est caractérisée en ce qu'elle est capable de réagir *in vivo* avec les aminoglycanes.

Selon un autre aspect, la présente invention a pour objet une séquence d'acides aminés qui présente la capacité de faciliter la pénétration d'une substance d'intérêt à l'intérieur des cellules et/ou des noyaux cellulaires, et qui est caractérisée en ce qu'elle est issue d'une protéine d'origine humaine et capable de réagir avec les aminoglycanes.

Plus particulièrement, dans l'un et l'autre cas, cette séquence est caractérisée en ce qu'elle réagit avec l'héparine, les chondroïtines sulfates et leurs dérivés.

Les travaux de recherches menés dans le cadre de la présente invention ont permis de mettre en évidence que la pénétration des peptides est complètement inhibée par l'incubation des cellules à 4°C. En outre, elle est inhibée en partie par des inhibiteurs du métabolisme cellulaire comme l'azide de sodium (inhibiteur de l'ATPase), la genisteine (inhibiteur de la tyrosine kinase et de la liaison à l'ATP). Le mécanisme d'internalisation des peptides de l'invention, et donc des substances d'intérêt couplées auxdits peptides, est donc dépendant de l'énergie. La vectorisation mettant en oeuvre les peptides de l'invention est donc remarquable par le fait qu'elle ne relève pas d'un système passif mais au contraire s'effectue via un récepteur. Les séquences d'acides aminés selon l'invention sont donc caractérisées, outre leur capacité de réagir *in vivo* avec les aminoglycanes, les aminoglycanes sulfates, les chondroïtines et les chondroïtines sulfates, par leur capacité à se fixer sur un récepteur de la membrane cellulaire et de traverser ladite membrane cellulaire grâce à ce récepteur. Ainsi, les séquences d'acides aminés de l'invention se distinguent de transporteurs peptidiques de l'art antérieur capables de traverser la membrane cellulaire de manière passive.

Les peptides selon l'invention sont donc remarquables en ce qu'ils présentent la capacité de pouvoir traverser les membranes cellulaires par un mécanisme actif, puis de se loger dans le cytoplasme et/ou le noyau des cellules et de permettre ainsi de disposer d'un vecteur dont l'emploi n'est pas limité, lors du passage dans la cellule, par la taille des substances à transporter. En effet, les vecteurs de l'invention sont capables de transporter des médicaments, allant des petites molécules chimiques (faible poids moléculaire) aux protéines ou acides nucléiques de type plasmide (haut poids moléculaire). La mise en oeuvre de ces vecteurs ouvre ainsi une nouvelle voie de thérapie protéique intracellulaire ou de thérapie génique. Cette capacité particulière de pénétration des vecteurs de l'invention, permet de cibler de manière préférentielle les "médicaments" dans les cellules, contribuant ainsi à une réduction potentielle de la toxicité des médicaments et une augmentation potentielle de l'index d'efficacité.

Par "dérivés de l'héparine ou de chondroïtines sulfates" ou par "aminoglycanes du type de l'héparine ou des chondroïtines sulfates", il faut entendre tout produit ou sous-produit comme défini dans les publications citées en références (**1**) à (**3**).

Par "faciliter la pénétration", on entend faciliter le passage, ou la translocation, d'une substance du milieu extérieur jusque dans le milieu intracellulaire, et tout particulièrement dans le cytoplasme et/ou le noyau de la cellule. Cette pénétration peut être déterminée par divers procédés, comme par exemple un test de pénétration cellulaire comportant une première étape d'incubation de la séquence d'acides aminés en présence de cellules en culture, suivie d'une étape de fixation et de perméabilisation de ces cellules, puis d'une révélation de la présence de ladite séquence d'acides aminés à l'intérieur de la cellule. L'étape de révélation peut être effectuée avec une autre incubation en présence d'anticorps marqués et dirigés contre ladite séquence, suivie d'une détection dans le cytoplasme ou à proximité immédiate du noyau cellulaire, ou même au sein de celui-ci, de la réaction immunologique entre la séquence et l'anticorps marqué. La révélation peut aussi être réalisée en marquant une séquence d'acides aminés selon l'invention et en détectant la présence dudit marquage dans ces compartiments cellulaires. Un test de pénétration cellulaire a été décrit par exemple dans la demande de brevet n° WO 97/02840 précitée.

Par "substance d'intérêt", on entend tout produit présentant un intérêt, notamment, biologique, pharmaceutique, diagnostique, de traçage, ou agroalimentaire. Il peut s'agir d'acides nucléiques (acide ribonucléique, acide désoxyribonucléique) pouvant être d'origines diverses, et notamment humaine, virale, animale, eucaryote ou procaryote, végétale, synthétique, etc., et pouvant avoir une taille variable, allant du simple oligonucléotide au génome ou fragment de génome. Il peut s'agir aussi d'un génome viral ou d'un plasmide. La substance peut également être une protéine, telle qu'une enzyme, une hormone, une cytokine, une apolipoprotéine, un facteur de croissance, un antigène, un anticorps, etc. Il peut aussi s'agir d'une toxine, d'un antibiotique, d'une molécule antivirale ou d'un immuno-modulateur.

De manière générale, la substance d'intérêt peut être tout principe actif de médicament, qu'il s'agisse d'un produit chimique, biochimique, naturel ou synthétique. Il peut s'agir de petites molécules, d'un poids moléculaire de l'ordre 500 D ou de grosses molécules comme des protéines de plusieurs milliers de daltons.

La substance d'intérêt peut être directement active ou être activable *in situ* par la séquence d'acides aminés, par un agent distinct ou par les conditions environnementales.

L'invention étend sa portée aux associations de la séquence d'acides aminés avec une substance d'intérêt telle que définie ci-dessus.

Dans un mode de réalisation préféré de la présente invention, afin de pallier les risques décrits plus haut, la séquence d'acides aminés est codée par la lignée germinale, et donc ne porte pas de mutations (substitution, délétion, addition, etc.).

Dans la présente invention, une séquence d'acides aminés tout particulièrement préférée est issue d'une protéine synthétisée par une cellule humaine. De manière avantageuse, ladite protéine synthétisée par une cellule humaine est choisie parmi les protéines se liant avec les aminoglycanes du type héparine ou chondroïtine sulfate. Il s'agit par exemple d'une séquence d'acides aminés issue de la lipoprotéine B humaine.

De manière générale, la séquence d'acides aminés comprend un nombre élevé d'acides aminés basiques, comme c'est le cas dans la lysine, l'arginine ou l'histidine, par exemple.

Par "nombre élevé", il faut comprendre au moins égal à 3.

Un type de séquences d'acides aminés préférées, pour la mise en oeuvre de la présente invention, est constitué par, ou comporte, au moins un groupe d'acides aminés répondant à l'une des formules suivantes : a) (XBBBXXBX)ₙ ; b) (XBBXBX)ₙ ; c) (BBXₘYBBXₒ)ₙ ; d) (XBBXXBX)ₙ ; et e) (BXBB)ₙ ;
dans lesquelles :
B est un acide aminé basique ; X est un acide aminé non basique, de préférence hydrophobe, tel que l'alanine, l'isoleucine, la leucine, la méthionine, la phénylalanine, le tryptophane, la valine ou encore la tyrosine ; Y représente soit une liaison directe, soit 1 à 20 acides aminés ; m est un nombre entier compris entre 0 et 5 ; n est un nombre entier compris entre 1 et 10, de préférence entre 1 et 3 ; et o est un nombre entier compris entre 0 et 5.

De manière générale, les séquences d'acides aminés ont moins de 100 acides aminés, mieux moins de 50 acides aminés, et encore mieux moins de 25 acides aminés.

Avantageusement, les séquence d'acides aminés selon l'invention comprennent de 6 à 25 acide aminés.

Des séquences d'acides aminés préférées pour la mise en oeuvre de la présente invention sont celles identifiées par SEQ ID NO : 2, 3, 5, 7, 9, 10, 13, 16, 17, 19, 20, 21, 23, 25, 26, 30, 33, 34, 35, 36, 37, 38 et 39 sur la liste annexée qui fait partie de la présente description. Parmi celles-ci, on préfère tout particulièrement les séquences identifiées par SEQ ID NO : 2, 3, 5, 7, 9, 10, 13, 16, 19, 20, 21, 23, 25, 26, 30, 36 et 38.

Un autre type de séquences préférées, pour la mise en oeuvre de la présente invention, est constitué par, ou comporte, au moins deux domaines, l'un de ces domaines comprenant une séquence d'acides aminés de formule : a) XBBBXXBX ; b) XBBXBX ; c) BBXₘYBBXₒ ; d) XBBXXBX ; ou e) BXBB ;
et l'autre de ces domaines comprenant une séquence d'acides aminés de formule : a) XBBBXXBX ; b) XBBXBX ; c) BBXₘYBBXₒ ; d) XBBXXBX ; e) BXBB ; ou f) un fragment d'anticorps ;
formules dans lesquelles : B est un acide aminé basique, X est un acide aminé non basique, de préférence hydrophobe, Y représente soit une liaison directe, soit 1 à 20 acides aminés, m est un nombre entier compris entre 0 et 5, o est un nombre entier compris entre 0 et 5.

Une séquence d'acides aminés particulièrement intéressante est la séquence SEQ ID NO : 1, dans la mesure où (1) à l'état au moins de dimère, elle a les propriétés attendues et (2) à l'état de monomère ou de polymère, elle confère, à une autre séquence d'acides aminés à laquelle elle est couplée, lesdites propriétés ou potentialise considérablement ces propriétés lorsque ladite séquence les possède déjà. De même les peptides désignés HBP3, HBP7, (HBP3)2, HBP6, HBP7, HBP10 et HBP13 présentent cette capacité de potentialisation.

Les peptides tels que définis plus haut sont capables de transporter, à l'intérieur des cellules, des molécules qui leur sont associées de façon covalente ou non covalente, et sont ainsi des vecteurs efficaces pour le transfert intracellulaire des substances d'intérêt.

Les sept séquences décrites dans le tableau 1 ci-après sont particulièrement utiles pour le transport intracytoplasmique. Il s'agit des séquences (HB1)3, HBP6, (HBP3)2, HBP7, HBP11, HBP 13 et HBP2.

**Tableau 1**

| Identitée | Séquence AA | % K Résidus totaux | % R Residus totaux | % K+R |
|---|---|---|---|---|
| (HB1)₃ | VKRGLKLVKRGLKRVKRGLKR | 28 | 24 | 52 |
| HBP6 | GRPRESGKKRKRKRKRLKP | 32 | 31 | 63 |
| (HBP)₂ | SERKKRRRESRKKRRRES | 22 | 44 | 67 |
| HBP7 | GKRKKKGKLGKKRDP | 46 | 13 | 60 |
| HBP11 | AKTGKRKRSG | 30 | 20 | 50 |
| HBP13 | KHLKKHLKKHLK | 50 | 0 | 50 |
| HBP2 | RKGKFYKRKQCKPSRGRK | 33 | 22 | 55 |

Deux séquences sont particulièrement utiles pour le transport intranucléaire. Il s'agit des séquences HBP10 et HBP15. Ces deux séquences se caractérisent par leur contenu élevé en acides aminés basiques, et plus particulièrement par le fait qu'elles ne contiennent pas de résidu Lysine, contrairement aux séquences utiles pour le transport intracytoplasmique.

Chacune des séquences décrites dans le tableau 1 possède au moins 20% de résidus de lysine et au moins 50% de résidus sont des acides aminés basiques sur le nombre total des résidus de la séquence

La présente invention met en évidence que le couplage de peptides tels que définis plus haut avec des vecteurs polypeptidiques capables de pénétrer à l'intérieur des cellules augmente considérablement le pouvoir de translocation de ces vecteurs.

La présente invention met aussi en évidence que le couplage de peptides tels que définis plus haut, ou de leurs formes polymères, avec un ligand, dont la fonction est de réagir avec un récepteur présent sur la membrane cellulaire, augmente considérablement la capacité de ce ligand à se fixer sur la membrane cellulaire.

Selon un autre de ses aspects, la présente invention a pour objet l'utilisation des séquences d'acides aminés définies plus haut pour la préparation de compositions destinées au transfert de substances d'intérêt dans des cellules. Cette capacité des peptides de l'invention est avantageuse pour permettre le transport de substances actives à travers des membranes biologiques et tout particulièrement à travers les barrières hématoencéphalique, hématorétinienne, intestinale, pulmonaire. Les peptides de l'invention présentent l'intérêt de pouvoir être utilisé sous des formes d'administration adaptées tant à la substance active à laquelle ils sont couplés qu'au type de cellule ciblée, notamment celles nécessitant le passage des barrières ci-dessus.

Dans un autre mode de réalisation, la présente invention concerne l'utilisation desdites séquences d'acides aminés dans un vecteur peptidique. Ces vecteurs, du fait des propriétés desdites séquences d'acides aminés, peuvent être facilement utilisés pour le transfert intracytoplasmique et intranucléaire chez l'homme, sans aucun risque pour celui-ci, ni aucune dégradation de la substance d'intérêt couplée au vecteur.

Pour atteindre ce but, il faut notamment qu'un vecteur soit capable de véhiculer des quantités relativement importantes de molécules à l'intérieur des cellules et qu'il ne soit pas reconnu comme antigène étranger par le système immunitaire humain.

Le tableau 1 ci-dessous indique pour plusieurs peptides de l'invention leur niveau de pénétration intracytoplasmique et/ou intranucléaire couplé à un marqueur de faible poids moléculaire permettant leur détection comme la biotine ou la fluorescéine (Marq) ou à une substance d'intérêt biologique (Subst).

Les résultats rapportés dans le tableau 1bis sont basés sur les données expérimentales rapportées ci-après.

**Tableau 1bis**

| Peptide | SEQ ID NO | intracytoplasmique | | intranucléaire | |
|---|---|---|---|---|---|
| | | Marq | Subst | Marq | Subst |
| HBP1 | 1 | +/- | ND | - | - |
| (HBP1)₂ | 2 | +/- | + | - | - |
| (HBP1)₃ | 3 | ++ | ++ | +/- | +/- |
| | 4 | | | | |
| | 5 | | | | |
| | 6 | | | | |
| | 7 | | | | |
| | 8 | | | | |
| | 9 | | | | |
| | 10 | | | | |
| | 11 | | | | |
| | 12 | | | | |
| | 13 | | | | |
| | 14 | | | | |
| | 15 | | | | |
| | 16 | | | | |
| HBP4 | 17 | +/- | ND | - | - |
| HBP5 | 18 | +/- | ND | - | - |
| (HPB5)₂ | 19 | + | + | - | - |
| HBP2 | 20 | + | ++ | - | - |
| HBP6 | 21 | +++ | +++ | +/- | +/- |
| | 22 | | | | |
| | 23 | | | | |
| | 24 | | | | |
| | 25 | | | | |
| (HBP3) ₂ | 26 | +++ | +++ | + | + |
| | 27 | | | | |
| | 28 | | | | |
| | 29 | | | | |
| HBP7 | 30 | +++ | +++ | + | +/- |
| | 31 | | | | |
| | 32 | | | | |
| HBP8 | 33 | +/- | +/- | - | - |
| HBP9 | 34 | +/- | +/- | - | - |
| HBP10 | 35 | +/- | +/- | ++ | ++ |
| HBP11 | 36 | ++ | ++ | +/- | +/- |
| HBP12 | 37 | +/- | +/- | - | - |
| HBP13 | 38 | ++ | ++ | +/- | +/- |
| HBP15 | 39 | +/- | + | +++ | +++ |

Ces résultats indiquent que les peptides qui sont capables d'une translocation nucléaire massive sont ceux dont la charge positive est surtout constituée par la présence d'arginine et l'absence presque totale de lysine.

Un premier groupe de peptides selon l'invention comprend les séquences d'acides aminés capables de permettre la pénétration d'une substance d'intérêt dans la cellule mais peu ou pas dans le noyau. A titre d'exemple on peut citer les peptides de séquences SEQ ID NO : 2, 3, 17, 18, 19, 20, 21, 33, 34, 37 et 38.

Un second groupe de peptides selon l'invention comprend les séquences d'acides aminés capables de permettre la pénétration d'une substance d'intérêt dans la cellule et dans le noyau de la cellule. A titre d'exemple on peut citer les peptides de séquences SEQ ID NO : 26, 35 et 39.

Un vecteur selon la présente invention est caractérisé en ce qu'il est constitué par, ou comprend, une séquence d'acides aminés telle que définie plus haut.

En variante, le vecteur est basé sur le couplage, d'une part, de séquences d'acides aminés réagissant avec les aminoglycanes et, d'autre part, de peptides nouveaux issus de la partie variable d'anticorps humains anti-ADN. Le couplage à l'intérieur d'une même molécule de séquences d'acides aminés réagissant avec les aminoglycanes et de peptides dérivés des parties variables d'anticorps humains anti-ADN aboutit à la préparation d'un vecteur peptidique particulièrement efficace pour la translocation et le transfert intracellulaire de substances d'intérêt, surtout lorsque les séquences d'acides aminés réagissant avec les aminoglycanes sont d'origine humaine.

Cette association, de plus, donne naissance à un vecteur de translocation et de transfert particulièrement adapté pour l'utilisation chez l'homme. En effet, comme indiqué plus haut, bien que les vecteurs peptidiques d'origine murine connus d'après WO 97/02840 soient codés par la lignée germinale et ne portent pas de mutations, et par conséquent devraient être antigéniquement proches de ceux rencontrés chez l'homme, il est possible que leur injection chez l'homme induise une réaction immunitaire. Le vecteur peptidique formé du PLH selon la présente invention et de peptides dérivant d'anticorps anti-ADN, tous deux d'origine humaine, codés par la lignée germinale et ne portant pas de mutations, évite ce problème.

Les caractéristiques générales de ces peptides dérivant d'anticorps humains anti-ADN sont proches de celles des peptides d'origine murine décrits dans la demande de brevet WO 99/07414, tout en possédant des propriétés additionnelles qui les distinguent de ces derniers, à savoir :
1) ils nécessitent, pour pénétrer à l'intérieur des cellules, un métabolisme actif des cellules (température de culture comprise entre 25 et 39°C, et de préférence 37°C), tandis que les peptides murins sont nettement moins dépendants ;
2) ils réagissent beaucoup moins fortement avec l'ADN que les vecteurs murins ;
3) leur capacité de pénétration n'est pas influencée de façon significative par la molécule qu'ils vont transporter à l'intérieur de la cellule ;
4) ils pénètrent mieux dans les cellules d'origine humaine que dans celles d'autres origines.

Il a été réalisé selon la présente invention un vecteur constitué par un PLH et un ou plusieurs fragments d'anticorps, de préférence polyréactifs, et plus particulièrement un ou plusieurs fragments issus des régions hypervariables des anticorps. De manière préférée, le vecteur objet de l'invention se caractérise par le fait qu'il comprend un fragment de la chaîne lourde d'un anticorps.

Dans la demande de brevet WO 99/07414 précitée, il n'avait été utilisé que des fragments d'une IgG monoclonale, qui est une immunoglobuline monomère, de petite taille et de faible poids moléculaire. La présente invention met en évidence pour la première fois qu'il est aussi possible d'utiliser un fragment issu d'une IgM, qui est une immunoglobuline pentamère, de très haut poids moléculaire. En effet, jusqu'à présent, à la connaissance de la déposante, personne n'avait effectué de recherche sur l'utilisation d'un fragment d'IgM comme vecteur d'internalisation cellulaire, du fait du caractère dissuasif de la grande taille et du poids moléculaire élevé de tels fragments.

La présente invention a donc pour objet un vecteur d'internalisation cellulaire, caractérisé en ce qu'il comprend un ou plusieurs PLH et un ou plusieurs fragments d'une IgM, ou d'une IgG.

De manière préférée, ledit vecteur comprend tout ou partie de la région CDR2 d'un anticorps. En variante, ledit vecteur comprend tout ou partie de la région CDR3 d'un anticorps. Plus particulièrement, ledit vecteur contient au moins une région CDR3 d'un anticorps anti-ADN humain, sélectionné dans le groupe consistant en RTT79, NE-1, et RT72.

Dans une autre variante, la vecteur objet de la présente invention peut aussi comprendre tout ou partie de la région CDR2, et tout ou partie de la région CDR3.

Par "tout ou partie" il faut comprendre que le vecteur de l'invention peut comporter soit l'ensemble de la région CDR concernée, soit une partie seulement de celle-ci, à condition que le vecteur conserve la capacité de pénétrer dans les cellules (homologue fonctionnel). Par "partie de région CDR", il faut comprendre une région CDR dépourvue d'un ou plusieurs acides aminés terminaux. Il peut également s'agir d'une région CDR dans laquelle un ou plusieurs résidus internes ont été délétés ou substitués par d'autres acides aminés, de préférence des acides aminés de même nature (acides aminés basiques, par exemple).

Une partie des exemples qui sont donnés dans la présente demande de brevet se basent sur l'emploi de la SEQ ID NO : 1 provenant de la lipoprotéine B humaine mais, pour l'homme du métier, il est évident que tout PLH naturel
ou artificiel peut être utilisé.

Comme indiqué plus haut, un vecteur selon la présente invention est particulièrement bien adapté pour le transport et le transfert intracellulaire et intranucléaire de substances d'intérêt.

La présente invention vise donc à fournir un vecteur tel que décrit plus haut, caractérisé en ce qu'il comprend une substance d'intérêt naturellement, ou non naturellement, incorporable aux cellules et/ou aux noyaux desdites cellules.

Plus particulièrement, la présente invention a pour objet un vecteur dont la capacité de pénétration est sensiblement indépendante de la nature de la substance d'intérêt qui lui est couplée. Cette caractéristique, propre à ces vecteurs humains par comparaison aux vecteurs murins, est d'un intérêt capital pour l'utilisation envisagée de ces vecteurs. Mais l'invention s'intéresse également à des vecteurs qui sont adaptés à la substance d'intérêt qui lui est couplée.

On entend par "couplage", tout type d'interaction permettant une association physique entre la substance d'intérêt et le vecteur. Il peut s'agir d'un couplage clivable ou non clivable en fonction du milieu biologique et/ou de la substance d'intérêt transporté par les peptides de l'invention ou encore clivable par des moyens physiques appliqués à l'organisme auquel a été administré le vecteur couplé à la substance active. Ainsi, l'expression de l'effet biologique de la substance peut nécessiter qu'elle soit libérée du vecteur. A titre d'exemple d'une substance d'intérêt dont il est préférable qu'elle soit libéree du vecteur, on peut citer la doxorubicine.

Il faut cependant que l'interaction soit suffisamment solide pour que le vecteur ne se dissocie pas avant ou pendant la pénétration cellulaire. Pour cette raison, le couplage préféré selon l'invention est le couplage covalent, il pourrait cependant s'agir d'un couplage non covalent. La substance d'intérêt peut être couplé directement au peptide soit à l'une de ces extrémités terminales soit au niveau d'une chaîne latérale d'un des acides aminés. La substance d'intérêt peut aussi être couplé indirectement par le biais d'un bras de liaison soit à l'une des extrémités terminales des peptides soit au niveau d'une chaîne latérale d'un des acides aminés.

Le couplage peut être réalisé par tout procédé de couplage chimique, biochimique ou enzymatique ou génétique connu de l'homme du métier, mais on préférera généralement utiliser un réactif de pontage homo- ou hétérofonctionnel du type 4-(N-maléimidométhyl)cyclohexane-1-carboxylate de succinimidyle (SMCC). On peut encore citer comme moyen de couplage ceux choisis parmi : des agents bi ou multifonctionnels contenant des groupements alkyle, aryle, aralkyle ou peptidique, des esters, aldéhydes ou acides d'alkyle, aryle ou aralkyle, des groupements anhydrides, sulfhydriles, ou carboxyles tels que les dérivés de l'acide maleymil benzoïque, de l'acide maleymil propionique et des dérivés succynimidyle, des groupes dérivés du bromure ou chlorure de cianogène, carbonyldiimidazole, des esters de succinimide ou des halogenures sulphoniques.

Dans une autre forme de réalisation de la présente invention, le couplage de ladite substance d'intérêt peut aussi être réalisé par toute technique de génie génétique connue de l'homme de l'art. Par "génie génétique", on entend l'utilisation d'un vecteur d'expression dans lequel l'ADN codant pour les peptides-vecteurs est cloné en phase en 5' et/ou 3' de l'ADN complémentaire du gène d'intérêt. L'expression de la protéine de fusion est placée sous le contrôle d'un promoteur. Le système d'expression peut être utilisé dans une cellule hôte procaryote ou eucaryote pour la production de la protéine de fusion.

Dans une première forme de réalisation, le couplage de ladite substance d'intérêt est réalisé au niveau de l'extrémité N-terminale de la séquence d'acides aminés selon l'invention. Dans une seconde forme d'exécution de l'invention, le couplage de ladite substance d'intérêt est réalisé au niveau de l'extrémité C-terminale de ladite séquence.

De manière surprenante, il a été montré que le vecteur objet de l'invention est capable de potentialiser l'activité biologique et, potentiellement, de réduire la toxicité de ladite substance couplée. La présente invention a donc aussi pour objet un vecteur caractérisé en ce qu'il permet d'augmenter l'activité biologique de la substance d'intérêt à laquelle il est couplé.

Il a aussi été montré que le vecteur objet de l'invention permet la transfection *in vitro* de cellules.

Dans une forme d'exécution particulière de l'invention, le vecteur est couplé à la substance d'intérêt par l'intermédiaire d'au moins une molécule (dite "molécule d'ancrage") présentant une forte affinité naturelle pour la substance d'intérêt à internaliser. L'affinité naturelle de la molécule d'ancrage pour la substance d'intérêt permet au transporteur d'interagir, de façon non covalente, avec ladite substance d'intérêt, et ainsi de l'entraîner lors des déplacements intracellulaires.

Un autre avantage particulièrement intéressant de ce type de transporteur consiste en ce que, de par l'affinité naturelle de la molécule d'ancrage pour la substance d'intérêt, le couplage entre ces deux éléments s'effectue de manière totalement naturelle, sans aucune interaction chimique ou biochimique.

Ce type de transporteur est particulièrement intéressant dans le cas où la substance d'intérêt, de par sa taille et/ou sa structure, s'avère difficile à coupler directement à la séquence d'acides aminés. Ce type de transporteur peut aussi s'avérer particulièrement utile lorsque la substance d'intérêt n'est pas très stable, et qu'une quelconque interaction chimique pour son couplage pourrait la détériorer, ou modifier son activité.

En outre, le transporteur selon l'invention peut ne pas être spécifique d'une seule substance d'intérêt, mais pourra au contraire permettre l'internalisation dans les cellules et/ou les noyaux cellulaires de plusieurs substances d'intérêt différentes.

L'invention concerne également des cellules eucaryotes qui renferment une séquence d'acides aminés conforme à la présente invention. Elle concerne aussi des cellules eucaryotes qui renferment un vecteur et/ou un transporteur conforme à l'invention. Elle concerne également tout type de cellule eucaryote qui a été transfectée par un vecteur et/ou transporteur conforme à la présente invention.

L'invention concerne également un procédé de transfert *in vitro* d'une substance d'intérêt à l'intérieur d'une cellule, et d'augmentation de l'activité biologique desdites substances d'intérêt qui comprend les étapes suivantes .
a) le couplage de la substance à une séquence d'acides aminés, à un vecteur, ou à un transporteur conformes à l'invention, tels que décrits plus haut, et
b) l'incubation de la cellule avec ledit produit de couplage à une température de culture permettant le métabolisme actif de ladite cellule.

Une telle température est comprise entre 25 et 39°C, préférentiellement 37°C.

La présente invention concerne encore une composition comprenant, comme principe actif, soit des vecteurs ou transporteurs chargés avec au moins une substance d'intérêt conforme à la présente invention, soit des cellules eucaryotes qui ont été transfectées conformément à la présente invention. Elle a aussi pour objet l'utilisation de telles compositions pour la formulation et la préparation de produits biologiques, pharmaceutiques, cosmétiques et agro-alimentaires.

L'invention étend sa portée aux sels d'addition basique ou acide pharmaceutiquement acceptables, hydrates, esters, solvates, précurseurs, métabolites ou stéréoisomères, desdits vecteurs et transporteurs chargés avec au moins une substance d'intérêt. L'invention étend également sa portée aux formulations pharmaceutiques comprenant un vecteur ou un transporteur chargés avec au moins une substance d'intérêt en association avec un véhicule, diluant ou excipient pharmaceutiquement acceptable.

L'expression "sels pharmaceutiquement acceptables" se réfère aux sels non toxiques des séquences d'acides aminés selon l'invention qui peuvent être généralement préparés en faisant réagir la base libre avec un acide organique ou inorganique convenable. Ces sels conservent l'efficacité biologique et les propriétés des bases libres. Comme exemples représentatifs de tels sels, on peut citer les sels hydrosolubles et hydroinsolubles, tels que les acétates, ansonates (4,4-diaminostilbènes-2,2'-disulfonates), benzènesulfonates, benzonates, bicarbonates, bisulfates, bitartrates, borates, bromures, buryrates, édétates de calcium, camsylates, carbonates, chlorures, citrates, clavulariates, dichlorhydrates, édétates, édisylates, estolates, ésylates, fumarates, gluceptates, gluconates, glutamates, glycolylarsanylates, hexafluorophosphates, hexylrésorcinates, hydrabamines, bromhydrates, chlorhydrates, hydroxynaphtoates, iodures, isothionates, lactates, lactobionates, laurates, malates, maléates, mandélates, mésylates, méthylbromures, méthylnitrates, méthylsulfates, mucates, napsylates, nitrates, 3-hydroxy-2-naphtoates, oléates, oxalates, palmitates, pamoates (1,1-méthylène-bis-2-hydroxy-3-naphtoates, emboates), pantothénates, phosphates/diphosphates, picrates, polygalacturonates, propionates, p-toluènesulfonates, salicylates, stéarates, subacétates, succinates, sulfates, sulfosalicylates, suramates, tannates, tartrates, téoclates, tosylates, triéthiodides, valérates et les sels de N-méthylglucamine ammonium.

Un sujet peut être traité avec une quantité pharmaceutiquement efficace d'un peptide, d'un vecteur ou d'un transporteur selon l'invention, chargés avec au moins une substance d'intérêt. L'expression "quantité pharmaceutiquement efficace" signifie une quantité capable de faire pénétrer suffisamment de substance d'intérêt pour induire la réponse biologique ou médicale d'un tissu, système, animal ou humain telle qu'attendue par le chercheur ou le médecin traitant.

L'invention vise également des compositions pharmaceutiques convenant à l'introduction d'une substance d'intérêt dans une cellule ou un noyau cellulaire. Les compositions comprennent une quantité efficace d'un vecteur ou transporteur selon l'invention, chargé avec au moins une substance d'intérêt, seul ou en combinaison avec un ou plusieurs supports pharmaceutiquement acceptables. Les compositions sont particulièrement utiles en ce sens qu'elles ont une très faible toxicité, ou ne sont pas toxiques.

L'administration des vecteurs ou transporteurs selon l'invention, ou de leurs sels, chargée avec au moins une substance d'intérêt, peut se faire par l'un quelconque des modes d'administration accepté pour les agents thérapeutiques. Ces procédés comprennent l'administration systémique par exemple orale, nasale, parentérale, ou l'administration topique, par exemple transdermique, ou encore l'administration centrale, par exemple par voie chirurgicale intracrânienne, ou encore l'administration intraocculaire.

L'administration orale peut se faire au moyen de comprimés, gélules, capsules molles (y compris les formulations à libération différée ou prolongée), pilules, poudres, granules, élixirs, teintures, suspensions, sirops et émulsions. Cette forme de présentation est plus particulièrement adaptée au passage de la barrière intestinale.

L'administration parentérale se fait généralement par injection sous-cutanée, intramusculaire ou intraveineuse ou par perfusion. Les compositions injectables peuvent être préparées sous des formes classiques, soit en suspension ou solution liquide soit sous forme solide convenant à une dissolution extemporanée dans un liquide. Cette forme de présentation est plus particulièrement adaptée au passage de la barrière hématoencéphalique.

Une possibilité pour l'administration parentérale utilise l'implantation d'un système à libération lente ou libération prolongée qui assure le maintien d'un niveau constant de dose, par exemple, selon US-A-3 710 795.

Pour l'administration intranasale, on peut utiliser des véhicules intranasaux convenables.

Pour l'administration transdermique, on peut utiliser des timbres cutanés transdermiques bien connus de l'homme du métier. Un système à libération transdermique permet une administration continue.

D'autres préparations topiques préférées comprennent les crèmes, les onguents, les lotions, les sprays aérosols et les gels.

En fonction du mode prévu d'administration, les composés peuvent être sous forme solide, semi-solide ou liquide.

Pour les compositions solides, telles que comprimés, pilules, poudres ou granulés à l'état libre ou inclus dans des gélules, le principe actif peut être combiné avec : a) des diluants, par exemple le lactose, le dextrose, le sucrose, le mannitol, le sorbitol, la cellulose et/ou la glycine ; b) des lubrifiants, par exemple la silice, le talc, l'acide stéarique, son sel de magnésium ou de calcium et/ou le polyéthylèneglycol ; c) des liants, par exemple le silicate de magnésium et d'aluminium, la pâte d'amidon, la gélatine, la gomme adragante, la méthylcellulose, la carboxyméthylcellulose sodique et/ou la poly-vinylpyrrolidone ; le cas échéant, d) des désintégrants, par exemple l'amidon, l'agar, l'acide alginique ou son sel de sodium, ou des mélanges effervescents ; et/ou e) des absorbants, colorants, aromatisants et édulcorants. Les excipients peuvent être par exemple du mannitol, lactose, amidon, stéarate de magnésium, saccharine sodique, talc, cellulose, glucose, sucrose, carbonate de magnésium et analogues de qualité pharmaceutique.

Pour les compositions semi-solides, telles que suppositoires, l'excipient peut, par exemple, être une émulsion ou suspension grasse, ou à base de polyalkylèneglycol, tel que de polypropylène-glycol.

Les compositions liquides, en particulier injectables ou à inclure dans une capsule molle, peuvent être préparées par exemple par dissolution, dispersion, etc. du principe actif dans un solvant pharmaceutiquement pur tel que, par exemple, l'eau, le sérum physiologique, le dextrose aqueux, le glycérol, l'éthanol, une huile et analogues.

Les vecteurs ou transporteurs selon l'invention, chargés avec au moins une substance d'intérêt, peuvent également être administrés sous la forme de systèmes de libération du type liposomes, tels que sous la forme de petites vésicules unilamellaires, de grandes vésicules unilamellaires et de vésicules multilamellaires. Les liposomes peuvent être formés à partir d'une diversité de phospholipides, contenant du cholestérol, de la stéarylamine ou des phosphatidylcholines. Dans une forme d'exécution, un film de composants liquides peut être hydraté avec une solution aqueuse du médicament pour former une couche lipidique encapsulant le médicament, comme décrit dans US-A-5 262 564.

Les compositions selon l'invention peuvent être stérilisées et/ou contenir des adjuvants et substances auxiliaires non toxiques tels que des agents de conservation, de stabilisation, de mouillage ou d'émulsification, des agents favorisant la dissolution, des sels pour régler la pression osmotique et/ou des tampons. En outre, elles peuvent également contenir d'autres substances présentant un intérêt thérapeutique. Les compositions sont préparées, respectivement, par des procédés classiques de mélange, granulation ou enrobage et elles contiennent d'environ 0,1 à 75%, de préférence d'environ 1 à 50 %, de principe actif.

Les vecteurs ou transporteurs selon l'invention, chargés avec au moins une substance d'intérêt, peuvent être également couplés avec des polymères solubles tels que des supports de médicament ciblables. De tels polymères peuvent comprendre la polyvinylpyrrolidone, le copolymère pyrane, le polyhydroxypropyl-méthacrylamide-phénol, le polyhydroxy-éthyl-aspanamide-phénol ou le poly(oxyde d'éthylène)-polylysine substitué par des résidus palmitoyle. En outre, les composés selon la présente invention peuvent être couplés à une classe de polymères biodégradables utiles pour réaliser une libération maîtrisée d'un médicament, par exemple, le poly(acide lactique), la poly(epsilon-caprolactone), le poly(acide hydroxybutyrique), les polyorthoesters, les polyacétals, les polydihydropyranes, les polycyanoacrylates et les copolymères d'hydrogel séquencés réticulés ou amphipatiques.

La posologie pour l'administration des vecteurs ou transporteurs selon l'invention, chargés avec au moins une substance d'intérêt, est choisie selon une diversité de facteurs y compris le type, l'espèce, l'âge, le poids, le sexe et l'état médical du sujet ; la gravité de l'état à traiter ; la voie d'administration ; l'état des fonctions rénale et hépatique du sujet et la nature du composé particulier, ou sel, employé. Un médecin ou vétérinaire normalement expérimenté déterminera facilement, et prescrira, la quantité efficace du vecteur ou transporteur chargé de la substance d'intérêt voulue pour prévenir, contrarier ou stopper le progrès de l'état médical à traiter.

L'une quelconque des compositions pharmaceutiques ci-dessus peut contenir de 0,1 à 99%, de préférence 1 à 70% de principe actif.

A titre d'exemples, les posologies orales des vecteurs ou transporteurs selon l'invention, chargés avec au moins une substance d'intérêt, lorsqu'ils sont utilisés pour les effets indiqués, seront comprises entre environ 0,05 et 1.000 mg/jour par voie orale et, de préférence fournies sous la forme de comprimés contenant 0,5, 1,0, 2, 5, 5,0, 10,0, 15,0, 25,0, 50,0, 100,0, 250,0, 500,0 et 1.000,0 mg de principe actif. Les niveaux efficaces plasmatiques des vecteurs ou transporteurs chargés avec au moins une substance d'intérêt seront compris dans la gamme allant de 0,002 mg à 50 mg par kg de poids corporel et par jour.

Les vecteurs ou transporteurs selon l'invention, chargés avec au moins une substance d'intérêt, peuvent être administrés sous la forme de doses quotidiennes uniques, ou la posologie quotidienne totale peut être administrée en deux, trois ou quatre doses par jour.

Dans une application particulière, la présente invention est relative à un agent de diagnostic, pour mise en oeuvre *in vitro*, constitué par ou refermant au moins un vecteur, un transporteur et/ou une cellule conformes à l'invention. Un tel agent de diagnostic peut également être utilisé *in vivo.*

La présente invention a donc aussi pour objet un kit de diagnostic qui comprend ledit agent de diagnostic. Plus particulièrement, le kit de diagnostic comprend, dans un ou plusieurs récipients, une quantité prédéterminée d'une composition selon l'invention.

De même, la séquence d'acides aminés selon l'invention, ou un vecteur et/ou un transporteur renfermant cette séquence d'acides aminés, ou des cellules transfectées à l'aide dudit vecteur sont susceptibles d'être utilisés *in vivo* à des fins préventives, par exemple et de manière non limitative, pour la prévention des infections virales, des métastases, de l'apoptose cellulaire (maladies dégénératives, ischémie tissulaire...), ou à des fins thérapeutiques, par exemple le traitement des maladies infectieuses (virales, bactériennes ... ), le cancer et la néo-angiogénèse pathologique.

D'autres avantages et caractéristiques de l'invention apparaîtront des exemples de réalisation qui suivent faisant référence aux dessins annexés.

### I - MATERIEL ET METHODE.

### 1) Lignées cellulaires.

a) Cellules normales :
   - PtK2, fibroblastes de rein de rat kangourou
   - 3T3, fibroblastes d'embryon de souris
   - HUVEC, cellules endothéliales humaines
   - CHO, cellules d'ovaires de hamster chinois
   - HUVEC, cellules endothéliales transfectées par un gène de multiplication cellulaire
   - CHO, cellules de rein de hamster
   - CHO-745, cellules de la lignée CHO défectueuses pour la synthèse de xylosyltransférase.
b) Cellules tumorales :
   - H1299, carcinome pulmonaire humain
   - HH9, cellules épithéliales tumorales du sein humain transfectées par un gène codant pour un facteur de croissance
   - MCF7, cellules épithéliales tumorales humaines
   - MCF7 ras, cellules MCF7 transfectées par le gène ras
   - HeLa, carcinome du col de l'utérus humain
   - HCT 116, carcinome du colon humain
   - HT-29, adenocarcinome du colon humain
   - LS174T, adenocarcinome du colon humain
   - B16-F10, cellules de mélanome murin
   - Daudi, lymphome humain de Burkitt
c) Culture des cellules:
   - Les cellules sont cultivées dans du milieu DMEM contenant 2% de L-glutamine, 1% de pyruvate de sodium, de pénicilline (100 U/ml), de streptomycine (100 µg/ml) et 10% de sérum de veau foetal (milieu complet) à 37°C en présence de 5% de CO₂.
   - Les cellules CHO et CHO-745 sont cultivées dans du milieu MEM alpha contenant 2% de L-glutamine, 1% de pyruvate de sodium, pénicilline (100 U/ml), streptomycine (100µg/ml) et 6% de sérum de veau foetal (milieu complet) à 37°C en présence de 5% de CO2.

### 2) Préparation des peptides.

a) Synthèse chimique.
   Les synthèses peptidiques ont été réalisées selon les techniques connues de l'homme de métier (Altergen et Neosystem). Elles sont mises en oeuvre en phase solide sur résine Fmoc. Le clivage est réalisé par l'acide trifluoroacétique (TFA) et les peptides ont été purifiés sur colonne HPLC-CR C5 semi-préparative et élués avec une solution de TFA à 0,1% et un gradient d'acétonitrile (10-70%) dans le TFA. Les peptides lyophilisés ont été dissous dans du NaCl 0,15 M.
b) Construction moléculaire permettant la préparation de protéines comportant des peptides de l'invention.
   Les techniques de biologie moléculaire permettent de construire des plasmides qui une fois introduits dans des cellules adéquates, permettent la synthèse de macromolécules vectorisées.

### - Construction des vecteurs d'expression de protéines recombinantes :

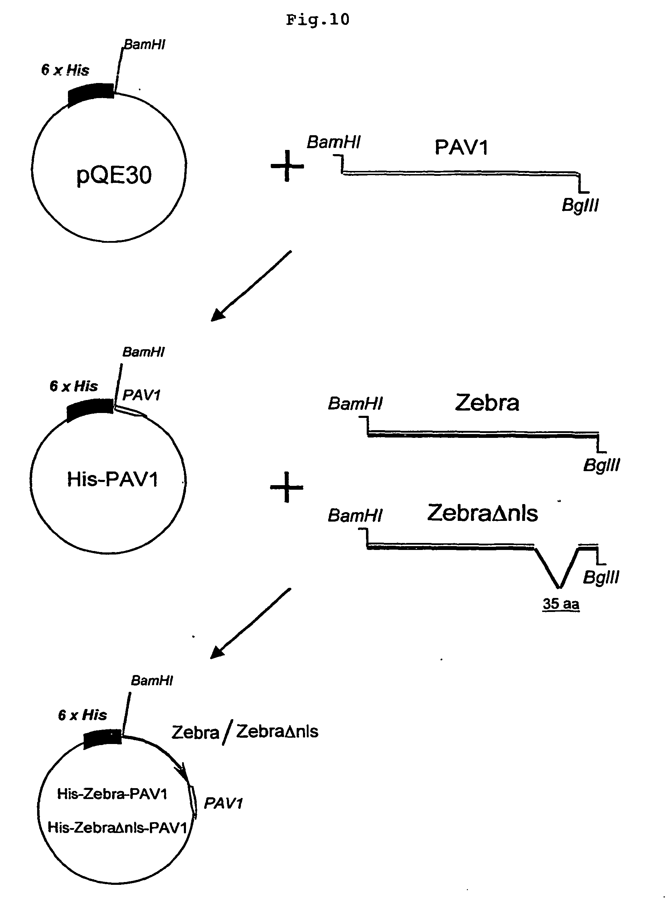

La figure 10 en annexe représente la préparation de vecteurs permettant l'expression de protéines recombinantes contenant les séquences peptidiques de l'invention. Le vecteur procaryote pQE30 (Qiagen) permet l'expression de gènes sous la forme de protéines de fusion (ou protéines recombinantes) avec la séquence 6XHis. Ce vecteur porte l'origine de réplication ColE1, le promoteur fort du phage T5 inductible par l'IPTG, le gène de la p-lactamase conférant la résistance à l'ampicilline et un site multiple de clonage en 3' de la séquence codant l'étiquette 6XHis permettant le clonage d'un ADNcomplémentaire en phase avec la séquence 6Xhis.

Les oligonucléotides complémentaires de 63-mer :
. PAV1U :
. PAV1L :
sont hybridés. Le segment d'ADN obtenu possède un site BamHI en 5*'* et un site BglII en 3*'*. Il code (caractères en gras) pour la séquence peptidique PAV1 : VKRGLKLRHVRPRVTRMDV. Ce fragment est cloné au site BamHI du vecteur pQE30. Les ADN complémentaires (ADNc) codant pour la protéine virale Zebra (BZLF1) du virus d'Epstein-Barr (EBV) ou la protéine Zebra délétée de son domaine de localisation nucléaire (nls) de 35 acides aminés ont été obtenus par PCR. Ils ont été clonés au site BamHI du vecteur His-PAV1 ou pQE30. Les plasmides résultants permettent l'expression de protéines recombinantes His₆-Zebra-PAV1, His₆-ZebraΔnls-PAV1, His₆-Zebra et His₆-ZebraΔnls après transformation des bactéries E. coli.

### - Induction, extraction et purification des protéines recombinantes :

La production des protéines recombinantes est induite à 37°C par addition d' IPTG (isopropyl-β-D-thiogalactopyranoside) à 1mM aux cultures bactériennes en phase exponentielle de croissance en milieu Luria Bertani supplémenté à 40 µg/ml d'ampicilline. 12 heures après addition d'IPTG, les bactéries sont centrifugées à 5700g pendant 15 min. à 4°C. Le culot bactérien est repris dans 5 volumes de tampon de lyse dénaturant (20mM Tris-HCl pH7.8 ; 0.5M NaCl ; 10% glycérol ; 6M Guanidine-HCl). Après une incubation de 20 min. à température ambiante sous agitation lente, le lysat est clarifié par une centrifugation de 30 min. à 15000g à 4°C. Le surnageant contenant la protéine recombinante est conservé à -80°C.

Les protéines recombinantes 6XHis sont purifiées par chromatographie d'affinité sur une colonne de résine " TALON " (CLONTECH) préalablement équilibrée avec du tampon de lyse dénaturant. Après 3 lavages successifs de la résine avec 10 volumes de tampon de lyse dénaturant contenant 10 mM d'imidazole, la protéine recombinante liée à la colonne est renaturée par un gradient de 6 à 0 M de Guanidine-HCl en tampon 20mM Tris-HCl pH7.8 ; 0.5M NaCl ; 10% glycérol ; 0.5 mM PMSF. La protéine recombinante est éluée par un gradient de 20mM à 1 M d'imidazole pH 8.0. Les différents éluats sont analysés sur gel dénaturant SDS-Acrylamide 12%. Les fractions contenant la protéine purifiée sont rassemblées et dialysées 2 heures à 4°C contre le tampon 20 mM HEPES pH 7.5, 150 mM NaCl. La protéine est concentrée, aliquotée et congelée rapidement dans l'azote liquide et conservée à -80°C.

### 3) Peptides utilisés.

a) Peptides non fonctionnalisés.
   Les séquences suivantes (SEQ ID NO : 1 à SEQ ID NO : 48) sont listées en annexe, conformément à la norme ST-25.
   SEQ ID NO : 1. Peptide réagissant avec l'héparine et issu de la séquence d'acides aminés (3358-3372) de la lipoprotéine B humaine (**12**), aussi désigné ci-après HBP1.
   SEQ ID NO : 2. Peptide réagissant avec l'héparine dimère de la SEQ ID NO : 1, aussi désigné ci-après (HBP1)2.
   SEQ ID NO : 3. Peptide réagissant avec l'héparine trimère de la SEQ ID NO : 1, aussi désigné ci-après (HBP1)3.
   SEQ ID NO : 4. Peptide correspondant à la région CDR3 hypervariable de l'anticorps monoclonal murin anti-ADN F4.1 (**13**).
   SEQ ID NO : 5. Peptide contenant les SEQ ID NO : 1 et SEQ ID NO : 4.
   SEQ ID NO : 6. Peptide contenant une partie des régions CDR2 et CDR3 de l'anticorps monoclonal murin F4.1 (**13**).
   SEQ ID NO : 7. Peptide contenant les SEQ ID NO : 1 et SEQ ID NO : 6.
   SEQ ID NO : 8. Peptide correspondant à la région hypervariable CDR3 de l'anticorps monoclonal humain anti-ADN RTT79 (**14**).
   SEQ ID NO : 9. Peptide contenant les SEQ ID NO : 1 et SEQ ID NO : 8.
   SEQ ID NO : 10. Peptide réagissant avec l'héparine et contenant la SEQ ID NO : 1 et la séquence du peptide correspondant à la région hypervariable CDR3 de l'anticorps monoclonal humain anti-ADN NE-1 (**15**), aussi désigné ci-après sous le No. 1047.
   SEQ ID NO : 11. Peptide contenant la SEQ ID NO : 1 et la séquence du peptide correspondant à la région hypervariable CDR3 de l'anticorps monoclonal humain anti-ADN RT72 (**16**).
   SEQ ID NO : 12. Peptide contenant la séquence du NLS (nuclear localisation signal) des cellules 3T3 et la SEQ ID NO : 6.
   SEQ ID NO : 13. Peptide contenant la SEQ ID NO : 1 et la séquence des régions CDR2 et CDR3 de l'anticorps monoclonal humain anti-ADN NE-1.
   SEQ ID NO : 14. Peptide contenant une partie de la région CDR3 de l'anticorps monoclonal murin F4.1 et de la SEQ ID NO : 6.
   SEQ ID NO : 15. Peptide contenant deux fois la séquence du peptide correspondant à la région hypervariable CDR3 de l'anticorps monoclonal humain anti-ADN NE-1.
   SEQ ID NO : 16. Peptide résultant de l'inclusion, en position 13-19, de la SEQ ID NO : 1 dans la SEQ ID NO : 15.
   SEQ ID NO : 17. Peptide réagissant avec l'héparine dérivé de la séquence d'acides aminés de la lipoprotéine E humaine (12), aussi désigné HBP4.
   SEQ ID NO : 18. Peptide réagissant avec l'héparine dérivé de la séquence d'acides aminés de l'agrine (**17**), protéine de la matrice extracellulaire qui régule la différenciation de la jonction neuromusculaire.
   SEQ ID NO : 19. Dimère de la SEQ ID NO : 18.
   SEQ ID NO : 20. Peptide réagissant avec l'héparine dérivé de la séquence d'acides aminés de "l'insulin growth factor binding protein" (**18)**.
   SEQ ID NO : 21. Peptide réagissant avec l'héparine et dérivé de la séquence d'acides aminés de la partie C-terminale de la chaîne A du facteur de croissance des plaquettes (**19**), aussi désigné HPB6.
   SEQ ID NO : 22. Peptide contenant 12 lysines (K) et la SEQ ID NO : 6.
   SEQ ID NO : 23. Peptide contenant 12 lysines (K) et la SEQ ID NO : 5.
   SEQ ID NO : 24. Peptide présentant une activité anti-microbienne (**29**).
   SEQ ID NO : 25. Peptide réagissant avec l'héparine et correspondant à la séquence de l'«insulin-like growth factor-binding protein »(**18**), aussi désigné ci-après HBP2.
   SEQ ID NO : 26. Peptide réagissant avec l'héparine et dimère d'un peptide dérivé de la partie C-terminale de la séquence de la superoxide dismutase humaine (**20**), aussi désigné ci-après (HBP3)₂.
   SEQ ID NO : 27. Peptide réagissant avec l'héparine et correspondant à la séquence SEQ ID NO : 26 dans laquelle les acides aminés sont en configuration D.
   SEQ ID NO : 28. Peptide réagissant avec l'héparine et dont la séquence dérive de la séquence SEQ ID NO : 26 et contient le motif RGD liant sélectivement les αv intégrines **(21).**
   SEQ ID NO : 29. Peptide réagissant avec l'héparine et constitué des peptides de SEQ ID NO : 1 et SEQ ID NO : 17, aussi désigné ci-après HBP1-HBP4.
   SEQ ID NO : 30. Peptide réagissant avec l'héparine et dérivé de la partie C-terminale de la séquence du facteur de croissance des cellules épidermales (EGF) (**22**), aussi désigné ci-après HBP7.
   SEQ ID NO : 31. Peptide réagissant avec l'héparine et correspondant au peptide dont la séquence est SEQ ID NO : 12 où les acides aminés sont en position recto verso.
   SEQ ID NO : 32. Peptide réagissant avec l'héparine et correspondant à la séquence SEQ ID NO : 30 dans laquelle les acides aminés sont en configuration D.
   SEQ ID NO : 33. Peptide réagissant avec l'héparine et contenant une partie de la séquence du facteur acide de croissance (aFGF) des fibroblastes (**6**), aussi désigné ci-après HBP8.
   SEQ ID NO: 34. Peptide réagissant avec l'héparine et contenant une partie de la séquence du facteur basique de croissance (bFGF) des fibroblastes, aussi désigné ci-après HBP9.(**23**).
   SEQ ID NO : 35. Peptide réagissant avec l'héparine et correspondant à une partie C-terminale de la séquence des mucines intestinales (**24**), aussi désigné ci-après HBP10.
   SEQ ID NO : 36. Peptide réagissant avec l'héparine et contenant une partie de la séquence C-terminale de l'interféron γ humain **(25),** aussi désigné ci-après HBP11.
   SEQ ID NO : 37. Peptide réagissant avec l'héparine et contenant une partie de la séquence de la sous-unité p40 de l'interleukine 12 humaine (**26**), aussi désigné ci-après HBP12.
   SEQ ID NO : 38. Peptide réagissant avec l'héparine et contenant une partie de la séquence du facteur la dérivé des cellules stromales (**27**), aussi désigné ci-après HBP13.
   SEQ ID NO : 39. Peptide réagissant avec l'héparine et comprenant une partie de la séquence de l'« heparin binding protein » (CAP37) (**28**), aussi désigné ci-après HBP15.
   SEQ ID NO : 40. Peptide réagissant avec l'héparine correspondant au peptide de séquence SEQ ID NO : 10 (1047) additionné de 13 lysines en N-terminal.
   SEQ ID NO : 41. Peptide réagissant avec l'héparine correspondant au peptide de séquence SEQ ID NO : 28 ((HBP3)2) additionné de 13 lysines en N-terminal.
   SEQ ID NO : 42. Peptide réagissant avec l'héparine correspondant au peptide de séquence SEQ ID NO : 39 (HBP10) additionné de 13 lysines en N-terminal.
   SEQ ID NO : 43. Peptide présentant une activité anti-microbienne et contenant les peptides de séquences SEQ ID NO : 10 (1047) et SEQ ID NO : 24.
   SEQ ID NO : 44. Peptide présentant une activité anti-microbienne et contenant les peptides de séquences SEQ ID NO : 24 et SEQ ID NO : 30 (HBP7).
   SEQ ID NO : 45. Peptide présentant une activité anti-microbienne et contenant les peptides de séquences SEQ ID NO : 24 et SEQ ID NO : 38 (HBP13).
   SEQ ID NO : 46. Peptide comprenant le peptide de séquence SEQ ID NO : 26 (HBP3)₂ additionné en N-terminal de glycine-phtaloyl.
   SEQ ID NO : 47. Peptide comprenant le peptide de séquence SEQ ID NO : 21 (HBP6) additionné en N-terminal d'un motif salicylyl.
   SEQ ID NO : 48. Peptide comprenant le peptide de séquence SEQ ID NO : 21 (HBP6) additionné en C-terminal d'un motif salicylyl.
b) Peptides fonctionnalisés.
   Ces peptides correspondent aux SEQ ID NO : 1 à 48 ci-dessus mais portant, du côté N-terminal, ou bien une cystéine qui permet le couplage covalent à des substances d'intérêt (voir ci-dessous) ou de la biotine permettant l'association non-covalente des peptides avec la streptavidine ou l'avidine conjuguée à de la peroxydase (voir points (1) et (2) ci-dessous)

### II - Mécanisme de pénétration des peptides.

### 1) Implication des glycosaminoglycanes (GAG).

Les peptides pénètrent dans les cellules par endocytose médiée par les GAG présents sur les membranes de toutes les cellules eucaryotes. Des tests ont été effectués pour voir leur rôle dans la pénétration des peptides.
a) L'héparine (50 µg/ml) a été incubée avec les cellules pendant 1 heure avant d'ajouter les conjugués peptide-péroxydase à 0,2µg/ml pendant 2 heures. Les cellules ont ensuite été lysées et la peroxydase dosée.
L'héparine à 50 µg/ml inhibe totalement l'internalisation de tous les peptides dans les cellules H1299 et HeLa.
La quantité d'héparine nécessaire pour inhiber 50% de la pénétration des peptides couplés à la peroxydase a été évaluée avec ces deux types de cellules, en incubant les cellules avec les conjugués peptide-péroxydase avec des concentrations croissantes d'héparine. Les résultats de l'inhibition de l'internalisation des peptides sont exprimés en concentration d'héparine (µg/ml) dans le tableau 2 ci-dessous.

**Tableau 2**

| | Peptide SEQ ID NO : 26 (HBP3)₂ | Peptide SEQ ID NO : 21 (HBP6) |
|---|---|---|
| Cellules 3T3 | 14,5 µg/ml | 4,1 |
| Cellules HeLa | 8,5 | 2,3 |

Ces expériences démontrent le rôle des sulfates d'héparane dans la pénétration des peptides.
b) Les cellules CHO-745 sont dérivées des cellules CHO. Elles sont déficientes en xylosyltransférase qui participe à la formation des sulfates de chondroïtine et d'héparane de la membrane. Les complexes peptides-avidine peroxydase ne pénètre pas dans les cellules CHO-745 quel que soit le peptide testé.

Ceci démontre l'importance des chondroïtines héparane sulfate de la membrane cellulaire dans la pénétration des peptides.

### 2) Implication du métabolisme cellulaire.

Afin de comprendre les mécanismes biologiques par lesquels les peptides pénètrent dans les cellules et leur compartimentalisation, des expériences sont faites avec des drogues, dont l'action sur certains compartiments cellulaires est spécifique. Les peptide-péroxydase ont été incubés avec des cellules H1299 à une concentration de 0,2µg/ml pendant 2 heures en présence ou non de drogue. Les cellules ont été lysées et la peroxydase dosée dans le lysat cellulaire.

Le tableau 3 ci-dessous rapportent le pourcentage d'inhibition de l'internalisation des peptides calculé par rapport aux valeurs données par le lysat des cellules incubées sans drogue.

**Tableau 3**

| | Peptide SEQ ID NO : 26 (HBP3)₂ | Peptide SEQ ID NO : 30 (HBP7) | Peptide SEQ ID NO : 35 (HBP10) |
|---|---|---|---|
| la température (4°C) | 100 | 100 | 100 |
| l'azide de sodium (0,1%) | 70 | 70 | 50 |
| le chlorate de sodium (80mM) | 51 | 69 | 27 |
| le chlorure d'ammonium (50mM) | 76 | 70 | 30 |
| la genisteine (200µM) | 59 | 68 | 70 |
| la chloroquine (100µM) | 82 | 79 | 76 |

La pénétration des peptides testés est complètement inhibée par l'incubation des cellules à 4°C avec tous les peptides. Elle est inhibée en partie par des inhibiteurs du métabolisme cellulaire comme l'azide de sodium (inhibiteur de l'ATPase), la genisteine (inhibiteur de la tyrosine kinase et de la liaison à l'ATP). Le mécanisme d'internalisation est donc dépendant de l'énergie.

La pénétration des peptides HBP3 et HBP7 est inhibée d'un même ordre de grandeur par la chloroquine ce qui reflète une internalisation dans les mêmes compartiments du cytoplasme cellulaire (vésicules endosomiales, réticulum endoplasmique) alors que le chlorate de sodium et le chlorure d'ammonium (qui évite l'acidification des vésicules endosomiales inhibant ainsi le trafic intracellulaire) intervient beaucoup moins sur l'internalisation du peptide de séquence SED ID NO : 35. Ceci suggère que les peptides pénètrent dans les cellules par des mécanismes similaires à ceux utilisés par différentes toxines sans cependant présenter de toxicité. Les routes intracellulaires jusqu'à la zone du Golgi et le trafic rétrograde semblent distincts selon les peptides.

### III - RÉSULTATS.

### 1) Evaluation de la capacité des peptides à réagir avec l'ADN et avec l'héparine.

La capacité des peptides SEQ ID NO : 1 à 21 à se fixer sur l'ADN et l'héparine est évaluée sur des plaques ELISA sensibilisée avec de l'ADN ou de l'héparine. Des dilutions des peptides biotinylés sont déposées sur les plaques et leur fixation est mise en évidence à l'aide de streptavidine conjuguée à de la peroxydase. L'activité de la peroxydase est révélée avec de l'orthodianisidine et H₂O₂ comme substrat. L'avidité de chacun des peptides pour l'ADN ou l'héparine est évaluée par la quantité (x 10⁻⁶ M) de peptides nécessaire pour obtenir 50% de fixation. Les résultats obtenus sont rapportés dans le tableau 4 ci-dessous.

**Tableau 4**

| SEQ ID NO | ADN | Héparine | Chondroïtine A | Chondroïtine B | Chondroïtine C |
|---|---|---|---|---|---|
| 1 | >100 | >100 | >100 | >100 | >100 |
| 2 | 2,2 | 0,76 | 0,55 | 0,6 | 0,55 |
| 3 | 0,05 | 0,041 | 0,037 | 0,041 | 0,035 |
| 4 | 4 | 5 | 5,6 | 6,2 | 5 |
| 5 | 0,11 | 0,11 | 0,13 | 0,11 | 0,11 |
| 6 | 0,19 | 0,16 | 0,24 | 0,25 | 0,22 |
| 7 | 0,05 | 0,05 | 0,06 | 0,04 | 0,06 |
| 8 | >150 | >150 | >150 | >150 | >150 |
| 9 | 3,7 | 3,7 | 0,017 | 0,5 | >8 |
| 15 | 3 | >30 | 0,2 | 4 | 5 |
| 16 | 0,019 | 0,024 | 0,012 | 0,009 | 0,002 |
| 17 | >150 | >150 | >150 | >150 | >150 |
| 18 | >134 | >134 | >134 | >134 | >134 |
| 19 | >74 | >74 | >74 | >74 | >74 |
| 20 | 0,09 | 0,07 | 0,06 | 0,04 | 0,1 |
| 21 | 0,2 | 0,07 | 0,12 | 0,12 | 0,12 |

Il ressort de ce tableau que l'affinité de la SEQ ID NO : 1 pour l'ADN est très faible tandis que celle de son dimère (SEQ ID NO : 2) et de son trimère (SEQ ID NO : 3) est très grande. Il apparaît en outre que l'association de la SEQ ID NO : 1 avec des vecteurs peptidiques augmente considérablement l'affinité de ces vecteurs pour l'ADN [comparer les SEQ ID NO : 4 et 5, 6 et 7, 8 et 9, et 15 et 16].

De façon similaire, l'affinité de la SEQ ID NO : 17 et de la SEQ ID NO : 18 pour l'ADN, l'héparine et les chondroïtines sulfates est très faible. Par contre, l'affinité pour ces molécules du dimère de la SEQ ID NO : 18 (SEQ ID NO : 19) est plus grande. Les SEQ ID NO : 20 et 21 qui contiennent un plus grand nombre d'acides aminés parmi lesquels un nombre élevé d'acides aminés basiques possèdent une grande affinité pour l'ADN, l'héparine et les chondroitines sulfates.

L'avidité des peptides SEQ ID NO : 25 à 39 pour divers protéoglycanes (Kd x 10⁻⁹M) l'héparine et les sulfates de chondroïtines est évaluée par la quantité de peptides nécessaires pour obtenir 50% de fixation sur les différents antigènes. Les résultats sont rapportés dans le tableau 5 ci-dessous.

**Tableau 5**

| Peptides | Héparine | Chondroïtine A | Chondroïtine B | Chondroïtine C |
|---|---|---|---|---|
| (HBP1)₃ | 41 | 37 | 41 | 35 |
| HBP2 | 70 | 60 | 40 | 100 |
| (HBP3)₂ | 20 | 51 | 43 | 6 |
| (HBP₃)2RGD | 407 | nt | nt | nt |
| HBP6 | 16 | 135 | 110 | 12 |
| HBP7 | 54 | 78 | 58 | 19 |
| HBP8 | 168 | 225 | 260 | 110 |
| HBP9 | 45 | 47 | 47 | 25 |
| HBP10 | 78 | 112 | 229 | 39 |
| HBP11 | 382 | 287 | 301 | 205 |
| HBP12 | 295 | 176 | 173 | 93 |
| HBP13 | 56 | 56 | 38 | 16 |
| HBP15 | 100 | 100 | 100 | 100 |

Il ressort de ce tableau que l'avidité pour l'héparine des peptides SEQ ID NO : 33 (HBP8), 36 (HBP11) et 37 (HBP12) est nettement plus faible (>100x10⁻⁹M) que celle des autres peptides qui est en moyenne comprise entre 20 et 80x10⁻⁹M. L'avidité des différents peptides avec les sulfates de chondroïtines A, B, et C est dans l*'*ensemble du même ordre de grandeur que celle pour l'héparine. Ainsi l'avidité des peptides SEQ ID NO : 34 (HBP9) et SEQ ID NO : 38 (HBP13) aussi bien pour l'héparine et pour les 3 chondroïtines est 46-50 x10⁻⁹M, tandis que celle des peptides SEQ ID NO : 33 (HBP8), 36 (HBP11) et 37 (HBP12), aussi bien pour l'héparine que pour les 3 chondroïtines, est >100x10⁻⁹M. Le peptide modifié SEQ ID NO : 28 (HBP3 +RGD) a perdu son affinité pour l'héparine.

### 2) Évaluation de la pénétration peptides dans les cellules.

Les cellules sont cultivées en présence des différents peptides portant, du côté N-terminal, de la biotine à des concentrations décroissantes (50 à 6 µg/ml) dans le milieu de culture pendant des temps variables (1-18 heures).

A la fin de la culture, les cellules sont lavées trois fois avec du PBS (0,15 M NaCl tamponné avec 0,01 M tampon phosphate de potassium pH 7,4) puis fixées 15 minutes dans l'éthanol à -20°C. La pénétration du peptide est évaluée après incubation pendant 30 minutes avec de la streptavidine conjuguée à la peroxydase (en abrégé S-PO pour streptavidine-peroxydase). Ensuite, la S-PO est éliminée et les cellules sont lavées. L'activité de la peroxydase internalisée est révélée par la diaminobenzidine en présence d'H₂O₂ et les cellules sont examinées au microscope (**30**).

L'examen au microscope a montré que certains des peptides utilisés (SEQ ID NO : 1, 4, 11, 12, 17 et 18) ne donnent pas de coloration cytochimique positive intracellulaire. Avec les autres peptides, des colorations positives, mais d'intensité variable, ont été notées. Une faible coloration a été observée avec les SEQ ID NO : 2, 6, 8 et 19 tandis qu'avec les autres peptides des colorations intenses ont été obtenues.

Il est intéressant de noter que tous les peptides qui contiennent la SEQ ID NO : 1, c'est-à-dire le PLH de la lipoprotéine B humaine, donnent une coloration positive à l'exception de la SEQ ID NO : 1 elle-même (monomère) et de la SEQ ID NO : 11.

En ce qui concerne les SEQ ID NO : 1, 17 et 18, il s'agit de peptides courts qui correspondent aux PLH de la lipoprotéine B et la lipoprotéine E humaines, et de l'agrine. A la différence de ces peptides monomères, leurs dimères (SEQ ID NO : 2) et surtout le trimère de la SEQ ID NO : 1 (SEQ ID NO : 3) donnent des colorations nettement positives.

Pour ce qui est de la SEQ ID NO : 11, il est constitué de la SEQ ID NO : 1 et du CDR3 de l'anticorps monoclonal humain anti-ADN RT72 (codé par la lignée germinale) qui ne contient qu'un seul acide aminé basique (K). A la différence de la SEQ ID NO : 11, les SEQ ID NO : 5, 7, 9 et 10 constitués de la SEQ ID NO : 1 et des CDR2 et/ou CDR3 d'anticorps monoclonaux anti-ADN (codés par la lignée germinale), contiennent un nombre élevé d'acides aminés basiques, donnent des colorations intenses.

D'une part, ces résultats suggèrent qu'un minimum de 3 à 5 acides aminés basiques en excès doivent être présents dans la séquence des peptides pour que le peptide soit capable de pénétrer à l'intérieur des cellules. D'autre part, la SEQ ID NO : 1 n'est capable d'augmenter le pouvoir de translocation des CDR2 et/ou CDR3 anti-ADN que lorsque ces derniers possèdent un nombre élevé d'acides aminés basiques.

L'examen au microscope a montré que tous les peptides SEQ ID NO : 25 à 48 donnent des colorations cytochimiques positives à une concentration de 6 µg/ml, bien que d'intensité variable selon les peptides.

### 3) Evaluation de la pénétration dans les cellules des complexes peptide/streptavidine ou peptide/avitidine couplés à la peroxydase ou des conjugués peptides/peroxydase.

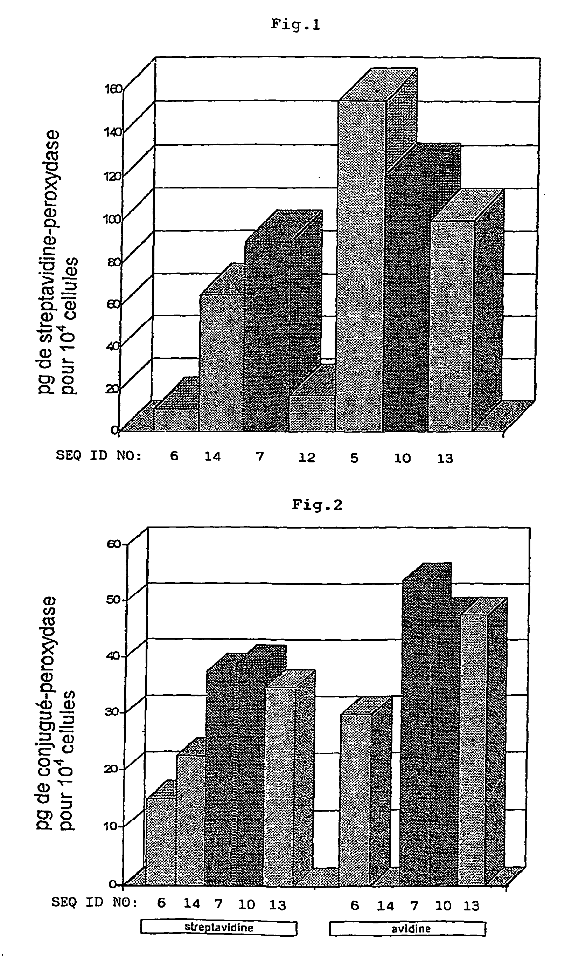

a) Un complexe peptide biotinylé/S-PO ou biotinylé/A-PO (A-PO pour avidine-peroxydase) est préparé extemporanément de deux façons :
- soit dans un rapport molaire peptide:S-PO ou A-PO de 4:1 (par exemple 1,4 µg de peptide de poids moléculaire 3500 pour 10 µg de S-PO ; le poids de S-PO utilisé varie selon les peptides puisque leurs poids moléculaires sont différents).
- soit dans un rapport molaire peptide : S-PO
ou A-PO de 25:1 (par exemple 1,7 µg de peptide de poids moléculaire 3500 pour 1 µg de S-PO).
Les complexes sont préparés en incubant le peptide biotinylé avec la S-PO ou la A-PO dans du PBS sous un volume de 5 à 10 µl pendant 15 minutes à la température du laboratoire. Ils sont dilués ensuite dans 1 ml de milieu complet de culture, filtrés sur membrane de 0,2 micron et incubés avec les cellules pendant 4 heures. Les cellules sont ensuite lavées trois fois avec du PBS.
Pour évaluer au microscope la pénétration des complexes, les cellules sont fixées comme précédemment pendant 15 minutes dans l'éthanol à -20°C, lavées au PBS, et l'activité de la peroxydase internalisée est révélée par la diaminobenzidine en présence d'H₂O₂.
Pour mesurer la quantité des complexes peptide/S-PO ou A-PO internalisée, les cellules sont trypsinisées, transférées dans des microtubes et comptées. Elles sont lavées deux fois par centrifugation puis le culot est mis en suspension dans 200 µl de tampon de lyse (tampon Tris, 0,1 M, pH8, 0,5% Nonidet). Après 15 minutes, la peroxydase contenue dans le tampon de lyse est dosée sur des plaques de 96 puits avec de l'ortho-dianisidine et H₂O₂ par comparaison avec une courbe étalon de S-PO. La lecture se fait à 450 nm. La quantité contenue dans les différents échantillons est exprimée en picogrammes (pg) pour 10⁴ cellules.
En général, les résultats obtenus en examinant les cellules au microscope optique sont en bonne corrélation avec les résultats obtenus en mesurant la peroxydase internalisée. C'est pour cette raison que, dans ce qui suit, seuls les résultats quantitatifs obtenus avec un certain nombre de peptides sont donnés dans le tableau 6 et 7 et les figures 1 à 3.
Le tableau 6 représente l'évaluation de la capacité de pénétration de peptides constitués de différents PLH, issus de protéines variées dans les cellules H1299, en utilisant des complexes peptide:S-PO de 4:1 et une concentration de peptide de 1 µg.

**Tableau 6**

| | Cellules H1299 (pg/10⁴ cellules) |
|---|---|
| SEQ ID NO : 1 | 3 |
| SEQ ID NO : 2 | 84 |
| SEQ ID NO : 3 | 642 |
| SEQ ID NO : 17 | 3 |
| SEQ ID NO : 18 | 3 |
| SEQ ID NO : 19 | 167 |
| SEQ ID NO : 20 | 431 |
| SEQ ID NO : 21 | 1680 |

Les résultats montrent clairement que, pour qu'un vecteur soit efficace dans le transfert intracellulaire du complexe S-PO (PM = 100.000), un minimum de 6 acides aminés basiques doit être présent (SEQ ID NO : 2, 3, 19-21).
Le tableau 7 ci-dessous rapporte les résultats de l'évaluation de l'influence de la SEQ ID NO : 1 sur la pénétration, dans les cellules H1299, de peptides couplés à la SEQ ID NO : 1, où :
Concentration a : 25 peptides pour une molécule S-PO (1 µg),
Concentration b : 4 peptides pour une molécule de S-PO (10 µg).

**Tableau 7**

| PEPTIDES N'INCLUANT PAS LA SEQ ID NO : 1 (pq/10⁴ cellules) | | PEPTIDES INCLUANT LA SEQ ID NO : 1 (pq/10⁴ cellules) | |
|---|---|---|---|
| SEQ ID NO : 4 | | SEQ ID NO: 5 | |
| conc. a | 2 | conc. a | 100 |
| conc. b | 280 | conc. b | 6250 |
| SEQ ID NO : 6 | | SEQ ID NO : 7 | |
| conc. a | 1 | conc. a | 240 |
| conc. b | 130 | conc. b | 220 |
| SEQ ID NO : 8 | | SEQ ID NO : 9 | |
| conc. a | 50 | conc. a | 340 |
| conc. b | 1310 | conc. b | 8280 |
| SEQ ID NO : 15 | | SEQ ID NO : 16 | |
| conc. a | 250 | conc. a | 430 |
| conc. b | 6670 | conc. b | 5220 |

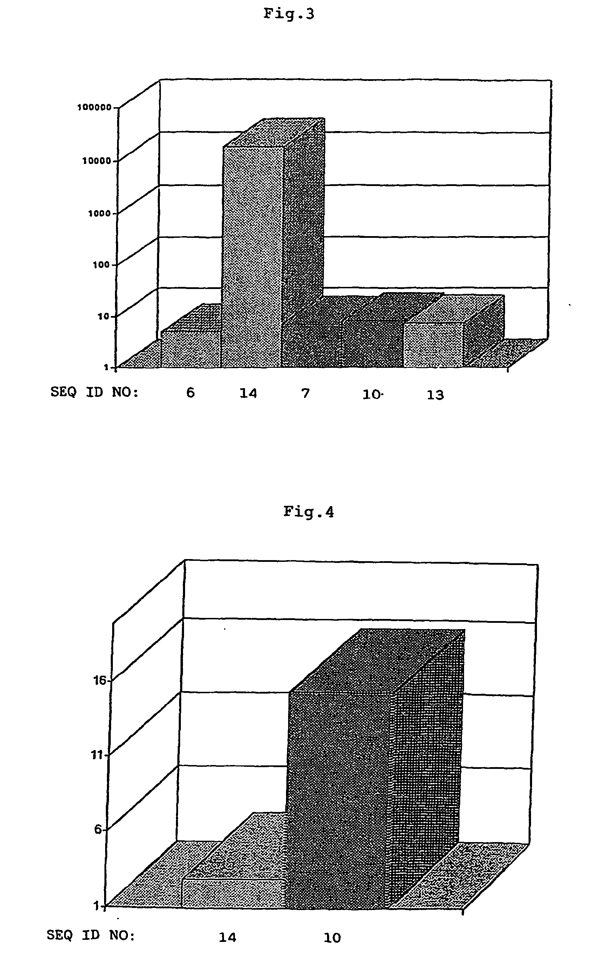

Il ressort du tableau 7 que le couplage de la SEQ ID NO : 1 avec des vecteurs peptidiques issus des anticorps anti-ADN humains ou murins potentialise considérablement le pouvoir de translocation de ces vecteurs.
La figure 1 est un graphique à barres illustrant l'internalisation de la streptavidine-peroxydase **transportée par divers peptides biotinylés une nuit à 37°C,** à la concentration a (0,5 nmole de peptide biotynilé pour 0,02 nmole de streptavidine-peroxydase).
Comme il ressort de cette figure 1, le couplage de la SEQ ID NO : 1 avec un vecteur donné (SEQ ID NO : 6), ce qui débouche sur la SEQ ID NO : 7, augmente significativement la capacité de transfert dudit vecteur. Cependant, le couplage, avec ce même vecteur (SEQ ID NO : 6), d'un peptide de même longueur que la SEQ ID NO : 1 et de proche composition en acides aminés, ce qui débouche sur la SEQ ID NO : 12, augmente à peine le pouvoir de translocation de ce vecteur. Même le couplage d'un second CDR3 identique sur ladite SEQ ID NO : 6, ce qui débouche sur la SEQ ID NO : 14, n'aboutit pas à une aussi forte augmentation que le couplage avec la SEQ ID NO : 1. En général, l'association de la SEQ ID NO : 1 à des vecteurs d'origine humaine (SEQ ID NO : 10 et 13) ou murin (SEQ ID NO : 5) donne naissance à des vecteurs particulièrement efficaces.
La figure 2 est un graphique à barres illustrant l'internalisation de la streptavidine-peroxydase (S-PO) et de l'avidine-peroxydase (A-PO) transportées par divers peptides biotinylés (2 heures à 37°C) à la concentration b (0,25 nmole de peptides biotinylés pour 0,01 nmole de S-PO ou de A-PO).
La figure 3 est un graphique à barres illustrant le rapport entre l'internalisation de la S-PO et celle de la A-PO par ces mêmes peptides biotinylés, 2 heures à 37°C, à la concentration b (0,25 nmole de peptide biotinylé pour 0,01 nmole de S-PO ou de A-PO).
La comparaison, d'après ces figures 2 et 3, du transfert de la S-PO et de la A-PO par les vecteurs suggère que la nature des substances à transférer influence plus la capacité de translocation des vecteurs d'origine totalement murine (SEQ ID NO : 14) que celle des vecteurs renfermant le PLH d'origine humaine (SEQ ID NO : 7, 10 et 13).
La figure 4 est un graphique à barres illustrant le rapport entre l'internalisation de la S-PO transportée par deux peptides biotinylés à 37°C pendant 2 heures et à 4°C pendant 2 heures.
Les résultats obtenus indiquent que le pouvoir de translocation du vecteur d'origine humaine (SEQ ID NO : 10) est beaucoup plus dépendant de la température (métabolisme actif des cellules) que celui du vecteur d'origine murine (SEQ ID NO : 14).
b) Pour évaluer d'une façon plus directe la pénétration des peptides en évitant le biais de l'avidine-peroxydase, certains des peptides de séquence SEQ ID NO : 25 à 48 ont été conjugués de façon covalente par l'intermédiaire de la cystéine en position C-terminal du peptide à de la péroxydase.

Le couplage des peptides avec la peroxydase a été réalisé de la façon suivante :
Un mg de peroxydase activée au maleimide (Sigma) dans 100µl de tampon phosphate de sodium, NaCl 0,15M, EDTA 5mM, est additionné de 1 mg de peptide. Après 2 heures à la température du laboratoire, le β-mercaptoethanol est ajouté à une concentration finale de 1,5 mM pendant 15 minutes. L'élimination du peptide non couplé se fait par centrifugation sur des membranes d'ultrafiltration (seuil de coupure = 30.000 daltons, Sartorius), suivie de trois lavages en tampon phosphate de sodium 0,1M, pH7,4 contenant 0,15M NaCl.

Les cellules H1299 sont incubées 2 heures avec les conjugués à des concentrations croissantes de peptide-péroxydase dans le milieu de culture puis lavées au PBS. Les cellules sont lysées et la quantité de peptide internalisé est évaluée par le dosage de la peroxydase dans le lysat des cellules en référence à une courbe établie avec le peptide-péroxydase.

La quantité de peptide internalisée (pg/10³ cellules) selon les concentrations de conjugué dans le milieu de culture est montrée dans le tableau 8 ci-dessous.

**Tableau 8**

| Concentration (µg/ml) | 0,01 | 0,1 | 0,5 | 1 |
|---|---|---|---|---|
| Peptide SEQ ID NO : 28 | 0,006 | 1,7 | 37,9 | 8860 |
| | (0,006)* | (1,6) | (7,6) | (8,8) |
| Peptide SEQ ID NO : 34 | 0,002 | 1,4 | 40,7 | 109 |
| | (0,01) | (1, 4) | (8,14) | (10,9) |

| | | | | |
|---|---|---|---|---|
| *: pourcentage de la quantité de peroxydase internalisée par rapport à la quantité déposée. | | | | |

Il apparaît que plus la concentration de conjugué peptide-péroxydase est élevée dans le milieu de culture, plus la quantité internalisée augmente.

Pour comparer la pénétration des conjugués selon le type cellulaire, les cellules de différentes lignées ont été incubées avec 0,2 µg/ml de conjugué peptide-péroxydase pendant 2 heures et la quantité de peroxydase dosée dans les lysats. Le tableau 9 et le tableau 9bis ci-après donnent les résultats obtenus exprimés en pg/10³ cellules. La quantité de chaque peptide internalisée dans les différentes lignées ne varie que du simple au double en général.

L'internalisation des peptides HBP3 (SEQ ID NO : 26) et HBP7 (SEQ ID NO : 32) en configuration D est du même ordre de grandeur que celle des peptides homologues en configuration L. Il en est de même avec le peptide de SEQ ID NO : 30 et son homologue de SEQ ID NO : 31, qui possède une séquence d'acides aminés en position recto-verso.

**Tableau 9 : Internalisation des peptide-péroxydase (pg/10³ cellules)**

| | Lignées cellulaires | | |
|---|---|---|---|
| Peptides | 3T3 | H1299 | HeLa |
| 1047 | 35 | 10 | 4.7 |
| (HBP1)3 | 24.6 | 17.7 | 11.2 |
| (HBP3)2 | 41 | 27.8 | 14.9 |
| HBP6 | 83.7 | 21.9 | 13.2 |
| HBP7 | 48.8 | 22.5 | 11 |
| HBP8* | 7.6 | 3.6 | 2.1 |
| HBP9* | 9 | 3.8 | 3.4 |
| HBP10 | 23.3 | 7.9 | 5.3 |
| HBP11 | 20.3 | 10.6 | 4.4 |
| HBP12* | 5.7 | 4.3 | 2.5 |
| HBP13 | 18.1 | 9.1 | 4.1 |

**Tableau 9bis : Internalisation des peptide-péroxydase (pg/10³ cellules)**

| | Lignées cellulaires | | |
|---|---|---|---|
| Peptides | 3T3 | H1299 | HeLa |
| (HBP3)₂ 2 | 72,9 | 67,4 | 11,4 |
| (HBP3)₂ Configuration D | 63,7 | 59,1 | 4,1 |
| HBP7 | 44,2 | 63,3 | 3,6 |
| HBP7 Configuration D | 50 | 56,7 | 7,7 |
| HBP7 recto verso | 75,9 | 70,6 | 8,7 |

| | | | |
|---|---|---|---|
| * à la limite de la méthode de dosage et par conséquent de la signification. | | | |

### 4) Evaluation de la pénétration des conjugués peptides-IgG dans les cellules

Deux mg d'anticorps IgG monoclonal ou de leur fragment F(ab')₂ dans 1 ml de tampon phosphate de sodium 0,1M, pH 7 contenant 0,15M NaCl sont additionnés de 200 µg de SMCC [4-(N-maléimidométhyl)cyclohexane-1-carboxylate de succinimidyle] dans 10 µl de diméthylsulfoxyde et la solution est incubée pendant 30 minutes à la température du laboratoire. L'élimination d'excès de réactif se fait par centrifugation sur des membranes d'ultrafiltration [seuil de coupure = 10.000 Daltons (Da), Vivascience], suivie de trois lavages en tampon phosphate de sodium 0,1M, pH 7 contenant 0,15M de NaCl.

Le couplage avec le peptide se fait ensuite dans un rapport molaire de 6 peptides pour 1 IgG dans le tampon phosphate de sodium, 0,1M, pH7, contenant 0,15M NaCl pendant 3 heures à la température du laboratoire. Ensuite l'excès de peptide non couplé est éliminé par centrifugation sur des membranes, comme à l'étape précédente.

Pour évaluer au microscope la pénétration des conjugués, les cellules sont cultivées en présence des différents conjugués anticorps-peptides à des concentrations décroissantes (50 à 6 µg/ml) dans le milieu de culture pendant 4 heures à 37°C.

A la fin de la culture, les cellules sont lavées trois fois avec du PBS, puis fixées 15 minutes dans l'éthanol à -20°C. La pénétration du conjugué IgG-peptide est évaluée après incubation pendant 1 heure avec un anticorps anti-IgG de souris couplé à la peroxydase. Les cellules sont ensuite lavées avec le PBS et l'activité de la peroxydase est révélée par la diaminobenzidine en présence d'H₂O₂.

Pour mesurer la quantité des conjugués peptides-IgG internalisée, les cellules sont trypsinisées, transférées dans des microtubes et comptées. Elles sont lavées deux fois par centrifugation puis le culot est mis en suspension dans 200 µl de tampon de lyse (tampon TRIS, O,1M, pH8, 0,5% Nonidet 40). La quantité d'IgG de souris présente dans le lysat est mesurée par ELISA sur des plaques enduites avec de l'anticorps de mouton anti-IgG de souris et révélées par un conjugué anticorps de mouton anti-IgG de souris couplé à la peroxydase, par référence à une courbe étalon établie avec le même anticorps monoclonal ayant été conjugué au peptide.

Les résultats sont regroupés dans le tableau 10, qui rapporte la pénétration d'anticorps monoclonaux murins ou de leurs fragments F(ab')₂ à l'intérieur de cellules humaines H1299, où :
a : anticorps monoclonal murin spécifique de la protéine p53, et
b : anticorps monoclonal murin spécifique de la protéine p21 issu d'un hybridome rat-rat.

**Tableau 10**

| Quantité d'anticorps internalisée | Conjugué SEQ ID NO: 10 IgG^{b} | Conjugué SEQ ID NO : 10 F(ab')₂^{b} | Conjugué SEQ ID NO : 10 F(ab')₂^{a} |
|---|---|---|---|
| pg/10⁴ cellules | 137 | 620 | 383 |

Comme il ressort de ce tableau, l'utilisation des vecteurs d'origine humaine associés à la SEQ ID NO : 1 permet une internalisation efficace d'anticorps dans des cellules humaines. Il est à noter aussi que de plus grandes quantités d'anticorps sont transférées dans les cellules quand les vecteurs sont conjugués au F(ab')₂ plutôt qu'à l'anticorps entier.

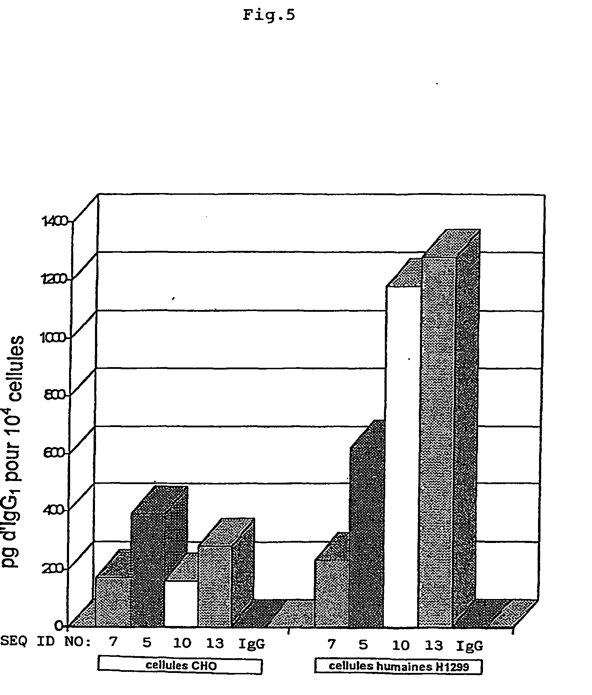

La figure 5 est un graphique à barres illustrant l'internalisation d'un anticorps monoclonal murin IgG₁ couplé à différents peptides (4 heures à 37°C ; 30 µg/ml de conjugué déposé) sur des cellules humaines H1299 et des cellules d'ovaires de hamster CHO.
Comme il ressort de ce graphique les vecteurs d'origine humaine (SEQ ID NO : 10 et 13) sont plus efficaces pour le transfert de substances à l'intérieur des cellules humaines que les vecteurs d'origine murine couplés à la SEQ ID NO : 1 (SEQ ID NO : 7 et 5). En outre, les vecteurs d'origine humaine (SEQ ID NO : 10 et 13) sont plus efficaces pour le transfert de substances à l'intérieur des cellules humaines qu'à l'intérieur des cellules de hamster.

### 5) Evaluation des capacités de pénétration intracellulaire des protéines recombinantes par immunofluorescence.

Les cellules Hela sont ensemencées à la concentration de 0,5 x 10⁴ cellules dans une plaque 24 puits contenant chacun une lamelle stérile. 24 heures après, 50µg/ml de protéine recombinante sont déposées sur les cellules. Six ou dix-huit heures après, les cellules sont lavées 2 fois en PBS (phosphate-buffered saline) et fixées par une solution 4% PFA (paraformaldehyde) en PBS pendant 10 min. à température ambiante ou dissociées par la trypsine et remises 18 heures en culture sur lamelle puis fixées. Après 3 lavages en PBS, les cellules sont perméabilisées par une solution 0.25% TritonX-100 en PBS pendant 5 min. à température ambiante. Après 3 lavage de 5 minutes dans du PBS, les cellules sont incubées 1 heure à température ambiante en chambre humide avec l'anticorps monoclonal anti-RGS.His (Qiagen) dilué au 1/50 dans du PBS-0.1%BSA (bovine serum albumin). Après trois lavages au PBS de 10 minutes, les lamelles sont incubées 30 minutes à température ambiante en chambre humide avec l'antisérum de chèvre anti-souris IgG conjugué FITC dilué au 1/200 dans du PBS-0.1% BSA. Les cellules sont lavées deux fois dans du PBS pendant 10 minutes puis les lamelles sont montées sur lame avec du liquide de montage (Mounting Medium from Sigma). Les cellules sont observées sous microscope confocal (Leica).

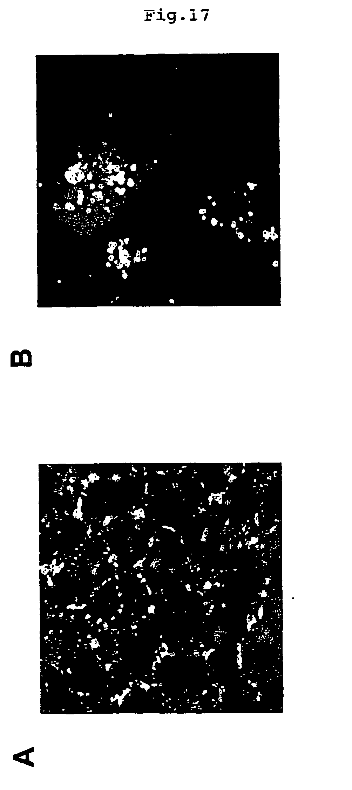

Les cellules Hela sont incubées 18 heures avec la protéine recombinante HiS6-Zebra-PAV1 et fixées (Figure 17A) ou dissociées et remises en culture 18 heures avant fixation (Figure 17B). L'observation des cellules au microscope confocal révèle que les cellules incubées 18 heures présentent à leur surface une fluorescence importante. Aucune fluorescence intracellulaire ne semble être détectée dans cette expérience. Cependant, une contre coloration des noyaux par DAPI ou HOECHST permettrait de l'affirmer. Il ne peut être exclu qu'une faible proportion de protéine ne soit internalisée. En outre, il apparaît qu'un unique motif peptidique PAV1 est suffisant pour adresser une protéine recombinante à la surface des cellules. Après dissociation des cellules, la fluorescence se localise dans des vésicules endosomales de différentes tailles. La protéine recombinante His₆-Zebra-PAV1 est internalisée mais ne semble pas être nucléaire malgré le domaine nls de la protéine Zebra.

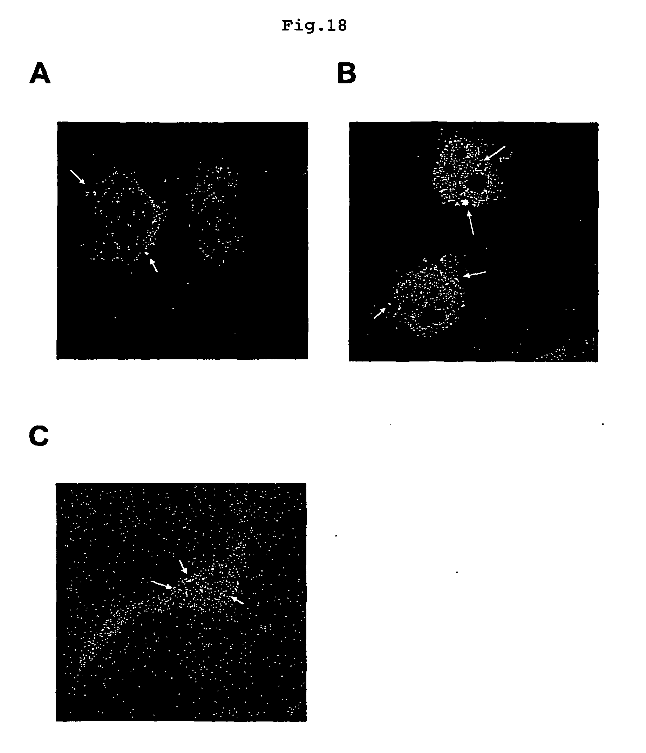

Les cellules Hela sont incubées 6 ou 18 heures avec la protéine recombinante His₆-ZebraΔnls-PAV1 et fixées (Figure 18A et 18B) ou dissociées après 18 heures et remises en culture 18 heures avant fixation (Figure 18C). Dès 6 heures, on observe une fluorescence nucléaire qui s'accentue à 18 heures. Des zones nucléaires indiquées par des flèches, présentent une fluorescence plus importante. Pour les cellules dissociées, une fluorescence plus marquée se localise autour des nucléoles. Contrairement à la protéine recombinante His₆-Zebra-PAV1, la protéine recombinante His₆-ZebraΔnls-PAV1 dépourvue du domaine nls a une localisation nucléaire. La séquence PAV1 semble adresser la protéine au noyau.

Pour les cellules Hela témoin (sans protéine) ou les cellules Hela incubées avec les protéines recombinantes His₆-Zebra et His₆-ZebraΔnls, il n'a pas été observé de fluorescence.

### 6) Evaluation in vitro de l'activité cytotoxique de la ribonucléase A (RNase A) conjuguée aux peptides.

5 mg de RNase A bovine sont dissous dans 1 ml de tampon phosphate de sodium 0,1M, pH 7 contenant 0,15 NaCl.

1 mg de SMCC est dissous dans 50 µl de diméthylsulfoxyde (solution finale 20 mg/ml).

L'activation de la RNase A se fait en ajoutant 12,5 µl de la solution de SMCC dans 200 µl de la solution de RNase A (rapport molaire = 10 molécules de SMCC pour 1 molécule de RNase A).

La réaction se fait pendant 30 minutes à la température du laboratoire.

L'élimination d'excès de réactif se fait par centrifugation sur des membranes d'ultrafiltration (seuil de coupure = 5.000 Da, Vivascience) suivie de 3 lavages avec un tampon 0,1 M phosphate de sodium contenant 0,15 M NaCl.

Le couplage avec le peptide se fait ensuite dans un rapport molaire peptides:RNase A de 6:1 dans le tampon 0,1 M phosphate de sodium, pH 7, contenant 0,15 M NaCl pendant 3 heures à la température ambiante.

L'excès de peptide non couplé est éliminé par centrifugation comme à l'étape précédente.

La quantité de peptide couplée à la RNase A est évaluée par le poids moléculaire des bandes obtenues après couplage sur un gel SDS-polyacrylamide à 15% en acrylamide.

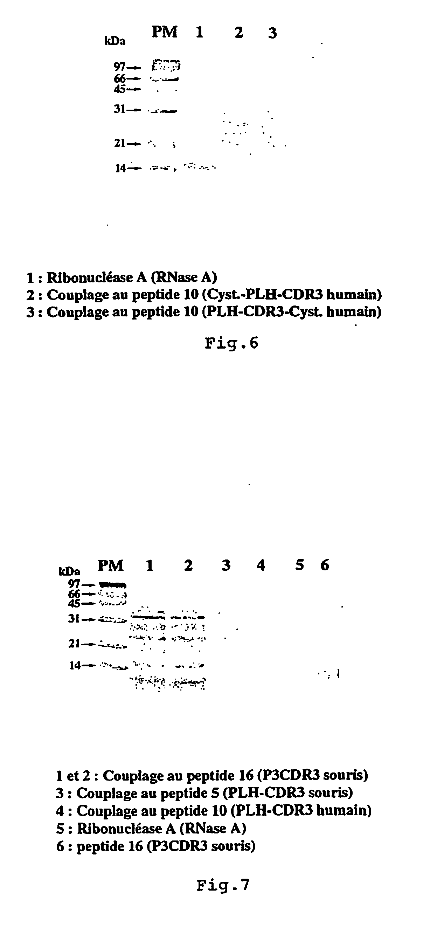

Le résultat de l'électrophorèse, comparant la RNase A seule (bande 1) à la RNase A couplée avec la SEQ ID NO : 10, est reproduit en figure 6 où sont présentés deux essais de couplage (bandes 2 et 3).

La figure 7 montre le résultat d'une électrophorèse, mise en oeuvre de même façon, et permettant de comparer la migration de la RNase A seule (bande 5) et celle de la SEQ ID NO : 14 seule (bande 6) avec celle du produit de couplage de la RNase A avec la SEQ ID NO : 14 (deux essais : bandes 1 et 2), avec la SEQ ID NO : 5 (bande 3) et avec la SEQ ID NO : 10 (bande 4).

La RNase A couplée à des vecteurs d'origine humaine (SEQ ID NO : 10, 15 et 16) a été testée pour son activité cytotoxique sur des cellules HH9 en culture par comparaison avec la RNase A native.

### a) Évaluation in vitro de l'activité cytotoxique de la Rnase sur des cellules HH9.

Les cellules sont ensemencées la veille dans des plaques de 96 puits à raison de 10³ cellules par puits.

Le lendemain, le surnageant est aspiré et remplacé par 100 µl de milieu contenant des dilutions successives de RNase A-vecteur ou de RNase A native et la culture poursuivie pendant 72 heures.

Les puits sont ensuite additionnés de 50 µl d'une solution de MTT (bromure de 3-(4,5-diméthylthiazol-2-yl)-2,5-diphényl tétrazolium) à 1 mg/ml dans le milieu de culture et cultivés pendant 4 heures.

Le surnageant est enlevé et les puits sont additionnés de 100 µl de diméthylsulfoxyde. Après dissolution des cristaux, la coloration est lue à 550 nm.

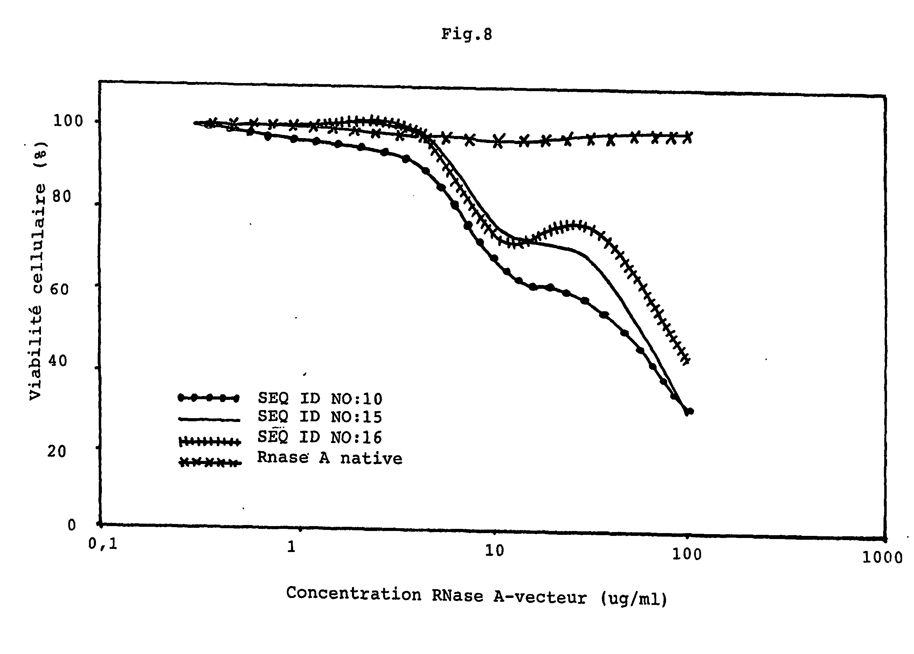

Les résultats sont calculés en pourcentage de la densité optique moyenne obtenue dans les puits de cellules contenant les dilutions des échantillons à tester et de la densité optique des puits n'ayant reçu que du milieu. Ces résultats sont représentés graphiquement à la figure 8.

Comme il ressort de la figure 8, la RNase A native n'a aucune activité cytotoxique. La cytotoxicité moyenne de 50% est obtenue à des concentrations de RNase A-vecteur comprises entre 33 et 100 µg/ml, c'est-à-dire entre les concentrations molaires de 2 à 7x10⁻⁷M.

### b) Evaluation in vitro de l'activité cytotoxique des conjugués peptide-RNase sur des cellules Hela.

Les différents conjugués peptide-RNase ont été testés sur des lignées cellulaires variées. Les résultats sont exprimés en IC50 qui correspond à la concentration du conjugué peptide-RNase donnant 50% d'inhibition de la croissance des cellules après 72 heures de culture.

Le tableau 11 résume les résultats obtenus. Il apparaît que tous les conjugués possèdent des activités cytotoxiques distinctes et que certains d'entre eux ont peu de pouvoir cytotoxique.

**Tableau 11**

| | Lignées Cellulaires | | | | | | | |
|---|---|---|---|---|---|---|---|---|
| Peptide-RNase | HT29 | H1299 | HH9 | HUVEC | 3T3 | MCF7 | HeLa | B16.F10 |
| (HBP1)3- | 70 | 100 | 20 | 80 | 40 | 90 | 60 | 25 |
| (HBP1)2- | nt* | 25 | nt | nt | nt | nt | nt | nt |
| HBP1-4- | nt | 25 | nt | nt | nt | nt | 6 | nt |
| HBP 3- | 10 | 12 | 2 | 15 | 3 | 15 | 7 | 12 |
| HBP6- | 45 | 3 | 3 | 50 | 3 | 20 | 12 | nt |
| HBP -7 | 45 | 50 | 2 | 50 | 6 | 25 | 15 | nt |
| HBP 8- | nt | nt | nt | nt | nt | nt | >100 | nt |
| HBP9- | nt | nt | 100 | nt | nt | nt | >100 | nt |
| HBP 10- | >100 | 150 | 33 | >100 | 50 | 150 | 25 | nt |
| HBP 11- | >100 | >100 | 60 | >100 | 60 | >100 | 20 | nt |
| HBP 12- | >100 | >100 | >100 | >100 | 40 | >100 | >100 | nt |
| HBP 13- | 40 | 100 | 8 | 65 | 14 | >100 | 3 | nt |
| HBP 14- | >100 | 250 | >100 | >100 | 55 | 90 | 25 | nt |
| HBP 1047- | >100 | 250 | 60 | >100 | 55 | 90 | 30 | >100 |

| | | | | | | | | |
|---|---|---|---|---|---|---|---|---|
| *nt : no | | | | | | | | |

### 7) Evaluation in vitro de l'activité cytotoxique de la RNase A conjuguée aux peptides sur des tumeurs humaines greffées sur la souris athymique (nude).

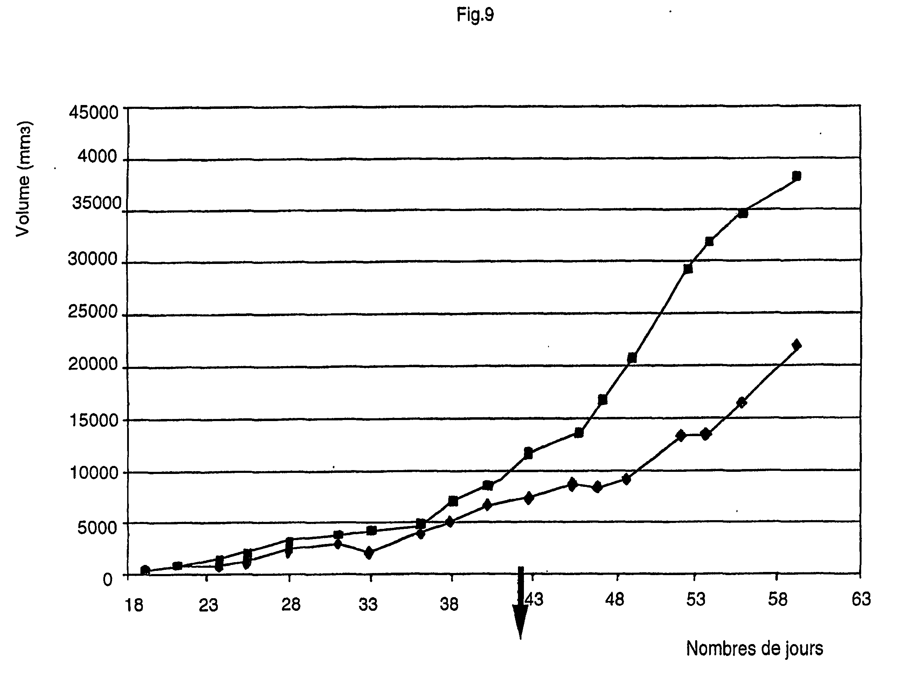

a) Protocole 1.
Des souris nudes femelles de 6 semaines ont été injectées en sous-cutané avec 3x10⁶ cellules HH9 sous un volume de 50 µl dans le flanc gauche. Au jour 17 après la greffe, les tumeurs sont mesurées et les souris sont divisées en 3 groupes : groupe 1 (7 souris) injecté avec 100 µg d'un couplage SEQ ID NO : 10/RNase A dans 50 µl de PBS, groupe 2 (7 souris) injecté avec 50 µg de RNase A native dans 100 µl de PBS, groupe 3 (6 souris) injecté avec 50 µl de PBS. Les injections sont poursuivies trois fois par semaine dans les mêmes conditions. A chaque fois, les tumeurs sont mesurées et leur volume est calculé selon la formule V = 4/3πxL²l (L>l) où L correspond au grand diamètre de la tumeur et l au petit diamètre de la tumeur.
Les résultats sont rapportés graphiquement à la figure 9.
Comme le montre cette figure, à partir du 33^{ème} jour, la progression du volume des tumeurs est nettement ralentie par la RNase A couplée à la SEQ ID NO : 10.
Il semblerait que la cytotoxicité soit plus importante pour certaines cellules tumorales que pour d'autres.
b) Protocole 2.
Les souris nues ont été greffées en sous-cutanée avec les cellules tumorales HH9 comme décrit précédemment. Le jour 9 après la greffe, les souris ont reçu 2 fois par semaine, en injection péritumorales, 100 µg de Rnase, ou 65 µg de peptide (HBP3)₂ ou 65 µg de peptide (HBP3)₂ + 100 µg de Rnase, ou 100 µg de conjugué peptide (HBP3)₂-RNase ou de conjugué HBP6-Rnase. Le jour 26, les souris ont été tuées et leur tumeur pesées.
L'inhibition de la croissance des tumeurs HH9 traitées par les conjugués peptide-RNase est donnée dans le tableau 12. L'inhibition de croissance des tumeurs des souris traitées est calculée par rapport au poids moyen des tumeurs de souris ayant reçu NaCl.

**Tableau 12**

| Groupe Rnase | NaCl | (HBP3)₂ | (HBP3)₂ +RNase | (HBP3)₂ -Rnase | HBP6-Rnase | HBP7-Rnase |
|---|---|---|---|---|---|---|
| Tumeurs* | 1720 | 1560 | 1500 | 790 | 810 | 830 |
| Inhibiti on** | - | 10 | 13 | 54 | 53 | 52 |

| | | | | | | |
|---|---|---|---|---|---|---|
| * poids moyen des tumeurs des 6 souris du même groupe en mg ** % | | | | | | |

Les 3 conjugués peptide-RNase inhibent la croissance des tumeurs de façon équivalente, alors que le peptide seul ou additionné de RNase a peu d'effet.

### 8) Transfection in vitro de cellules avec le plasmide de la luciférase à l'aide des peptides.

a) Transfection des cellules CHO.
Le plasmide utilisé est le PCMV-LUC (6,4kb) portant le gène de la luciférase sous le contrôle du promoteur du cytomégalovirus humain.
Les complexes plasmide-peptide sont préparés en additionnant 3 µg de plasmide dans 50 µl de NaCl, 0,15 M et 3,3 nmoles de SEQ ID NO : 22 et 23 pendant 15 minutes. Les complexes sont préparés en triplicata.
Des cellules CHO sont cultivées dans le milieu MEM Alpha complet contenant 6% de sérum de veau foetal et ensemencées à 5x10⁴ cellules par puits d'une plaque de 24 puits la veille de l'expérience. Les puits sont alors débarrassés du milieu et additionnés du complexe plasmide-peptide (3 µg de plasmide et 3,3 nmoles de peptide dans 0,5 ml de milieu complet pendant 5 heures à 37°C). Le milieu est alors remplacé par du milieu complet et la culture est poursuivie pendant 18-20 heures. Les cellules sont alors lavées trois fois avec du PBS puis lysées avec 100 µl de tampon de lyse (Promega) contenant 1% Triton X-100 et 2 mM DTT.
Il est alors prélevé 15 µl du lysat pour mesurer la luciférase avec le kit Promega luciférine et 15 autres µl pour calculer la quantité de protéines dans le lysat par le test de Bradford. Les unités relatives de luciférase (RLU) sont rapportées au mg de protéines.
Les résultats sont rapportés dans le tableau 13 ci-dessous.

**Tableau 13**

| | RLU 10⁶/mg de protéines |
|---|---|
| SEQ ID NO : 22 | 1,9 |
| SEQ ID NO : 23 | 11,5 |

Les résultats montrent que l'expression de la luciférase est possible en complexant le plasmide avec un des vecteurs et que cette expression est augmentée de 6 fois (SEQ ID NO : 23) par l'addition de PLH de l'apolipoprotéine à la SEQ ID NO : 22.
b) Transfection des cellules 3T3.
- Technique : Le plasmide pCMVLUC (portant le gène de la luciférase)fourni par Qiagen est utilisé selon leurs instructions. Des cellules 3T3 sont ensemencées la veille dans des plaques de 24 puits (8x10⁴ cellules/puits) dans du milieu de culture complet.
   Le peptides et le plasmide sont complexés dans un rapport de 1,6 nmoles/µg dans 50 µl de NaCl 0,15M pendant 20 minutes à la température du laboratoire. Les cellules sont alors lavées et le complexe peptide-plasmide ajouté sur les cellules dans 0,5ml de milieu complet. Après 5 heures d'incubation à 37°C, le milieu réactionnel est éliminé et 1 ml de milieu complet est ajouté aux cellules. Après 24 heures de culture, les cellules sont lavées alors 3 fois avec du PBS et lysées par 100 µl de tampon de lyse (Promega) contenant 1% de Triton X-100 et 2mM DTT pendant 15 minutes. Le test de la luciférase est effectué sur le lysat de cellules après centrifugation. La quantité de protéine dans le lysat est mesurée à l'aide du test de Bradford (Bio-Rad) et calculée selon une courbe établie avec des gamma globulines de boeuf. L'activité de la luciférase est estimée sur 15 µl de lysat additionné de 100 µl du réactif de la luciférase contenant de la luciférine (Promega). Les unités relatives (RLU) de luciférase sont mesurées dans un luminomètre (Berthold Systems) et rapportés aux mg de protéine.
- Résultats : La Quantité de luciférase exprimée par les cellules transfectées est montrée dans le tableau 14 ci-dessous.

**Tableau 14**

| pCMVLUC | seul | Peptide SEQ ID NO : 40 | Peptide SEQ ID NO : 41 | Peptide SEQ ID NO : 42 |
|---|---|---|---|---|
| RLU | 0,004* | 1,9 | 0,9 | 5 |

| | | | | |
|---|---|---|---|---|
| * x 10⁶/mg de protéines | | | | |

Le peptide complexé au plasmide permet son transfert à l'intérieur des cellules ainsi que l'expression du gène de la luciférase. L'efficacité de transfection exprimée par la quantité de luciférase (RLU/mg de protéines) varie selon les peptides utilisés. Le peptide SEQ ID NO : 42 et le peptide SEQ ID NO : 40 donnent des valeurs respectivement 5,5 fois et 2,6 fois plus élevées que le peptide SEQ ID NO : 41.

### 9) Evaluation de la pénétration des substances à l'aide de transporteurs couplés aux peptides.

Si l'on vient maintenant aux transporteurs selon l'invention, le tableau 15 ci-après donne quelques exemples de transporteurs et de substances d'intérêt pour lesquelles ils présentent une forte affinité.

**Tableau 15**

| TRANSPORTEURS | SUBSTANCES D'INTERET |
|---|---|
| Peptide - Protéine A | IgG humaines ou de lapin |
| Peptide - Protéine G | IgG bovines, de chèvre ou de souris |
| Peptide - F(abl)2 anti-IgG | Toutes IgG d'une même espèce |
| Peptide - IgG anti-peroxydase | Peroxydase et toute molécule contenant de la peroxydase |
| Peptide - F(abl)2 anti-RNase | Ribonucléase A |
| Peptide - Concanavaline A | Un grand nombre de glycoprotéines |
| Peptide - Streptavidine - Avidine | Biotine et toute molécule contenant de la biotine |

Dans le tableau ci-dessus, le terme générique "peptide" signifie "séquence d'acides aminés selon l'invention".

La protéine A est une protéine isolée à partir de *Staphyloccocus aureus* qui a un poids moléculaire de 42.000 Da. La propriété caractéristique de cette protéine est sa grande affinité pour la partie Fc des IgG. Cette propriété lui permet de fixer 2 à 4 molécules d'IgG sans interagir avec les sites actifs des anticorps. La protéine A est intéressante pour les IgG humaines, de lapin et de rat, et certaines IgG de souris.

La protéine G, à l'origine, est une protéine de 30.000 à 35.000 Da isolée à partir de la paroi cellulaire des souches bactériennes C ou D de streptocoques. La protéine G commercialisée par SIGMA est une protéine recombinante de 17.000 Da qui contient deux domaines de fixation des IgG. Comme la protéine A, la protéine G possède une forte affinité pour la fraction Fc des IgG, mais elle sera surtout utilisée pour les IgG bovines, de souris et de mouton.

Les F(ab')₂ proviennent d'anticorps. On utilisera comme exemples des IgG de mouton anti-IgG de souris obtenues à partir d'un immun sérum de mouton. Elles sont purifiées par une chromatographie d'affinité, dans laquelle la phase solide est constituée d'IgG de souris fixées sur des billes de polyacrylamide-agarose. C'est une purification par un immunoabsorbant qui permet la fixation spécifique des IgG de mouton anti-IgG de souris par une réaction antigène-anticorps. Cette liaison antigène-anticorps est dissociée par un pH acide. La digestion ménagée des IgG par des enzymes protéolytiques permet d'obtenir des fragments d'anticorps différents. A l'aide de la pepsine, on obtient des fragments F(ab')₂ bivalents, de taille réduite (PM=92.000 Da), et des fragments Fc totalement digérés en petits polypeptides. La suppression du fragment Fc permet de réduire la taille des anticorps et simplifie le couplage avec les peptides. La réalisation d'un transporteur peptide-F(ab')₂ anti-IgG permettra d'internaliser en forte quantité une grande variété d'IgG de souris.

L'IgG anti-peroxydase est un anticorps monoclonal d'isotype IgG1 qui est isolé à partir d'ascite de souris. Il est purifié par une chromatographie d'affinité sur protéine G (GammaBind Plus Sepharose : Pharmacia). Cet anticorps présente une forte affinité pour la peroxydase, et par la même, pour toutes les molécules contenant de la peroxydase. La peroxydase (PO) est une enzyme extraite du radis noir ayant un poids moléculaire de 44.000 Da, qui peut être associée très facilement à de nombreuses molécules. La réalisation d'un transporteur peptide-IgG anti-PO permettra d'internaliser, dans les cellules, de la PO et toute substance contenant de la PO.

La concanavaline, extraite de *Canavalia ensiformis,* est une lectine qui présente une affinité pour les groupements α-D-mannosyle et α-D-glucosyle terminaux des protéines. Cette propriété lui permet de réagir avec de nombreuses glycoprotéines. La réalisation d'un transporteur peptide-concanavaline permettra d'internaliser, dans les cellules, de nombreuses glycoprotéines ou molécules glycolisées.

La streptavidine, molécule de 60.000 Da extraite de *Streptomyces avidinii,* et l'avidine, extraite du blanc d'oeuf, ont toutes deux quatre sites de reconnaissance pour la biotine. Leur constante d'affinité pour cette petite molécule est très élevée (KD =10⁻¹³M) ce qui leur permet d'interagir avec toutes les substances contenant de la biotine. La réalisation d'un transporteur peptide-streptavidine ou peptide-avidine permettra d'internaliser, dans les cellules, de la biotine et toute molécule contenant de la biotine.

Les exemples détaillés ci-dessous illustrent la réalisation et l'utilisation de tels transporteurs.
a) Evaluation de la pénétration de la ribonucléase A bovine (RNase A) transportée par un transporteur peptide-F(ab')₂, anti-RNase dans les cellules.
Une digestion ménagée des IgG anti-RNase par une enzyme protéolytique, la pepsine, permet l'obtention des fragments F(ab')₂. Cette digestion, qui s'effectue dans une solution d'acétate de sodium pH 4,5 avec 2% de pepsine, permet la fragmentation du fragment Fc des IgG sans altérer les sites actifs de l'anticorps.
Deux mg d'anticorps F(ab')₂ anti-RNase dans 1 ml de tampon phosphate de potassium 0,1 M pH 7,4 sont additionnés à 110 µg de SMCC [4-(N-maléimidométhyl)cyclohexane-1-carboxylate de succinimidyle] dans 11 µl de diméthylsulfoxyde. La solution est incubée pendant 45 minutes à la température du laboratoire. L'élimination d'excès de réactifs se fait par centrifugation sur des membranes d'ultrafiltration (seuil de coupure = 10.000 Da, Vivascience), suivie de trois lavages en tampon phosphate de sodium 10 mM, pH 7 contenant 0,5 M de NaCl et 5 mM d'EDTA.
Le couplage avec le peptide se fait ensuite dans un rapport molaire de 6 peptides pour 1 F(ab')₂ dans le tampon phosphate de sodium 10 mM, pH 7, contenant 0,5 M de NaCl et 5 mM d'EDTA pendant trois heures à la température du laboratoire. Ensuite, l'excès de peptide non couplé est totalement éliminé par centrifugation sur des membranes d'ultrafiltration, comme à l'étape précédente.
Ces transporteurs [peptide-F(ab')₂ anti-RNase] sont additionnés à la ribonucléase A dans du milieu de culture complet et la solution est incubée pendant 1 heure à la température du laboratoire. Le rapport molaire de cette réaction est de 2 RNase/1 F(ab')₂.
Pour évaluer au microscope la pénétration de la RNase A, les cellules sont cultivées en présence des complexes [peptide-F(ab')₂ anti-RNase]-RNase A dilués à des concentrations décroissantes de RNase A (de 100 à 6 µg/ml) dans du milieu de culture pendant 4 heures à 37°C.
A la fin de l'incubation, les cellules sont lavées trois fois avec du PBS, puis fixées 15 minutes dans de l'éthanol absolu à -20°C. La pénétration de la RNase est évaluée après incubation pendant 1 heure avec un anticorps anti-RNase couplé à la peroxydase. Les cellules sont ensuite lavées avec du PBS et l'activité de la peroxydase est révélée par la diaminobenzidine en présence d'H₂O₂.
Pour mesurer la quantité de RNase A internalisée, les cellules sont trypsinisées, transférées dans des microtubes et comptées. Elles sont lavées deux fois par centrifugation avec du PBS puis le culot est mis en suspension dans 220 µl de tampon de lyse (tampon tris, 0,1M, pH 8, 0,5% Nonidet 40). La quantité de RNase A présente dans le lysat cellulaire est mesurée par ELISA sur des plaques sensibilisées avec de l'anticorps de lapin anti-RNase A et révélées par un conjugué anticorps de lapin anti-RNase A couplé à la peroxydase, par référence à une courbe étalon établie avec la RNase.
Les résultats regroupés dans le tableau 16, illustrent l'internalisation de la RNase A par le transporteur F(ab')₂ anti-RNase et l'internalisation de la RNase couplée de façon covalente au peptide à l'intérieur des cellules HeLa.

**Tableau 16**

| | **Transporteur + RNase A** | **Peptide-RNase A** | **RNase native** |
|---|---|---|---|
| Quantité de Rnase internalisée (pg/10⁴ cellules) | 476 | 433 | 0 |

b) Evaluation de la pénétration des IgG de souris (IgG) transportées par un transporteur peptide-F'(ab')₂, anti-IgG de souris dans les cellules.
Les transporteurs peptide-F(ab')₂ anti-IgG de souris sont obtenus selon la technique décrite précédemment.
Ces transporteurs [peptide-F(ab')₂ anti-IgG] sont additionnés aux IgG de souris dans du tampon NaCl 0,5 M. La solution est incubée pendant 1 heure à la température du laboratoire. Le rapport molaire de cette réaction est de 1 F(ab')₂/1 IgG. Après une heure d'incubation, les complexes formés sont dilués dans du milieu de culture complet.
Pour évaluer au microscope la pénétration des IgG, les cellules sont cultivées en présence des complexes [peptide-F(ab')₂ anti-IgG]-IgG dilués à des concentrations décroissantes d'IgG (de 100 à 6 µg/ml) dans du milieu de culture complet pendant 4 heures à 37°C.
A la fin de l'incubation, les cellules sont lavées trois fois avec du PBS, puis fixées 15 minutes dans de l'éthanol absolu à -20°C. La pénétration des IgG est évaluée après incubation pendant 1 heure avec un anticorps anti-IgG couplé à la peroxydase. Les cellules sont ensuite lavées avec du PBS et l'activité de la peroxydase est révélée par la diaminobenzidine en présence d'H₂O₂.
Pour mesurer la quantité d'IgG internalisée, les cellules sont trypsinisées, transférées dans des microtubes et comptées. Elles sont lavées deux fois par centrifugation avec du PBS puis le culot est mis en suspension dans 220 µl de tampon de lyse (tampon tris 0,1M, pH 8, 0,5% Nonidet 40). La quantité d'IgG présente dans le lysat cellulaire est mesurée par ELISA sur des plaques sensibilisées avec de l'anticorps de mouton anti-IgG et révélée par un conjugué anticorps de mouton anti-IgG couplé à la peroxydase, par référence à une courbe étalon établie avec les IgG. L'activité de la peroxydase est révélée avec de l'orthodianisidine et H₂O₂.
Un même transporteur [peptide-F(ab')₂ anti-IgG] peut transporter une grande variété d'IgG de souris à l'intérieur des cellules.
Les résultats regroupés dans le tableau 17, illustrent l'internalisation des différentes IgG de souris ou éventuellement de rat à l'intérieur des cellules HeLa par l'intermédiaire du même transporteur [peptide-F(ab')₂ anti-IgG].

**Tableau 17**

| | Quantité d'IgG internalisée (pg/10⁴ cellules) |
|---|---|
| Transporteur + IqG anti-p53 | 1100 |
| Transporteur + IqG anti-p21 | 1200 |
| Transporteur + IqG anti-PO | 695 |
| Transporteur + IqG biotinylées | 153 |
| Transporteur + IqG souris | 5960 |
| Transporteur + F.4.1 | 3200 |
| F.4.1. | 1299 |

| | |
|---|---|
| IgG anti-p53 : anticorps monoclonal murin spécifique de la protéine p53. IgG anti-p21 : anticorps monoclonal spécifique de la protéine p21 issu d'un hybridome rat-rat, réagissant avec les anticorps anti-IgG de souris. IgG anti-PO : anticorps monoclonal murin spécifique de la peroxydase F. 4.1 : anticorps monoclonal murin anti-ADN qui à lui seul pénètre à l'intérieur des cellules. | |

c) Evaluation de la pénétration de la peroxydase (PO) ou de molécules contenant la Peroxydase transportées par un transporteur peptide-IgG anti-PO dans les cellules.
Deux mg d'anticorps IgG monoclonal spécifique de la peroxydase ou de leur fragment F(ab')₂ dans 1 ml de tampon phosphate de potassium 0,1 M, pH 7,4 sont additionnés à 110 µg de SMCC [4-(N-maléimidométhyl)cyclohexane-1-carboxylate de succinimidyle] dans 11 µl de diméthylsulfoxyde. La solution est incubée pendant 45 minutes à la température du laboratoire. L'élimination de l'excès de réactifs se fait par centrifugation sur des membranes d'ultrafiltration (seuil de coupure = 10.000 Da, Vivascience), suivie de trois lavages en tampon phosphate de sodium 10 mM, pH 7 contenant 0,5 M de NaCl et 5 mM d'EDTA.
Le couplage avec le peptide se fait ensuite dans un rapport molaire de 6 peptides pour 1 IgG ou 1 F(ab')₂ dans le tampon phosphate de sodium 10 mM, pH 7, contenant 0,5 M de NaCl et 5 mM d'EDTA pendant trois heures à la température du laboratoire. Ensuite, l'excès de peptide non couplé est totalement éliminé par centrifugation sur des membranes d'ultrafiltration, comme à l'étape précédente.
Ces transporteurs [peptide-IgG anti-PO] sont additionnés à la peroxydase ou à la peroxydase biotinylée dans du tampon 0,5 M NaCl. La solution est incubée pendant 1 heure à la température du laboratoire. Le rapport molaire de cette réaction est de 2 PO / 1 IgG ou F(ab')₂.
Pour évaluer au microscope la pénétration de la PO ou des molécules contenant la PO, les cellules sont cultivées en présence des complexes [peptide-IgG anti-PO]-PO à des concentrations décroissantes de PO ou de molécules contenant la PO (100 à 6 µg/ml) dans du milieu de culture pendant 4 heures à 37°C.
A la fin de la culture, les cellules sont lavées trois fois avec du PBS, puis fixées pendant 15 minutes dans de l'éthanol absolu à -20°C. Les cellules sont ensuite lavées avec du PBS et la pénétration de la PO ou des molécules contenant la PO est évaluée par l'activité de la peroxydase révélée par la diaminobenzidine en présence d' H₂O₂
Pour mesurer la quantité de PO internalisée, les cellules sont trypsinisées, transférées dans des microtubes et comptées. Elles sont lavées deux fois par centrifugation avec du PBS puis le culot est mis en suspension dans 220 µl de tampon de lyse (tampon tris 0,1 M, pH 8, 0,5% Nonidet 40). La quantité de PO présente dans le lysat cellulaire est mesurée par ELISA sur des plaques sensibilisées avec de l'anticorps de souris anti-peroxydase et révélées avec de l'ortho-dianisidine et H₂O₂ par référence à une courbe étalon de PO.
Les résultats regroupés dans le tableau 18, illustrent l'internalisation de la PO et de la PO-biotinylée à l'intérieur des cellules HeLa par l'intermédiaire du transporteur [peptide-IgG anti-PO].

**Tableau 18**

| | Peroxydase (PO) | PO-biotinylée |
|---|---|---|
| Quantité de molécules internalisées (pg/10⁴ cellules) | 2100 | 1700 |

### d) Evaluation de la pénétration d'IgG transportées par un transporteur peptide-protéine A dans les cellules.

Un mg de protéine A dans 0,5 ml de tampon phosphate de sodium 0,1 M pH 7 est additionné à 120 µg de SMCC [4-(N-maléimidométhyl)cyclohexane-1-carboxylate de succinimidyle] dans 12 µl de diméthylsulfoxyde. La solution est incubée pendant 45 minutes à la température du laboratoire. L'élimination de l'excès de réactifs se fait par centrifugation sur des membranes d'ultrafiltration (seuil de coupure = 10.000 Da, Vivascience), suivie de trois lavages en tampon phosphate de sodium 0,1 M, pH 7 contenant 0,15M de NaCl.

Le couplage avec le peptide se fait ensuite dans un rapport molaire de 6 peptides pour 1 protéine A dans le tampon phosphate de sodium 0,1 M, pH 7, contenant 0,15 M de NaCl pendant trois heures à la température du laboratoire. Ensuite, l'excès de peptide non couplé est totalement éliminé par centrifugation sur des membranes d'ultrafiltration, comme à l'étape précédente.

Ces transporteurs [peptide-protéine A] sont additionnés à des IgG dans du tampon 0,15M NaCl. La solution est incubée pendant 20 minutes à la température du laboratoire. Le rapport molaire de cette réaction est de 2 IgG/1 protéine A.

Pour évaluer au microscope la pénétration des IgG, les cellules sont cultivées en présence des complexes [peptide-protéine A]-IGG dilués à des concentrations décroissantes d'IgG (100 à 6 µg/ml) dans du milieu de culture, pendant 4 heures à 37°C.

A la fin de la culture, les cellules sont lavées trois fois avec du PBS, puis fixées pendant 15 minutes dans de l'éthanol absolu à -20°C. La pénétration des IgG est évaluée après incubation pendant 1 heure avec un anticorps anti-IgG couplé à la PO ou avec de la PO seule pour les IgG anti-PO. Cette étape n'est pas nécessaire lors de l'internalisation d'IgG conjuguées à la PO (par exemple : IgG lapin-PO). Les cellules sont ensuite lavées avec du PBS et l'activité de la peroxydase est révélée par la diaminobenzidine en présence d'H₂O₂.

Pour mesurer la quantité d'IgG internalisée, les cellules sont trypsinisées, transférées dans des microtubes et comptées. Elles sont lavées deux fois par centrifugation avec du PBS puis le culot est mis en suspension dans 220 µl de tampon de lyse (tampon tris 0,1M, pH 8, 0,5% Nonidet 40). La quantité d'IgG présente dans le lysat cellulaire est mesurée par ELISA sur des plaques sensibilisées avec de l'anticorps anti-IgG et révélée par un conjugué anticorps anti-IgG couplé à la peroxydase, par référence à une courbe étalon établie avec les IgG. L'activité de la PO est révélée avec de l'ortho-dianisidine et H₂O₂. Les résultats regroupés dans le tableau 19, illustrent l'internalisation de différentes IgG à l'intérieur des cellules HeLa par l'intermédiaire du transporteur [peptide-protéine A].

**Tableau 19**

| | IgG lapin-PO | IgG souris anti-PO | IVIg |
|---|---|---|---|
| Quantité d'IgG internalisée (pg/10⁴ cellules) | 10800 | 480 | 26000 |

### 10) Transport de l'anticorps anti-CEA dans les cellules de cancer du colon.

a) Des expériences ont été réalisés avec l'Ac anti-CEA 35A7 et les peptides de séquence SEQ ID NO : 10, SEQ ID NO : 30 et SEQ ID NO : 35 aussi désignés respectivement 1047, HBP7 et HBP10.
   Le peptide 1047 a été couplé au 35A7 et à l'Herceptin^{®}. Après contrôle des activités anticorps des conjugués par ELISA, l'internalisation a été étudiée par immunofluorescence en comparaison avec l'anticorps de départ à 37°C et 4°C. Sur les cellules LS174T, carcinome colique humain CEA+, le conjugué 35A7-1047 internalise dès 1 h 15 alors que le 35A7 ne montre aucune internalisation jusqu'à 5 h. Sur cellules SKOv3, carcinome ovarien humain ErbB2+, le conjugué Herceptin^{®}-1047 présente une internalisation rapide proche de celle obtenue avec l'Herceptin^{®}. Après radiomarquage avec ¹²⁵I, les conjugués montrent une immunoréactivité proche de l'Ac de départ sur Ag immobilisé sur Sepharose. Une analyse par gel filtration démontre l'absence d'agrégat.
b) Comparaison des peptides 1047, HBP7 et HBP10. Cette comparaison ne concerne que l'Ac 35A7.
   - Expériences *in vitro*
      Une étude cinétique de l'internalisation des différents conjugués (35A7-1047, 35A7-HBP7 et 35A7-HBP10) sur cellules LS174T montre une internalisation plus importante avec le peptide HBP7 qu'avec le 1047 et le HBP10. Ces deux peptides donnant des résultats comparables. Sur cellules SKOv3, CEA-, 1047 et HBP7 induisent une internalisation comparable à celle obtenue sur LS174T alors que HBP10 donne un résultat diminué de moitié.
   - Expériences *in vivo*
      Les 3 conjugués 35A7-1047, 35A7-HBP7 et 35A7-HBP10 marqués avec ¹²⁵I ont été étudiés chez des souris nues porteuses de tumeurs LS174T en comparaison avec le 35A7 marqué. Le ¹³¹I-35A7 montre une captation tumorale d'environ 13% de la dose injectée par gramme (%ID/g) 6 h post-injection. Cette captation atteint entre 20 et 25%ID/g de tumeur 24 h post-injection. Le conjugué ¹²⁵I-35A7-1047 montre une élimination un peu plus rapide et une captation tumorale légèrement diminuée par rapport au ¹³¹I-35A7 (18.6 vs 21.2 %ID/g à 24 h). Le conjugué ¹²⁵ I-35A7-HBP7 montre une captation hépatique importante dès 6 h. Cela engendre une élimination rapide et une faible captation tumorale (8.5 %ID/g à 24 h). Le conjugué ¹²⁵I-35A7-HBP10, avec une biodistribution très proche de celle de l'anticorps natif, montre la meilleure captation tumorale des 3 conjugués.
c) L'effet d'induction de l'internalisation par le peptide 1047 est particulièrement visible sur le 35A7, Ac dirigé contre le CEA qui est un Ag qui n'internalise pas. L'effet est moins évident sur l'Herceptin^{®} qui internalise rapidement après fixation à son Ag, ErbB2.
   Les expériences *in vitro* réalisées avec 1047, HBP7 et HBP10 montrent que les trois peptides induisent une internalisation du 35A7. HBP7 semble être le plus actif mais HBP10 est celui qui semble le mieux respecter la spécificité de l'anticorps (effet marqué sur cellules CEA+, effet diminué sur cellules CEA-). Les expériences *in vivo* confirment les résultats obtenus *in vitro*.

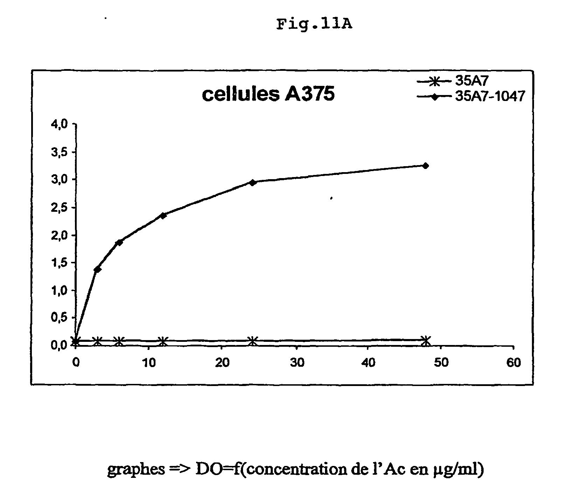

d) Les figures 11 à 16 en annexe illustrent les résultats ci-dessus.
   - Les peptides :
      - 1047 : 2420 Da => sur Ac anti-ACE et anti-erbB2
      - HBP7 : 1827 Da => sur Ac anti-ACE seulement
      - HBP10 : 1696 Da => sur Ac anti-ACE seulement
   - Le Couplage :
      Après mise au point, les conditions définies pour un couplage (en évitant la formation d'agrégats d'Anticorps) sont : 18 SMCC/Ig et 12 pept/Ig-SMCC
   - Essai d'internalisation sur cellules en cultures :
      - Les figures 11 A et B représentent le test 1 : sur cellules A375 (ACE négatives) et 5F12 (ACE positives), 40 000 cellules par puits (boîte 24puits). Incubation avec l'Anticorps (Ac) natif : 35A7 versus Ac conjugué : 35A7-1047, 4h à 37°C. Lavages, fixation, incubation avec Ac secondaire-Péroxidase, révélation OPD, lecture 490nm. DO en fonction de la concentration de l'Anticorps en µg/ml.

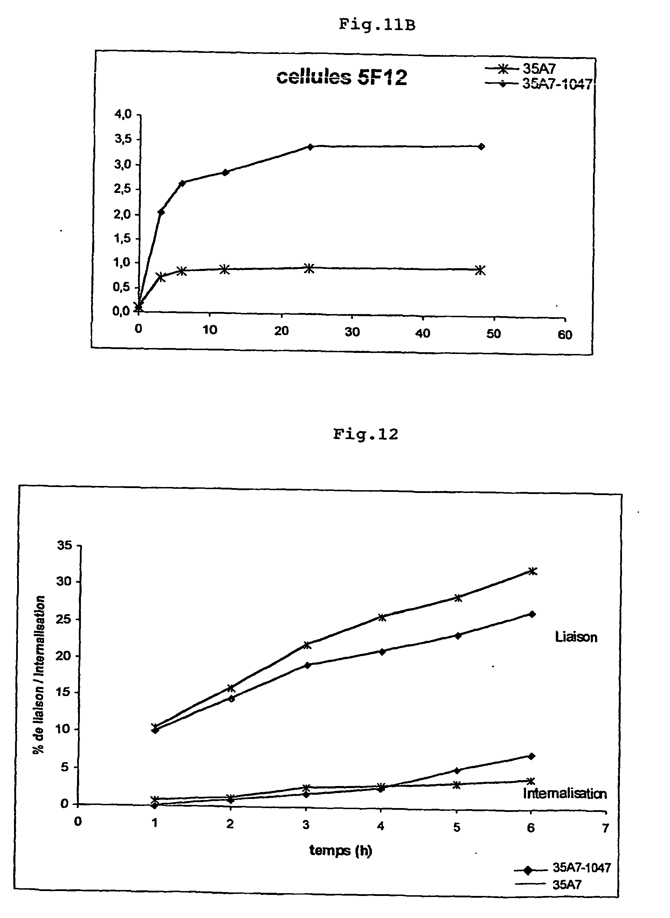

      - La figure 12 représente l'essai du test 1 avec les anticorps marqués à l'iode 125.

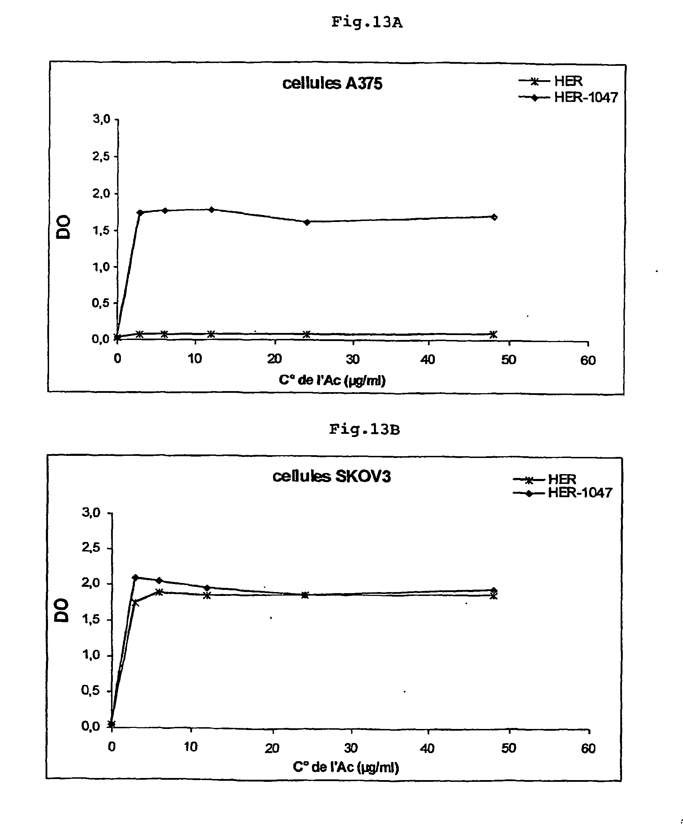

      - Les figures 13 A et B représentent le test **2 : sur cellules A375 (erbB2 négatives) et SKOV3 (erbB2** positives), 40 000 cellules par puits (boîte 24puits). Incubation avec l'Anticorps (Ac) natif : Herceptine versus Ac conjugué : HER-1047, 4h à 37°C. Lavages, fixation, incubation avec Ac secondaire-Péroxidase, révélation OPD, lecture 490nm.

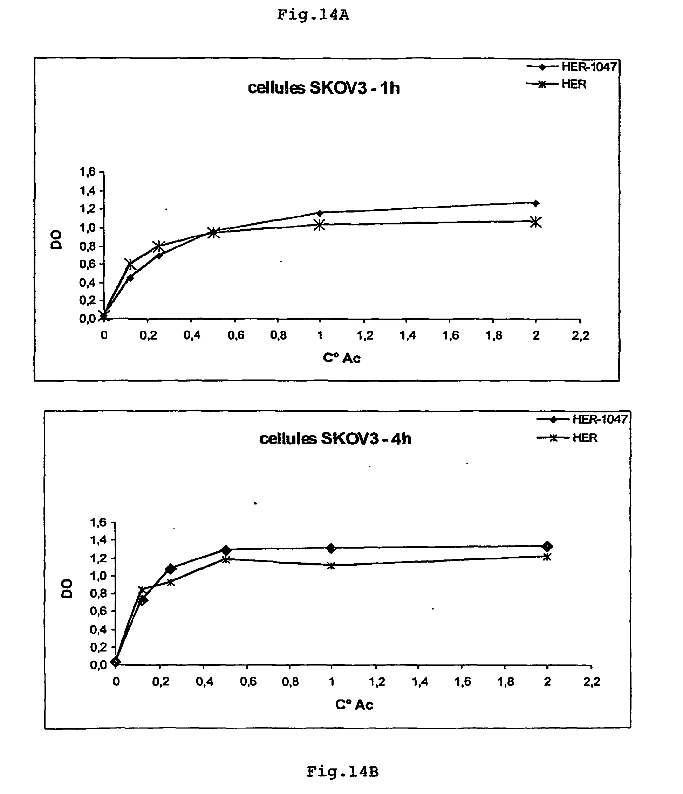

      - Les figures 14 A et B représentent le test 3 : en diminuant la concentration des Anticorps et faisant varier la durée d'incubation 1 h ou 4h.

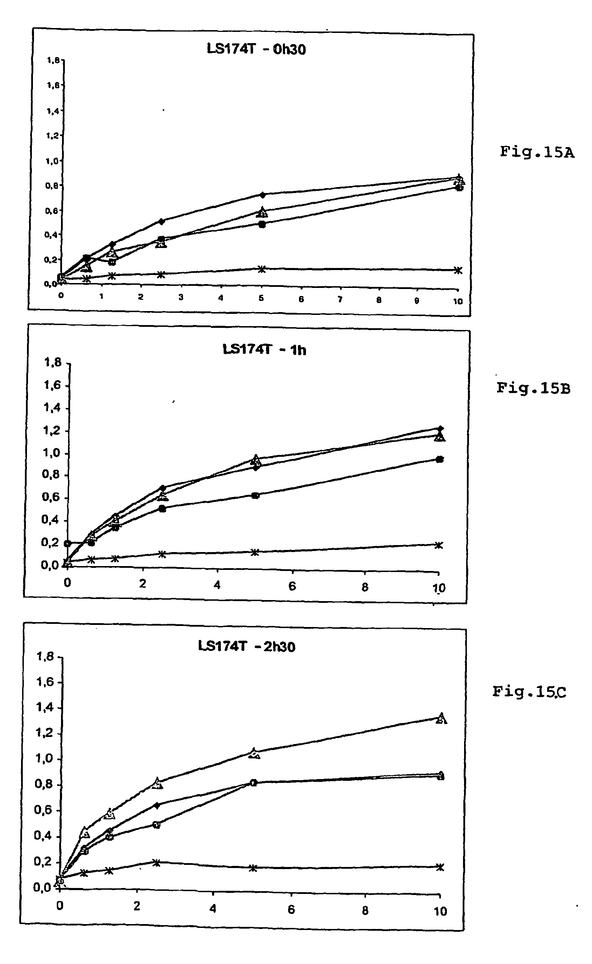

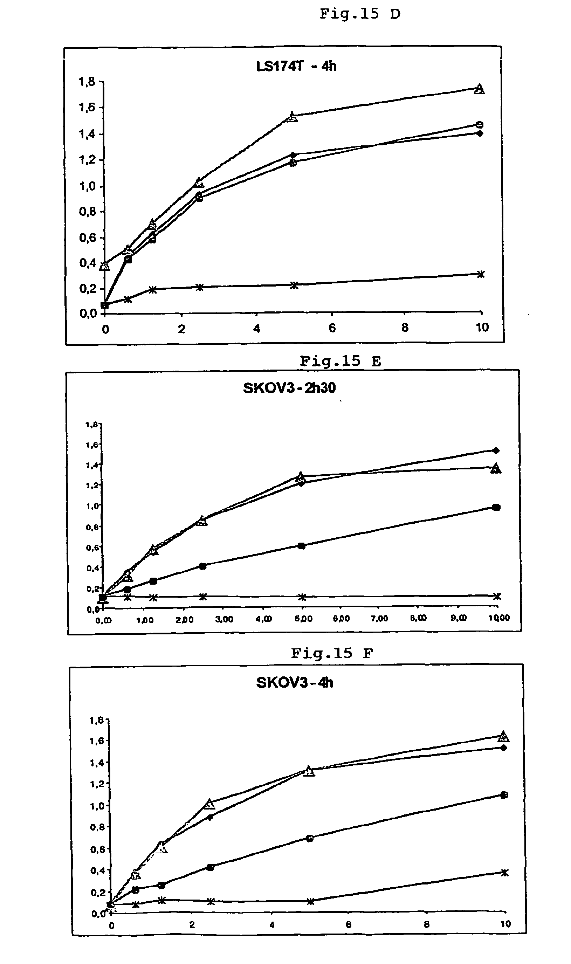

      - Les figures 15 A à F représente le test 4 : protocole identique mais en comparant les trois peptides (1047-HBP7-HBP10) sur cellules SKOV3 (ACE -), et LS174T (ACE+).

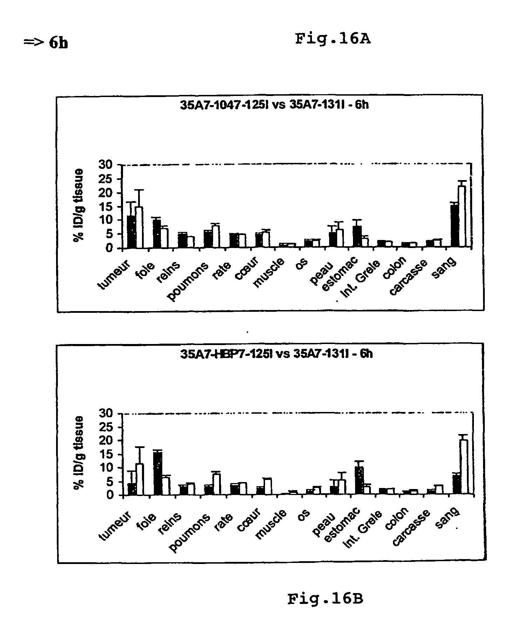

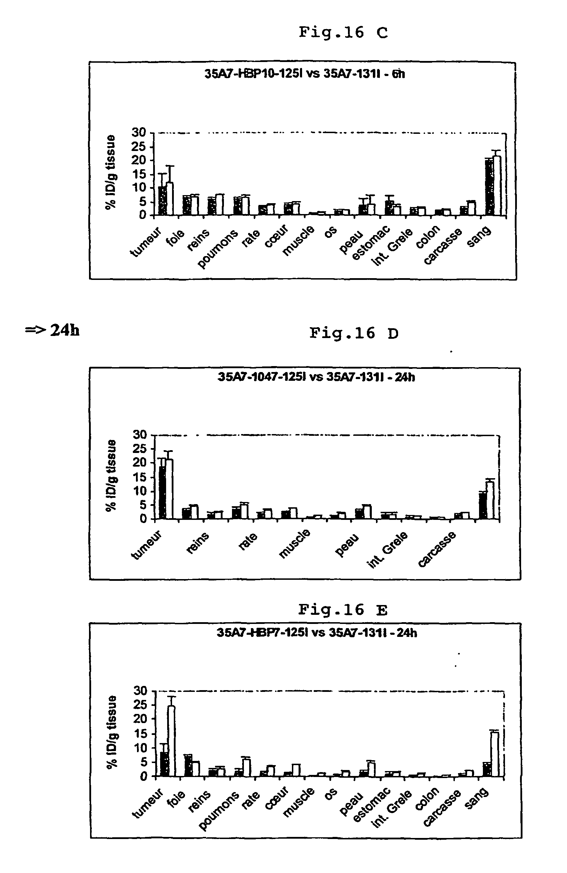

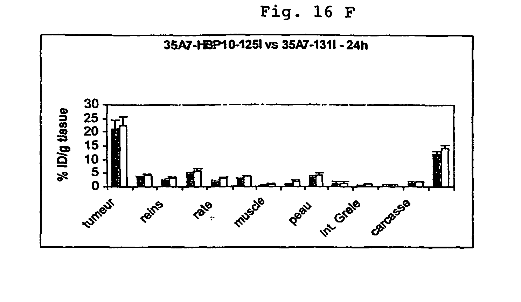

   Les figures 16 A à F illustrent l'expérimentation in vivo : distribution des Anticorps 35A7-1047, 35A7-HBP7, 35A7-HBP10 radiomarqués à l'iode 125, versus 35A7-iode 131 ; Souris Swiss nu/nu greffées avec des LS174T (carcinome colique humain, CEA+) ; co-injection de 5µg Ac-pept* et de 5ug d'Ac* natif par souris, en i.v. ; dissection 6h / 24h post-injection, expression des résultats en pourcentage de dose injectée par gramme de tissu. Barre noire : Ac conjugué - 125I Barre blanche : Ac natif - 131I.

### 11) Transport de particules à l'aide d'un dérivé peptide-IgG.

Le vecteur utilisé dans les exemples suivants est un anticorps monoclonal de souris (IgG1) couplé au peptide de séquence ID NO : 10 (1047). Le couplage du peptide à l'anticorps monoclonal a été réalisée comme décrit précédemment.

### a) Billes fluorescentes : Préparation du complexe peptide IgG-microsphère :

Des microsphères de polystyrene fluorescentes de 70 à 900nm portant des anticorps anti-IgG de souris à leur surface (Estapor, Merck eurolab) sont diluées au 1/100 dans NaCl 0,15M. 4µl de cette préparation sont ajoutés à un volume de 50 µl contenant 10 µg d'IgG monoclonales de souris conjuguées au peptide SEQ ID NO : 2 et laissé en incubation pendant 30 minutes à la température du laboratoire. Ce milieu réactionnel est déposé sur les cellules H1299 ensemencées (5x10⁴ cellules/puits) la veille pendant 18 heures.

Les cellules sont lavées et observées au microscope à fluorescence : les cellules incubées avec les complexes peptide-IgG-billes fluorescentes sont fortement marquées, quelle que soit la taille des billes alors que les contrôles (billes fluorescentes sans peptide-IgG, ou avec des IgG normales) sont négatives.

### - Or colloïdal : préparation du complexe peptide-IgG-or colloïdal :

Des billes d'or colloïdal (British Biocell International) de 10nm sont complexées à des IgG monoclonales de souris, préalablement conjuguées au peptide 1047, selon les instructions du fabricant dans un rapport de 750µg d'IgG pour 1ml de la solution d'or colloïdal. Pour la pénétration du complexe dans les cellules, le culot de la suspension est diluée au demi dans le milieu de culture.

### c) Evaluation de l'internalisation des complexes peptide-IgG-billes.

### - fluorescentes et -or colloïdal au microscope électronique.

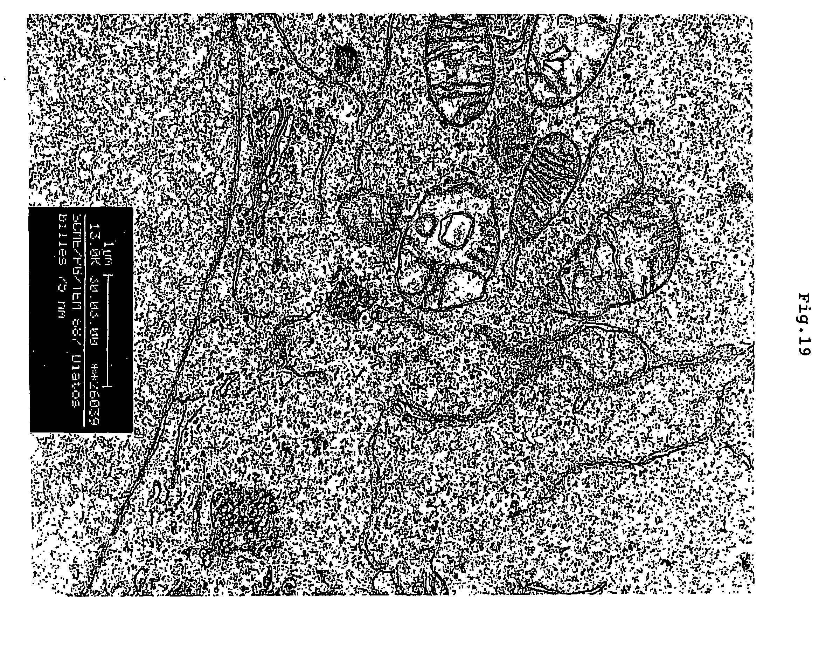

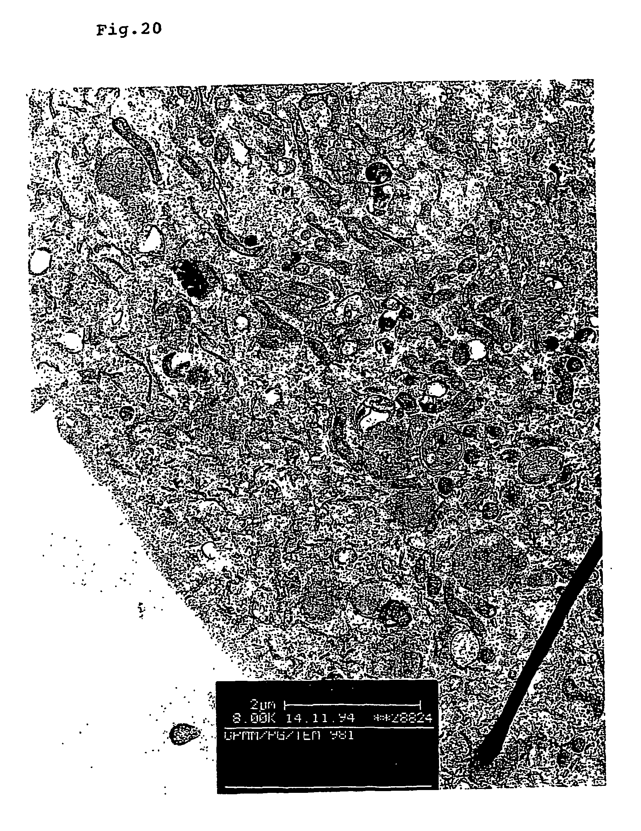

Après les étapes de conjugaison, les préparations sont déposées sur les cellules H1299 cultivées depuis la veille comme décrit précédemment. Après 18 heures de culture pour le complexe IgG-billes fluorescentes ou 4 heures pour le complexe IgG-or colloïdal, les cellules sont lavées au PBS trois fois puis fixées dans une solution de glutaraldehyde à 1,6% dans le tampon phosphate de sodium, 0,1M pendant une heure puis traitées comme habituellement pour la microscopie électronique.
d) Résultats : Les complexes peptides IgG-microsphères de 70nm (figure 19) ainsi que les comlexes peptide-IgG-or colloïdal (figure 20) sont visibles dans des vésicules et dans le réticulum endoplasmique du cytoplasme et autour de la région golgienne

### 12) Transport de la doxorubicine.

Les anthracyclines comme la doxorubicine sont parmi les agents les plus actifs dans le traitement des cancers humains. Leur mode d'entrée dans les cellules n'est pas encore résolu. Ce que l'on sait est qu'elles sont rapidement transportées dans le noyau des cellules. Leur toxicité, probablement due à leur large diffusion dans tout l'organisme, est une limitation dans le traitement, empêchant l'utilisation d'une grande quantité.

Nous avons voulu définir si le couplage de la doxorubicine aux peptides HBP améliorerait son pouvoir d'inhibition de croissance des tumeurs humaines tout en réduisant la toxicité de la drogue.
a) Couplage des peptides à la doxorubicine
La doxorubicine dont le groupement aminé est protégé est traitée par de l'acide 4-maleimidobutyrique en présence de carbodiimide, ce qui aboutit à la formation d'une liaison ester entre l'hydroxyle de la doxorubicine et le carboxyle de l'acide maleimidobutirique. Le groupement maleimide ainsi introduit dans la doxorubicine réagit avec la cystéine présente en -C ou -N terminal du peptide.
En suivant le schéma général, les conjugués suivants ont été préparés :
1047-doxorubicine ; HBP1-doxorubicine ; HBP3-doxorubicine ; HBP6-doxorubicine ; HBP7-doxorubicine ; HBP10-doxorubicine ; HBP13-doxorubicine.

b) Evaluation *in vitro* de l'activité biologique de la doxorubicine conjuguée aux peptides.
Pour évaluer l'activité biologique des conjugués peptide- doxorubine, l'inhibition de croissance des cellules tumorales en culture est mesurée.
Les cellules sont ensemencées la veille dans des plaques de 96 puits à raison de 10³ cellules par puits. Le lendemain, le surnageant est aspiré et remplacé par 100 µl de milieu contenant des dilutions successives de peptide-doxorubicine ou de doxorubicine native (10⁻⁶ M-10⁻⁸ M) et la culture poursuivie pendant 48 heures.
Les puits sont ensuite additionnés de 50 µl de MTT (bromure de 3-(4,5-diméthylthiazol-2yl)-2,5-diphényl tétrazolium) à 1 mg/ml dans le milieu de culture et cultivés pendant 4 heures. Le surnageant est enlevé et les puits sont additionnés de 100 µl de diméthylsulfoxide. Après dissolution des cristaux, la coloration est lue à 550 nm.
Les résultats sont calculés en pourcentage de la densité optique moyenne obtenue dans les puits de cellules contenant les dilutions des échantillons à tester et de la densité optique des puits n'ayant reçu que du milieu. Ils sont exprimés en concentration molaire donnant 50% d'inhibition de croissance des cellules et sont rapportés dans le tableau 20 ci-dessous.

**Tableau 20**

| | Lignées Cellulaires | | | | | | | |
|---|---|---|---|---|---|---|---|---|
| | H1299 | HH9 | HeLa | B16. F10 | K562 | K562R * | MCF7 | MCF7R * |
| Doxoru bicine native | 3x10⁻⁷ | 3x10⁻⁷ | 2x10⁻⁷ | 1x10⁻⁷ | 4x10⁻⁸ | 3x10⁻⁶ | 2x10⁻⁷ | 2x10⁻⁴ |
| (HBP1)₃ -Doxo | 7x10⁻⁷ | nt* | nt* | nt* | 2x10⁻⁷ | 5x10⁻⁶ | 6x10⁻⁷ | 1x10⁻⁴ |
| (HBP3)₂ -Doxo | nt* | nt* | nt* | nt* | 1x10⁻⁷ | 3x10⁻⁶ | 5x10⁻⁷ | 2,5x10⁻⁴ |
| HBP6-Doxo | nt* | nt* | nt* | nt* | 3x10⁻⁷ | 5x10⁻⁶ | 6x10⁻⁷ | 6,5x10⁻⁴ |
| HBP7-Doxo | nt* | nt* | nt* | nt* | 1x10⁻⁶ | 1x10⁻⁵ | 7x10⁻⁷ | 4x10⁻⁴ |
| HBP10-Doxo | nt* | nt* | nt* | nt* | 8x10⁻⁷ | 1x10⁻⁵ | 5x10⁻⁷ | 3,5x10⁻⁴ |
| HBP13-Doxo | nt* | nt* | nt* | nt* | 4x10⁻⁷ | 5x10⁻⁵ | 6x10⁻⁷ | 4,5x10⁻⁴ |
| HBP-1047-Doxo | 7x10⁻⁷ | nt* | 2,5x10⁻⁷ | 5x10⁻⁷ | 2x10⁻⁷ | 2x10⁻⁷ | 2x10⁻⁷ 8×10⁻⁷ | 1x10⁻³ |

Il apparaît que, *in vitro*, la sensibilité des cellules aux conjugués peptide-doxorubicine est du même ordre de grandeur que celui de la doxorubicine native. Les lignées résistantes à la doxorubicine (K562R et MCF7R) nécessitent une plus grande concentration de doxorubicine.
c) Evaluation *in vivo* de l'activité antitumorale des conjugués peptide-doxorubicine.
Les souris nues sont injectées avec 3x10⁶ cellules HH9 en sous-cutanée dans le flanc. Le jour 19 après la greffe des cellules, les tumeurs sont mesurées et 4 groupes de 6 souris sont formés qui reçoivent en injection péritumorale soit du NaCl, soit de la doxorubicine native ou les conjugués 1047-doxorubicine ou HBP1-doxorubine à raison de 30 µg de doxorubicine ou équivalent de conjugués toutes les deux semaines. Les tumeurs sont mesurées et leur volume calculé. Au jour 60, après l'injection d'un total de 120 µg de doxorubicine ou équivalent, la croissance des tumeurs est respectivement inhibée de 77% avec le conjugué 1047-doxorubicine, de 84% avec le conjugué HBP1-doxorubicine et de 79% avec la doxorubicine seule, ce qui démontre que *in vivo* ces conjugués sont au moins aussi efficaces que la doxorubicine native.
d) Evaluation *in vivo* de la toxicité des conjugués peptide-doxorubicine.
L'injection par voie intraveineuse de doxorubicine ou de doxorubicine-peptide permet de comparer la toxicité des différentes préparations. L'estimation se fait par la perte de poids des souris (5 souris par groupe). Deux expériences ont été faites :
Expérience 1 : les souris ont reçu 200µg par injection à 3 jours d'intervalle, soit 600µg de doxorubicine ou équivalent peptide-doxorubicine au total. Les souris sont pesées le lendemain de la dernière injection.
Expérience 2 : Les souris ont reçu 2 injections de 200µg de doxorubicine ou équivalent peptide-doxorubicine à une semaine d'intervalle soit 400µg au total et sont pesées le lendemain de la dernière injection.

Le tableau 21 ci-dessous rapporte la perte de poids des souris traitées avec de la doxorubicine ou des conjugués peptide-doxorubicine. La perte de poids moyenne est calculée par rapport au poids moyen des souris ayant reçu du NaCl.

**Tableau 21**

| Groupes | NaCl | Doxo. | HBP3 | HBP6 | HBP7 | HBP10 | HBP13 |
|---|---|---|---|---|---|---|---|
| Exp. 1 | | | | | | | |
| Poids | 23, 11 | 16, 06 | 21,45 | 20,3 | 19,11 | 21,15 | **nt |
| Perte* | - | 31* | 8 | 12 | 18 | 9 | - |

| Exp.2 | | | | | | | |
|---|---|---|---|---|---|---|---|
| Poids | 31,4 | 26,7 | 29,5 | 29,5 | 31,4 | 32,5 | 30 |
| Perte* | - | 15 | 6 | 6 | 0 | 0 | 4 |

| | | | | | | | |
|---|---|---|---|---|---|---|---|
| * : % ** : non testé | | | | | | | |

A la dose de 600 µg, les souris ayant reçu la doxorubicine native perdent 31% de leur poids alors que celles ayant reçu le peptide-doxorubicine ne perdent que 18 à 8% de leur poids. La dose de 400 µg entraine une perte de poids de 15 % chez les souris recevant la doxorubicine native, alors qu'avec les conjugués peptide-doxorubicine, la perte n'est pas significative.

### 13) Transport de l'ubiquitine.

L'ubiquitine est un polypeptide de' 76 acides aminés, hautement conservé durant l'évolution, présente dans l'organisme chez tous les eucaryotes, de poids moléculaire 8500 daltons L'ubiquitine intracellulaire est impliquée dans diverses fonctions cellulaires telles que la dégradation des protéines après leur ligation avec l'ubiquitine, la progression du cycle cellulaire, la régulation de l'activation du facteur de transcription NF-κB. L'ubiquitine extracellulaire a la propriété d'inhiber la prolifération des cellules souches hématopoïétiques () par induction d'apoptose.
a) Couplage de l'ubiquitine au peptide.
1 mg d'ubiquitine dans 0.3 ml de tampon phosphate de sodium 0,1M, pH7 contenant 0,15M NaCl sont additionnés de 390 µg de SMCC (4-(N-maléimidométhyl) cyclohexane-1-carboxylate de succinimidyle) dans 39 µl de diméthylsulfoxyde et la solution est incubée 30 minutes à la température du laboratoire. L'élimination d'excès de réactif se fait par centrifugation sur des membranes d'ultrafiltration (seuil de coupure = 5000 daltons, Sartorius), suivie de trois lavages en tampon phosphate de sodium 0,1M, pH7 contenant 0,15M NaCl. Le couplage avec le peptide se fait ensuite dans un rapport molaire de 5 peptides pour 1 ubiquitine dans 1 ml de tampon phosphate de sodium 0,1M, pH7, à la température du laboratoire. Ensuite l'excès de peptide non-couplé est éliminé par centrifugation sur des membranes, comme à l'étape précédente.
b) Evaluation de la pénétration des conjugués peptide-ubiquitine.
L'évaluation de la pénétration des conjugués peptide-ubiquitine se fait avec des conjugués préparés avec des peptides portant une biotine du côté N-terminal.
Des cellules HT29 sont ensemencées la veille dans des plaques de 24 puits (5x10⁴/puits).
Les différents conjugués peptides (biotinylé)-ubiquitine sont additionnés à des concentrations décroissantes (100 à 25 µg/ml) dans le milieu de culture pendant 4 heures à 37°C. A la fin de la culture, les cellules sont lavées trois fois avec du PBS, puis fixées 15 minutes dans l'éthanol à -20°C. La pénétration du conjugué peptide-biotine-ubiquitine est évaluée après incubation pendant 60 minutes avec de l'avidine couplée à la peroxydase. L'activité de la peroxydase est révélée par la diaminobenzidine en présence d*'*H₂O₂.
La pénétration, dépendante de la concentration des conjugués dans le milieu de culture, est évaluée au microscope par l'intensité de la coloration exprimée par des croix. Le tableau 22 ci-dessus indique la pénétration des conjugués peptide-ubiquitine dans les cellules.

**Tableau 22**

| Concentration (µg/ml) | 100 | 50 | 25 |
|---|---|---|---|
| Ubiquitine native | +/-* | +/- | +/- |
| 1047-ubiquitine | +++ | ++ | + |
| HBP7-ubiquitine | ++++ | +++ | ++ |
| HBP10-ubiquitine | +++ | ++ | ++ |

| | | | |
|---|---|---|---|
| ++++ : très intense ; +++ intense ; ++ : positive ; + faiblement positive ; +/- à la limite de la lecture. | | | |

c) Evaluation de l'activité biologique des conjugués peptide-ubiquitine.
La prolifération des cellules est mesurée par le test au MTT. Des cellules Daudi (3x10⁴ cellules/puits) sont cultivées dans des plaques de 96 puits pendant 48 heures à 37°C dans le milieu de culture RPMI complet additionné de concentrations décroissantes (200-12,5 µg/ml) d'ubiquitine native ou conjuguée aux peptides et la culture poursuivie pendant 48 heures.
Les puits sont ensuite additionnés de 50 µl de MTT (bromure de 3-,(4,5-diméthylthiazol-2y1)-2,5-diphényl tétrazolium) à 1 mg/ml dans le milieu de culture et cultivés pendant 4 heures.
Le surnageant est enlevé et les puits sont additionnés de 100 µl de diméthylsulfoxyde. Après dissolution des cristaux, la coloration est lue à 550 nm.
Le tableau 23 indique l'inhibition de la croissance des cellules Daudi par les conjugués peptide-ubiquitine. Les résultats, montrés dans le tableau 23, sont calculés en pourcentage de la densité optique moyenne obtenue dans les puits de cellules contenant les dilutions des échantillons à tester et de la densité optique des puits n'ayant reçu que du milieu. Ils sont exprimés en concentration donnant 50% d'inhibition de croissance des cellules.

**Tableau 23**

| Concentration (µg/ml | 200 | 100 | 50 |
|---|---|---|---|
| Ubiquitine native | 0* | 0 | 0 |
| 1047-ubiquitine | 60 | 21 | 0 |
| HBP7-ubiquitine | 96 | 68 | 35 |
| HBP10-ubiquitine | 16 | 11 | 0 |

| | | | |
|---|---|---|---|
| *% d'inhibition calculée par rapport à la croissance des cellules n'ayant reçu que du milieu de culture | | | |

L'ubiquitine native n'inhibe pas la croissance des cellules Daudi. Le conjugué préparé avec le peptide HBP10 inhibe peu, alors que le conjugué préparé avec le peptide HBP7 est efficace et donne encore 35% d'inhibition à 50µg/ml. Le conjugué préparé avec le peptide 1047 donne des valeurs d'inhibition intermédiaire.

### 14) Transport du cytochrome C.

Le cytochrome C est une hémoprotéine qui constitue le complexe lipide-protéines mitochondriales. Il joue un rôle vital dans les oxydations cellulaires des plantes et des mammifères. Il est formé d'une seule chaîne polypeptidique de 104 acides aminés (masse moléculaire 13000 daltons) avec le groupe hème attaché aux résidus cystéines. Le relargage du cytochrome C des mitochondries dans le cytosol entraine une apoptose des cellules par activation des caspases.
a) Couplage de cytochrome C aux peptides.
4 mg de cytochrome C dans 930 µl de tampon phosphate de sodium 0,1M, pH7 contenant 0,15M NaCl sont additionnés de 540 µg de SMCC (4-(N-maléimidométhyl) cyclohexane-1-carboxylate de succinimidyle) dans 54 µl de diméthylsulfoxyde et la solution est incubée 30 minutes à la température du laboratoire. L'élimination d'excès de réactif se fait par centrifugation sur des membranes d'ultrafiltration (seuil de coupure = 10000 daltons, Sartorius), suivie de trois lavages en tampon phosphate de sodium 0,1M, pH7 contenant 0,15M NaCl.
Le couplage avec le peptide se fait ensuite dans un rapport molaire de 10 peptides pour 1 cytochrome C dans 1 ml de tampon phosphate de sodium 0,1M, pH 7 contenant 0,15 M NaCl, pendant 3 heures à la température du laboratoire. Ensuite, l'excès de peptide non-coupléé est éliminé par centrifugation/filtration comme à l'étape précédente.
b) Evaluation de la pénétration des conjugués peptide-cytochrome C dans les cellules.
L'évaluation de la pénétration des conjugués peptide cytochrome C se fait avec des conjugués préparés avec des peptides portant une biotine du côté N-terminal.
Des cellules H1299 sont cultivées en présence des différents conjugués peptides (biotinylés)-cytochrome C à des concentrations décroissantes (100 à 25 µg/ml) dans le milieu de culture pendant 4 heures à 37°C. A la fin de la culture, les cellules sont lavées trois fois avec du PBS, puis fixées 15 minutes dans l'éthanol à -20°C. La pénétration du conjugué peptide-biotine-cytochrome C est évaluée après incubation pendant 60 minutes avec de l'avidine couplée à la peroxydase. Les cellules sont ensuite lavées avec le PBS et l'activité de la peroxydase est révélée par la diaminobenzidine en présence d'H₂O₂.
La pénétration dépendante de la concentration des conjugués dans le milieu de culture est évaluée au microscope par l'intensité de la coloration exprimée par des croix. Le tableau 24 indique la pénétration des conjugués peptide-cytochrome C dans les cellules.

**Tableau 24**

| Concentration (µg/ml) | 100 | 50 | 25 |
|---|---|---|---|
| Cytochrome C natif | -* | - | - |
| 1047-cytochrome C | +++ | ++ | + |

| | | | |
|---|---|---|---|
| +++ intense ; ++ : positive; + faiblement positive; - négatif. | | | |

Le tableau ci-dessus montre que le cytochrome C natif ne pénètre pas dans les cellules et que le conjugué 1047-cytochrome C pénètre d'une façon efficace jusqu'à 25µg/ml.
c) Evaluation de l'activité biologique *in vitro* des conjugués peptide-cytochrome C.
Des cellules H1299 ou HT29 (3 x 10⁴ cellules/puits) sont cultivées dans des plaques de 96 puits pendant 48 heures à 37°C dans le milieu de culture RPMI complet additionné de concentrations décroissantes (200-3 µg/ml) de cytochrome C natif ou conjugué aux peptides et la culture poursuivie pendant 48 heures.
Les puits sont ensuite additionnés de 50 µl de MTT (bromure de 3-,(4,5-diméthylthiazol-2yl)-2,5-diphényl tétrazolium) à 1 mg/ml dans le milieu de culture et cultivés pendant 4 heures.
Le surnageant est enlevé et les puits sont additionnés de 100 µl de diméthylsulfoxide. Après dissolution des cristaux, la coloration est lue à 550 nm.
Les résultats sont calculés en pourcentage de la densité optique moyenne obtenue dans les puits de cellules contenant les dilutions des échantillons à tester et de la densité optique des puits n'ayant reçu que du milieu. Le tableau 25 indique l'inhibition de la croissance des cellules par les conjugués peptide-ubiquitine.

**Tableau 25**

| Sur le cellules H1299 | | | | | | |
|---|---|---|---|---|---|---|
| Concentration (µg/ml) | 100 | 50 | 25 | 12,5 | 6 | 3 |
| Cytochrome C natif | 0* | 0 | 0 | 0 | 0 | 0 |
| 1047-cytochrome C | 46 | 40 | 29 | 15 | 14 | 0 |
| Sur des cellules HT29 | | | | | | |
| Concentration (µg/ml) | 200 | 100 | 50 | 25 | 12,5 | |
| Cytochrome C natif | 12* | 19 | 10 | 0 | 0 | |
| 1047-cytochrome C | 28 | 23 | 23 | 0 | 0 | |
| HBP3-cytochrome C | 42 | 22 | 15 | 0 | 0 | |
| HBP6-cytochrome C | 41 | 18 | 12 | 2 | 0 | |
| HBP7-cytochrome C | 35 | 14 | 5 | 0 | 0 | |

| | | | | | | |
|---|---|---|---|---|---|---|
| *% d'inhibition calculée par rapport à la croissance des cellules n'ayant reçu que du milieu de culture | | | | | | |

Le tableau ci-dessus montre l'efficacité des conjugués peptide-cytochrome C sur deux type de cellules. Le cytochrome C natif n'inhibe pas la croissance. A 50 µg/ml le peptide le plus efficace sur les cellules HT29 est le peptide 1047, les peptides HBP3 et HBP6 donnent des valeurs intermédiaires, tandis que le peptide HBP) est peu actif à cette concentration.

### 15) Transport de substances anti-tumorales et anti-inflammatoires à l'aide des peptides de l'invention.

a) Transport de dérivé de l'aspirine.
Les analgésiques comme l'aspirine utilisés depuis longtemps en thérapeutique se sont révélés posséder d'autres effets comme par exemple, la prévention des tumeurs du colon chez les hommes et les femmes prenant de l'aspirine régulièrement depuis longtemps. Chez l'animal, l'effet anti-cancéreux a été montré sur des tumeurs variées. Ces effets ont été attribués en partie à leur capacité d'inhiber la production des prostaglandines, par inhibition de l'enzyme prostaglandine H synthétase et des cyclooxygénases.
Deux dérivés de l'aspirine conjuguée au peptide de séquence SEQ ID NO : 30 : un dérivé sacilylyl-HBP6 en -N terminal (peptide SEQ ID NO : 47) et un dérivé en HBP6-salicylyl en -C terminal (SEQ ID NO : 48) ont été testés pour leur capacité à inhiber la croissance des cellules tumorales HT29 par comparaison avec le peptide natif.
Des cellules HT29 (3x10⁴ cellules/puits) sont cultivées dans des plaques de 96 puits pendant 48 heures à 37°C dans le milieu de culture DMEM complet additionné de concentrations décroissantes (200-3µg/ml) d'aspirine ou de conjugué peptide salicylyl et la culture poursuivie pendant 48 heures.
Les puits sont ensuite additionnés de 50 µl de MTT (bromure de 3-,(4,5-diméthylthiazol-2y1)-2,5-diphényl tétrazolium) à 1 mg/ml dans le milieu de culture et cultivés pendant 4 heures.
Le surnageant est enlevé et les puits sont additionnés de 100 µl de diméthylsulfoxide. Après dissolution des cristaux, la coloration est lue à 550 nm.
Les résultats d'inhibition de la croissance des cellules HT29 sont montrés dans le tableau 26 et sont calculés en pourcentage de la densité optique moyenne obtenue dans les puits de cellules contenant les dilutions des échantillons à tester et de la densité optique des puits n'ayant reçu que du milieu.

**Tableau 26**

| Concentration (µg/ml) | 1000 | 500 | 200 | 100 |
|---|---|---|---|---|
| Aspirine native | 48* | 6 | 3 | 0 |
| Salicylyl-HBP6 | 51 | 35 | 30 | 30 |
| HBP6-salicylyl | 50 | 45 | 20 | 20 |

| | | | | |
|---|---|---|---|---|
| *% d'inhibition | | | | |

L'adjonction du peptide à l'acide salicylique augmente le pouvoir de l'aspirine d'inhiber la croissance des cellules.
b) Transport de dérivé de l'acide phtalique.
Le N-phtalimidoglutarimide (thalidomide) est une molécule qui possède une grande variété de propriété comme l'effet tératogène, la réduction de la production de TNF-α par les monocytes, la suppression de l*'*angiogenèse. Il a été montré que l'activité tératogène dépend du résidu glutarimide tandis que l'effet antiangiogénique dépend du groupement phtaloyl. Nous avons donc préparé le peptide SEQ ID NO : 46 dérivé du peptide SEQ ID NO : 26 (HBP3)₂ auquel a été ajouté du glycyl phtaloyl en N-terminal. puits n'ayant reçu que du milieu. La technique employée pour tester l'activité du phtaloyl-HBP3 est la même que celle décrite précédemment pour l'aspirine.
Les résultats de l'inhibition de la croissance (%) des cellules HT29 montrés dans le tableau 27 sont calculés en pourcentage de la densité optique moyenne obtenue dans les puits de cellules contenant les dilutions des échantillons à tester et de la densité optique moyenne des puits de cellules n'ayant reçu que du milieu.

**Tableau 27**

| Concentration (µg/ml) | 1000 | 500 | 200 | 100 |
|---|---|---|---|---|
| Phtaloyl-HBP3 | 66 | 28 | 29 | 20 |
| HBP3 | 2 | 0 | 0 | |

| | | | | |
|---|---|---|---|---|
| *% | | | | |

Il apparaît que le dérivé phtaloyl inhibe la croissance des cellules HT29.

### 16) Augmentation de l'activité de substances Anti-microbiennes à l'aide de peptides de l'invention.

a) Le lysozyme.
Le lysozyme est un des constituants majeurs de granules des lymphocytes polynucléaires humains. On le trouve aussi dans les secrétions. C'est une muraminidase. Le lysozyme lyse et tue les microorganismes Gram-positif en modifiant les peptidoglycans de leur surface. Comme le lysozyme ne peut facilement pénétrer la membrane extérieure de la bactérie, le couplage du lysozyme aux peptides peut aider à la pénétration
- Couplage des peptides au lysozyme.
8 mg de lysozyme dans 2,7 ml de tampon phosphate de sodium 0,1M, pH7 contenant 0,15M NaCl sont additionnés de 1,8 mg de SMCC (4-(N-maléimidométhyl) cyclohexane-1-carboxylate de succinimidyle) dans 186 ml µl de diméthylsulfoxyde et la solution est incubée 30 minutes à la température du laboratoire. L'élimination d'excès de réactif se fait par centrifugation sur des membranes d'ultrafiltration (seuil de coupure = 5000 daltons, Sartorius), suivie de trois lavages en tampon phosphate de sodium O,1M, pH7 contenant 0,15M NaCl.
Le couplage avec le peptide se fait ensuite dans un rapport molaire de 5 peptides pour 1 lysozyme dans 1 ml de tampon phosphate de sodium 0,1M, pH7 contenant 0,15M NaCl, pendant 3 heures à la température du laboratoire. Ensuite l'excès de peptide non couplé est éliminé par centrifugation sur des membranes, comme à l'étape précédente.
- Evaluation de l'activité du lysozyme couplé aux peptides.
La souche bactérienne gram-positive : Escherischia coli (ATCC25922) est cultivée jusqu'à la phase semi-logarithmique dans le milieu Luria-Bertani (LB). Les bactéries sont collectées par centrifugation et resuspendues dans le milieu 1% bacto-peptone à une concentration de 5x10⁵ cfu/ml (colony forming unit/ml) Le lysozyme ou les conjugués peptide-lysozyme sont dilués de demi en demi (256 à 0,5µg/ml dans le milieu bacto-peptone et 50µl sont distribuées dans des plaques de 96 puits. 50 µl de la dilution de bactéries sont alors ajoutés dans chaque puits Après 16 heures d'incubation à 37°C, 10µl de chaque puits est étalé sur des boites de gélose en milieu LB. Après 18 heures d'incubation à 37°C, la concentration bactéricide minimale (MBC) est déterminée comme la concentration de lysozyme ou lysozyme-peptide qui inhibe 75% de la croissance.
- Résultats.
Le tableau 28 rapporte l'inhibition de croissance de *E. coli* (MBC µg/ml).

**Tableau 28**

| | |
|---|---|
| Lysozyme natif | > 256 |
| 1047-Lysozyme | 32 |
| (HBP1)₃-Lysozyme | 4 |
| (HBP3)₂-Lysozyme | 16 |
| HBP6-Lysozyme | 16 |

Les conjugués peptide-lysozyme ont une activité antibactérienne plus efficace que celle du lysozyme natif.
b) Peptides antimicrobiens.
Depuis quelques années, un intérêt considérable s'est développé pour l'étude structure-fonction de peptides courts possédant des activités antibactériennes et antifongiques. Beaucoup de peptides naturels, de séquences variées, trouvés dans le monde animal et végétal, possèdent des modes d'action similaires contre une large variété de microbes.
Les peptides antimicrobiens possèdent en général un nombre équivalent de résidus polaires et non-polaires à l'intérieur de domaines amphipatiques et suffisamment de résidus basiques pour donner un peptide d'une charge globale positive à pH neutre.
Les peptides objet de la présente invention sont aussi des peptides principalement basiques, de haute affinité pour l'héparine, et dans les exemples donnés plus bas, il est montré que plusieurs d'entre eux possèdent une activité antibactérienne *in vitro* à des concentrations auxquelles ils ne sont pas toxiques pour les cellules eucaryotes.
- Evaluation de l'activité antimicrobienne des peptides cationiques.
Les souches bactériennes gram-positives : *Enterococcus faecalis* (ATCC 29212), et *Staphylococcus* aureus (ATCC 25923) et les souches gram-négatives : *Escherichia.coli* (ATCC 25922), *Pseudomonas aeruginosa* (ATCC 27853) et *Salmonella typhimurium* (isolat clinique), *Salmonella typhiTy2* (WHO). Les bactéries sont cultivées avec agitation toute la nuit à 37°C, puis recultivées à la dilution de 1:50 dans du milieu frais de Luria-Bertani (LB)pendant 2 heures. La concentration bactérienne est ajustée à 106 cfu/ml et 50µl sont distribuées dans des plaques de 96 puits avec un égal volume des peptides dilués de demi en demi (256-0,5 µg/ml) dans le milieu LB. Après 16 heures à 37°C, la concentration minimale inhibitrice (MIC) est déterminée comme la concentration la plus faible qui inhibe totalement la croissance des bactéries (absence totale de turbidité). Pour déterminer la concentration minimale bactéricide, 10µl de chaque puits est étalé sur des boites de gélose en milieu LB Après 18 heures d'incubation à 37°C, la concentration bactéricide minimale (CMB) est définie comme la concentration de peptide qui ne laisse subsister que 0,01% de bactéries vivantes.
- Résultats.
Les résultats sont exprimés en concentration de peptide en MBC (µM).

**Tableau 29**

| Souches Peptide | E. coli | S. typhimu rium | S. typhi Ty2 | P. aeruginosa | S. aureus | E. fascalis |
|---|---|---|---|---|---|---|
| SEQ ID NO : 2 | >160 * | >160 | nt | >160 | >160 | >160 |
| SEQ ID NO : 3 | 13,2 5 | 13,25 | 1,65 | 26,5 | >106 | >106 |
| SEQ ID NO : 38 | >170 | >170 | >170 | >170 | >170 | >170 |
| SEQ ID NO : 24 | 55 | 110 | 27,5 | 27,5 | >110 | >110 |
| SEQ ID NO : 44 | 1,7 | 1,27 | 0,64 | >110 | >110 | >110 |
| SEQ ID NO : 45 | 1 | 1 | 0,66 | 0,5 | >680 | >680 |
| Ampiciline | 22 | 11 | 5,5 | >696 | <0,68 | 2,75 |

| | | | | | | |
|---|---|---|---|---|---|---|
| *µM | | | | | | |

Les résultats du tableau ci-dessus montre que le peptide (HBP1)3 possède une activité bactéricide, alors que les peptides (HBP1)2 et HBP13 ne sont pas actifs. Cependant, lorsque le peptides HPB13 (SEQ ID No : 38) est couplé au peptide SEQ ID NO : 24, il augmente son activité d'un facteur de 50 à 100 (peptide SEQ ID NO : 45). Il en est de même lorsque le peptide SEQ ID No : 24 est couplé au peptide SEQ ID No : 30 (HPB7) qui, lui, ne possède pas d'activité anti-microbienne.
La concentration minimale bactéricide (CMB) de tous les peptides du tableau ci-dessus est équivalente à la CMI ou diffère d'une dilution (CMB = 2 x CMI). Tous ces peptides ne sont pas actifs sur les bactéries Gram-positif.

### REFERENCES

1) Cardin A.D. & Weintraub H.J.R. Artheriosclerosis 9:21 (1989)
2) Merton B. et al. Annu. Rev. Cell Biol. 8:365 (1992)
3) David G. FASEB J. 7:1023 (1993)
4) Salmivira M & Jalkanen M Experentia 51:863 (1995)
5) Caldwell et al. Int. J. Biochem. Cell Biol. 28:203 (1996)
6) Fromm J.R. et al. Arch. Biochem. Bioph. 343:92 (1997)
7) Hirsch J. New Engl. J. Med. 324:1565 (1991)
8) Castellot J.J. et al. J. Cell Biol. 102:1979 (1986)
9) Ruoslahti E. & Yamazuchi Y. Cell 64:867 (1991)
10) Robinson D.S. Adv.Lip. Res. 1:1 (1963)
11) Kallunski P. & Tryggvason K. J. Cell Biol. 116:559 (1992)
12) Cardin A.D. et al. Biochem. Biophys. Res. Com. 154:741 (1988)
13) Avrameas A. et al. Proc. Natl. Acad. Sci. 95:5601 (1998)
14) Stevenson F.K. et al. J. Autoimmunity 6:809 (1993)
15) Hirabayashi Y. et al. Scand. J. Immunol. 37:533 (1993)
16) Kalsi J.K. et al. Lupus 4:375 (1995)
17) Campanelli JT, Gayer GG et Scheller RH Development (1996) 122: 1663-1672
18) Fowlkes JL, Thrailkill KM, George-Nascimento C.,Rosenberg CK & Serra DM. Endocrinol(1997) 138:2280-2285.
19) Maher DW, Lee BA & Donoghue DJ Mol Cell Biol(1989) 9:2251-2253.
20) Inoue M., Watanabe N., Morino Y., Tanaka Y., Amachi T & Sasaki J. FEBS(1990) 269:89-92.
21) Pasqualini R., Koivunen E. & Ruoslahti E. Nature Biotech. (1997) 15:542-546
22) Arkonac BM, Foster LC, Sibinga NES, Patterson C, Lai K, Tsai JC, Lee ME, Perrella MA & Haber E. J Biol Chem (1998) 273:4400-4405.
23) Yayon A, Klagsbrun M, Esko JD, Leder P. & Ornitz DM.. Cell (1991) 64:841-848.
24) Gongqiao XU, Fornster GG & Fornster JF Glyconjug J. (1996) 13:81-90.
25) Lortat-Jacob H & Grimaud JA. FEBS (1991) 280:152-154.
26) Hasan M., Najjam S., Gordon MY, Gibbs RV & Rider CC. J Immunol (1999)162:1064-1070.
27) Amara A, Lorthioir O, Valenzuela A, Magerus A, Thelen M, Montes M, Virelizier JL, Delepierre M, Baleux F, J. Biol. Chem. (1999) 272 :200-204.
28) Pohl J, Pereira HA, Martin NM & Spitznagel JK. Aminoacid sequence of CAP37 FEBS (1990) 272 :200-204.
29) Javadpour MM, Juban MM, Lo WCJ, Bishop SM, lberty JB. Cowell SSM, Becher CL & McLaughilin ML. J Med Chem (1996) 39: 3107-3113
30) Ternynck T. et Avrameas S. "Techniques immunoenzymatiques" Editions INSERM (1987)

## Revendications

1. Vecteur de transfert intracytoplasmique et/ou intranucléaire d'une substance d'intérêt, **caractérisé en ce qu'**il comprend au moins une séquence d'acides aminés réagissant *in vivo* avec les aminoglycanes et qui est issue de la lipoprotéine B humaine, couplée à au moins un fragment d'anticorps.

2. Vecteur selon la revendication 1, **caractérisé en ce que** la séquence d'acides aminés réagissant avec les aminoglycanes et qui est issue de la lipoprotéine B humaine est capable de réagir avec l'héparine, l'héparane sulfate, les chondroïtines sulfates, et leurs dérivés.

3. Vecteur selon l'une des revendications 1 ou 2, **caractérisé en ce que** la séquence d'acides aminés réagissant avec les aminoglycanes et qui est issue de la lipoprotéine B humaine est codée par la lignée germinale.

4. Vecteur selon l'une quelconque des revendications 1 à 3, **caractérisé en ce que** la séquence d'acides aminés réagissant avec les aminoglycanes et qui est issue de la lipoprotéine B humaine comprend un nombre d'acides aminés basiques au moins égal à 3.

5. Vecteur selon l'une quelconque des revendications précédentes, **caractérisé en ce que** la séquence d'acides aminés réagissant avec les aminoglycanes et qui est issue de la lipoprotéine B humaine est constituée par, ou comporte, au moins un groupe d'acides aminés répondant à l'une des formules suivantes : a) (XBBBXXBX)ₙ ; b) (XBBXBX)ₙ ; c) (BBXₘYBBXₒ)ₙ ; d) (XBBXXBX)ₙ ; ou e) (BXBB)ₙ ;
dans lesquelles : B est un acide aminé basique, X est un acide aminé non basique, Y représente soit une liaison directe soit 1 à 20 acides aminés, m est un nombre entier compris entre 0 et 5, n est un nombre entier compris entre 1 et 10, et o est un nombre entier compris entre 0 et 5.

6. Vecteur selon la revendication 5, **caractérisé en ce que** la séquence d'acides aminés réagissant avec les aminoglycanes et qui est issue de la lipoprotéine B humaine est constituée par, ou comporte, un groupe d'acides aminés répondant à la formule suivante : (XBBXXBX)ₙ.

7. Vecteur selon l'une des revendications 5 ou 6, **caractérisé en ce que** X est un acide aminé non-basique hydrophobe.

8. Vecteur selon l'une quelconque des revendications précédentes, **caractérisé en ce qu'**il comprend moins de 100 acides aminés.

9. Vecteur selon l'une quelconque des revendications précédentes, **caractérisé en ce qu'**il comprend moins de 50 acides aminés.

10. Vecteur selon l'une quelconque des revendications précédentes, **caractérisé en ce qu'**il comprend moins de 25 acides aminés.

11. Vecteur selon l'une quelconque des revendications précédentes, **caractérisé en ce que** la séquence d'acides aminés réagissant avec les aminoglycanes et qui est issue de la lipoprotéine B humaine est choisie dans le groupe comprenant SEQ ID NO.1 à l'état de monomère, dimère (SEQ ID.2), trimère (SEQ ID NO.3), polymère.

12. Vecteur selon l'une quelconque des revendications précédentes, **caractérisé en ce que** le fragment d'anticorps est un fragment d'anticorps polyréactif.

13. Vecteur selon l'une quelconque des revendications précédentes, **caractérisé en ce que** le fragment d'anticorps est une région hypervariable d'un anticorps.

14. Vecteur selon l'une quelconque des revendications précédentes, **caractérisé en ce que** le fragment d'anticorps est un fragment de la chaîne lourde d'un anticorps.

15. Vecteur selon l'une quelconque des revendications précédentes, **caractérisé en ce que** le fragment d'anticorps est un fragment d'anticorps anti-ADN humain.

16. Vecteur selon l'une quelconque des revendications 1 à 15, **caractérisé en ce que** le fragment d'anticorps est un fragment d'IgM.

17. Vecteur selon l'une quelconque des revendications 1 à 15, **caractérisé en ce que** le fragment d'anticorps est un fragment d'IgG.

18. Vecteur selon l'une quelconque des revendications précédentes, **caractérisé en ce que** le fragment d'anticorps est tout ou partie d'au moins une région CDR2 d'un anticorps.

19. Vecteur selon l'une quelconque des revendications 1 à 17, **caractérisé en ce que** le fragment d'anticorps est tout ou partie d'au moins une région CDR3 d'un anticorps.

20. Vecteur selon la revendication 19, **caractérisé en ce que** la région CDR3 est sélectionnée dans le groupe consistant en RTT79, NE-1, et RT72.

21. Vecteur selon l'une quelconque des revendications précédentes, **caractérisé en ce qu'**il comprend l'une des séquence suivantes : SEQ ID NO : 5; SEQ ID NO : 7; SEQ ID NO : 9; SEQ ID NO : 10; SEQ ID NO : 13; SEQ ID NO : 16 ; SEQ ID NO : 23.

22. Association d'un vecteur selon l'une quelconque des revendications 1 à 21 avec une substance d'intérêt.

23. Association selon la revendication 22, **caractérisée en ce** le vecteur et la substance d'intérêt sont conjugués.

24. Association selon la revendication 22, **caractérisée en ce que** le vecteur et la substance d'intérêt sont associés par un couplage chimique.

25. Association selon la revendication 24, **caractérisée en ce que** ladite substance d'intérêt est couplée au niveau de l'extrémité N ou C-terminale de la séquence d'acides aminés du vecteur.

26. Association selon l'une des revendications 24 ou 25, **caractérisée en ce que** le couplage est réalisé par l'intermédiaire de réactifs de pontage homo- ou hétérofonctionnels, du type 4-(N-maléimidométhyl) cyclohexane-1-carboxylate de succinimidyle (SMCC).

27. Association selon l'une des revendications 24 ou 25, **caractérisée en ce que** le couplage est réalisé par l'intermédiaire d'une molécule d'ancrage choisie parmi la protéine A, la protéine G, le fragment F(ab')₂ anti-IgG, l'IgG anti-peroxydase, la F(ab')₂ anti-RNase, la concanavaline A, la streptavidine et l'avidine.

28. Association selon l'une des revendications 24 ou 25, **caractérisée en ce que** le couplage est réalisé par génie génétique.

29. Association selon l'une quelconque des revendications 22 à 28, **caractérisée en ce que** ladite substance d'intérêt est choisie dans le groupe comprenant : un acide nucléique, une protéine, un médicament, un antigène, un anticorps.

30. Association selon la revendication 29, caracractérisée en ce que ladite substance d'intérêt est la doxorubicine.

31. Cellule eucaryote hôte, **caractérisée en ce qu'**elle comprend un vecteur selon l'une quelconque des revendications 1 à 21 ou une association selon l'une quelconque des revendication 22 à 30.

32. Procédé de transfert *in vitro* d'une substance d'intérêt à l'intérieur d'une cellule, **caractérisé en ce qu'**il comprend les étapes suivantes :
a) l'association de ladite substance d'intérêt à un vecteur selon l'une des revendications 1 à 21, et
b) l'incubation de la cellule avec ladite association à une température de culture permettant le métabolisme actif de ladite cellule.

33. Procédé selon la revendication 32, **caractérisé en ce que** l'incubation de l'étape b) s'effectue à une température comprise entre 25°C et 39°C.

34. Procédé selon la revendication 33, **caractérisé en ce que** l'incubation de l'étape b) s'effectue à 37°C.

35. Composition biologique, pharmaceutique, cosmétique, agroalimentaire, de diagnostic ou de traçage, **caractérisée en ce qu'**elle comprend comme principe actif, soit un vecteur selon l'une des revendications 1 à 21, soit une association selon l'une des revendications 22 à 30, soit des cellules selon la revendication 31.

36. Agent de diagnostic **caractérisé en ce qu'**il comprend au moins un vecteur selon l'une des revendications 1 à 21, ou une association selon l'une des revendications 22 à 30, ou une cellule selon la revendication 31.

37. Kit de diagnostic **caractérisé en ce qu'**il comprend, dans un ou plusieurs récipients, une quantité prédéterminée d'un agent selon la revendication 36.

## Claims

1. Intracytoplasmic and / or intranuclear transfer vector for a substance of interest, **characterised in that** it comprises at least one amino acid sequence reacting *in vivo* with aminoglycans and that is derived from human lipoprotein B coupled to at least one antibody fragment.

2. Vector according to claim 1, **characterised in that** the sequence of amino acids reacting with the aminoglycans and that is derived from human lipoprotein B is capable of reacting with heparin, heparane sulphate, chondroitin sulphates and their derivatives.

3. Vector according to either of claims 1 or 2, **characterised in that** the amino acid sequence reacting with the aminoglycans and that is derived from human lipoprotein B is coded by the germ line.

4. Vector according to any one of claims 1 to 3, **characterised in that** the sequence of amino acids reacting with the aminoglycans and that is derived from human lipoprotein B comprises at least 3 basic amino acids.

5. Vector according to any one of the previous claims, **characterised in that** the sequence of amino acids reacting with the aminoglycans and that is derived from human lipoprotein B is constituted of or comprises at least one group of amino acids satisfying one of the formulas a) (XBBBXXBX)ₙ; b) (XBBXBX)ₙ; c) (BBXₘYBBXₒ)ₙ; d) (XBBXXBX)ₙ; and e) (BXBB)ₙ;
in which B is a basic amino acid, X is a non-basic amino acid, Y is either a direct link or 1 to 20 amino acids, m is an integer number comprised between 0 and 5, n is an integer number comprised between 1 and 10, and o is an integer number comprised between 0 and 5.

6. Vector according to claim 5, **characterised in that** the sequence of amino acids reacting with the aminoglycans and that is derived from human lipoprotein B is constituted of or comprises a group of amino acids satisfying the formula (XBBXXBX)ₙ.

7. Vector according to any one of claims 5 or 6, **characterised in that** X is a non-basic hydrophobic amino acid.

8. Vector according to any one of the previous claims, **characterised in that** it comprises less than 100 amino acids.

9. Vector according to any one of the previous claims, **characterised in that** it comprises less than 50 amino acids.

10. Vector according to any one of the previous claims, **characterised in that** it comprises less than 25 amino acids.

11. Vector according to any one of the previous claims, **characterised in that** the sequence of amino acids reacting with the aminoglycans and that is derived from human lipoprotein B is chosen from the group comprising SEQ ID No. 1 in the monomer, dimer (SEQ ID 2), trimer (SEQ ID No. 3), polymer state.

12. Vector according to any one of the previous claims, **characterised in that** the antibody fragment is a fragment of polyreactive antibody.

13. Vector according to any one of the previous claims, **characterised in that** the antibody fragment is a hypervariable region of an antibody.

14. Vector according to any one of the previous claims, **characterised in that** the antibody fragment is a fragment of the heavy chain of an antibody.

15. Vector according to any one of the previous claims, **characterised in that** the antibody fragment is a fragment of human anti-DNA antibody.

16. Vector according to any one of claims 1 to 15, **characterised in that** the antibody fragment is an IgM fragment.

17. Vector according to any one of claims 1 to 15, **characterised in that** the antibody fragment is an IgG fragment.

18. Vector according to any one of the previous claims, **characterised in that** the antibody fragment is all or part of at least one CDR2 region of an antibody.

19. Vector according to any one of the claims 1 to 17, **characterised in that** the antibody fragment is all or part of at least one CDR3 region of an antibody.

20. Vector according to claim 19, **characterised in that** the region CDR3 is selected from the group consisting of RTT79, NE-1 and RT72.

21. Vector according to any one of the previous claims, **characterised in that** it comprises one of the sequences SEQ ID No. 5; SEQ ID No. 7; SEQ ID No. 9; SEQ ID No. 10; SEQ ID No. 13: SEQ ID No. 16; SEQ ID No. 23.

22. Association of a vector according to any one of claims 1 to 21 with a substance of interest.

23. Association according to claim 22, **characterised in that** the vector and the substance of interest are conjugated.

24. Association according to claim 22, **characterised in that** the vector and the substance of interest are associated by chemical coupling.

25. Association according to claim 24, **characterised in that** the said substance of interest is coupled at the N or C-terminal end of the sequence of amino acids in the vector.

26. Association according to any one of claims 24 or 25, **characterised in that** the coupling is made by means of homo or heterofunctional bridging reagents of the succinimidyl 4-(N-maleimidomethyl) cyclohexane-1-carboxylate (SMCC) type.

27. Association according to any one of claims 24 or 25, **characterised in that** the coupling is made by means of an anchor molecule chosen from among protein A, protein G, fragment F(ab')₂ anti-IgG, anti-peroxydase IgG, F(ab')₂ anti-RNase, concanavalin A, streptavidin and avidin.

28. Association according to either of claims 24 or 25, **characterised in that** coupling is made by generic engineering.

29. Association according to any one of claims 22 to 28, **characterised in that** the said substance of interest is chosen from the group comprising a nucleic acid, a protein, a medicine, an antigen and an antibody.

30. Association according to claim 29, **characterized in that** the said substance of interest is doxorubicin.

31. Eukaryote host cell **characterised in that** it comprises a vector according to any one of claims 1 to 21 or an association according to any one of claims 22 to 30.

32. Process for in vitro transfer of a substance of interest inside a cell, **characterised in that** it includes the following steps:
a) association of the said substance of interest with a vector according to one of claims 1 to 21, and
b) incubation of the cell with the said association at a culture temperature enabling active metabolism of the said cell.

33. Process according to claim 32, **characterised in that** incubation in step b) is done at a temperature between 25°C and 39°C.

34. Process according to claim 33, **characterised in that** incubation in step b) is done at 37°C.

35. Biological, pharmaceutical, cosmetic, food processing, diagnostic or tracing composition, **characterised in that** its active constituent is either a vector according to one of claims 1 to 21, or an association according to one of claims 22 to 30, or cells according to claim 30.

36. Diagnostic agent **characterised in that** it comprises at least one vector according to one of claims 1 to 21, or an association according to one of claims 22 to 30, or a cell according to claim 31.

37. Diagnostic kit **characterised in that** it comprises a predetermined quantity of an agent according to claim 36, in one or several receptacles.

## Patentansprüche

1. Vektor für den intrazytoplasmischen und/oder intranukleären Transfer einer interessierenden Substanz, **dadurch gekennzeichnet, dass** er mindestens eine *in vivo* mit den Aminoglykanen reagierende und aus dem Human-Lipoprotein B hervorgegangene Aminosäurensequenz aufweist, die mindestens an ein Antikörperfragment gekoppelt ist.

2. Vektor nach Anspruch 1, **dadurch gekennzeichnet, dass** die mit den Aminoglykanen reagierende und aus dem Human-Lipoprotein B hervorgegangene Aminosäurensequenz mit Heparin, Heparansulfat, Chondroitinsulfaten und ihren Derivaten reagieren kann.

3. Vektor nach Anspruch 1 oder 2, **dadurch gekennzeichnet, dass** die mit den Aminoglykanen reagierende und aus dem Human-Lipoprotein B hervorgegangene Aminosäurensequenz von der Keimlinie kodiert wird.

4. Vektor nach einem der Ansprüche 1 bis 3, **dadurch gekennzeichnet, dass** die mit den Aminoglykanen reagierende und aus dem Human-Lipoprotein B hervorgegangene Aminosäurensequenz eine Anzahl von basischen Aminosäuren von mindestens gleich 3 aufweist.

5. Vektor nach einem der vorhergehenden Ansprüche, **dadurch gekennzeichnet, dass** die mit den Aminoglykanen reagierende und aus dem Human-Lipoprotein B hervorgegangene Aminosäurensequenz von mindestens einer Aminosäurengruppe gebildet wird oder mindestens eine Aminosäurengruppe umfasst, die einer der folgenden Formeln a) (XBBBXXBX)ₙ, b) (XBBXBX)ₙ, c) (BBXₘYBBXₒ)ₙ, d) (XBBXXBX)ₙ oder e) (BXBB)ₙ entspricht,
wobei B eine basische Aminosäure ist, X eine nicht basische Aminosäure ist, Y entweder eine direkte Bindung oder 1 bis 20 Aminosäuren darstellt, m eine ganze Zahl von 0 bis 5 ist, n eine ganze Zahl von 1 bis 10 ist und o eine ganze Zahl von 0 bis 5 ist.

6. Vektor nach Anspruch 5, **dadurch gekennzeichnet, dass** die mit den Aminoglykanen reagierende und aus dem Human-Lipoprotein B hervorgegangene Aminosäurensequenz von einer Aminosäurengruppe gebildet wird oder eine Aminosäurengruppe umfasst, die der Formel (XBBXXBX)ₙ entspricht.

7. Vektor nach Anspruch 5 oder 6, **dadurch gekennzeichnet, dass** X eine hydrophobe, nicht basische Aminosäure ist.

8. Vektor nach einem der vorhergehenden Ansprüche, **dadurch gekennzeichnet, dass** er weniger als 100 Aminosäuren aufweist.

9. Vektor nach einem der vorhergehenden Ansprüche, **dadurch gekennzeichnet, dass** er weniger als 50 Aminosäuren aufweist.

10. Vektor nach einem der vorhergehenden Ansprüche, **dadurch gekennzeichnet, dass** er weniger als 25 Aminosäuren aufweist.

11. Vektor nach einem der vorhergehenden Ansprüche, **dadurch gekennzeichnet, dass** die mit den Aminoglykanen reagierende und aus dem Human-Lipoprotein B hervorgegangene Aminosäurensequenz aus der Gruppe ausgewählt wurde, die SEQ ID NO.1 im Monomer-, Dimer-(SEQ ID.2), Trimer- (SEQ ID NO.3), Polymerzustand aufweist.

12. Vektor nach einem der vorhergehenden Ansprüche, **dadurch gekennzeichnet, dass** das Antikörperfragment ein polyreaktives Antikörperfragment ist.

13. Vektor nach einem der vorhergehenden Ansprüche, **dadurch gekennzeichnet, dass** das Antikörperfragment eine hypervariable Region eines Antikörpers ist.

14. Vektor nach einem der vorhergehenden Ansprüche, **dadurch gekennzeichnet, dass** das Antikörperfragment ein Fragment der schweren Kette eines Antikörpers ist.

15. Vektor nach einem der vorhergehenden Ansprüche, **dadurch gekennzeichnet, dass** das Antikörperfragment ein Fragment eines humanen Anti-DNA-Antikörpers ist.

16. Vektor nach einem der Ansprüche 1 bis 15, **dadurch gekennzeichnet, dass** das Antikörperfragment ein IgM-Fragment ist.

17. Vektor nach einem der Ansprüche 1 bis 15, **dadurch gekennzeichnet dass** das Antikörperfragment ein IgG-Fragment ist.

18. Vektor nach einem der vorhergehenden Ansprüche, **dadurch gekennzeichnet, dass** das Antikörperfragment ganz oder teilweise von mindestens einer CDR2-Region eines Antikörpers ist.

19. Vektor nach einem der Ansprüche 1 bis 17, **dadurch gekennzeichnet, dass** das Antikörperfragment ganz oder teilweise von mindestens einet CDR3-Region eines Antikörpers ist.

20. Vektor nach Anspruch 19, **dadurch gekennzeichnet, dass** die Region CDR3 aus der Gruppe ausgewählt wird, die aus RTT79, NE-1 und RT72 besteht.

21. Vektor nach einem der vorhergehenden Ansprüche, **dadurch gekennzeichnet, dass** er eine der folgenden Sequenzen SEQ ID NO : 5, SEQ ID NO : 7, SEQ ID NO : 9, SEQ ID NO : 10, SEQ ID NO : 13, SEQ ID NO : 16, SEQ ID NO : 23 aufweist.

22. Assoziation eines Vektors nach einem der vorhergehenden Ansprüche 1 bis 21 mit einer interessierenden Substanz.

23. Assoziation nach Anspruch 22, **dadurch gekennzeichnet, dass** der Vektor und die interessierende Substanz konjugiert sind.

24. Assoziation nach Anspruch 22, **dadurch gekennzeichnet, dass** der Vektor und die interessierende Substand durch eine chemische Kopplung assoziiert sind.

25. Assoziation nach Anspruch 24, **dadurch gekennzeichnet, dass** diese interessierende Substanz auf Ebene des Endes N oder der C-Terminale der Aminosäurensequenz des Vektors gekoppelt ist.

26. Assoziation nach Anspruch 24 oder 25, **dadurch gekennzeichnet, dass** die Kopplung über homo- oder heterofunktionale Überbrückungsreagenzien vom Typ Succinimidyl-4-(N-maleidomethyl)-cyclohexan-1-carboxylat (SMCC) durchgeführt wird.

27. Assoziation nach Anspruch 24 oder 25, **dadurch gekennzeichnet, dass** die Kopplung über ein Ankermolekül durchgeführt wird, das aus dem Protein A, dem Protein G, dem Fragment F(ab')₂ Anti-IgG, der IgG Antiperoxydase, der F(ab')₂ Anti-RNase, dem Concanavalin A, dem Streptavidin und dem Avidin ausgewählt wird.

28. Assoziation nach Anspruch 24 oder 25, **dadurch gekennzeichnet, dass** die Kopplung gentechnisch durchgeführt wird.

29. Assoziation nach einem der Ansprüche 22 bis 28, **dadurch gekennzeichnet, dass** diese interessierende Substanz aus der Gruppe ausgewählt wird, die eine Nukleinsäure, ein Protein, ein Medikament, ein Antigen, einen Antikörper aufweist.

30. Assoziation nach Anspruch 29, **dadurch gekennzeichnet, dass** diese interessierende Substanz ist doxorubicin.

31. Eucaryotische Wirtszelle, **dadurch gekennzeichnet, dass** sie einen Vektor nach einem der Ansprüche 1 bis 21 oder eine Assoziation nach einem der Ansprüche 22 bis 30 aufweist.

32. Verfahren zum *in vitro*-Transfer einer interessierenden Substanz in eine Zelle, **dadurch gekennzeichnet, dass** es die folgenden Schritte umfasst:
a) die Assoziation dieser interessierenden Substanz an einen Vektor nach einem der Ansprüche 1 bis 21, und
b) die Inkubation der Zelle mit dieser Assoziation bei einer Kulturtemperatur, die den aktiven Stoffwechsel dieser Zelle ermöglicht.

33. Verfahren nach Anspruch 32, **dadurch gekennzeichnet, dass** die Inkubation von Schritt b) bei einer Temperatur von 25 °C bis 39 °C erfolgt.

34. Verfahren nach Anspruch 33, **dadurch gekennzeichnet, dass** die Inkubation von Schritt b) bei 37 °C erfolgt.

35. Biologische, pharmazeutische, kosmetische, Lebensmittel-, diagnostische oder Markierungsverbindung, **dadurch gekennzeichnet, dass** sie als Wirkprinzip entweder einen Vektor nach einem der Ansprüche 1 bis 21 oder eine Assoziation nach einem der Ansprüche 22 bis 30 oder Zellen nach Anspruch 30 aufweist.

36. Diagnosemittel, **dadurch gekennzeichnet, dass** es mindestens einen Vektor nach einem der Ansprüche 1 bis 21 oder eine Assoziation nach einem der Ansprüche 22 bis 30 oder eine Zelle nach Anspruch 31 aufweist.

37. Diagnosekit, **dadurch gekennzeichnet, dass** es in einem oder mehreren Behältern eine vorbestimmte Menge eines Mittels nach Anspruch 36 aufweist.


### SEQUENCE LISTING

<110> DIATOS S.A.
<120> SÉQUENCES D'ACIDES AMINÉS FACILITANT LA PÉNÉTRATION D'UNE SUBSTANCE D'INTÉRÊT À L'INTÉREIUR DES CELLULES ET/OU DES NOYAUX C ELLULAIRES.
<130> 12052-PCT-DIATOS
<140> PCT/FR00/02621
   <141> 2001-03-01
<150> FR00/02621
   <151> 2000-03-01
<160> 48
<170> PatentIn version 3.0
<210> 1
   <211> 7
   <212> PRT
   <213> Homo sapiens
<400> 1
   Val Lys Arg Gly Leu Lys Leu
   1               5
<210> 2
   <211> 14
   <212> PRT
   <213> Homo sapiens
<400> 2
   Val Lys Arg Gly Leu Lys Leu Val Lys Arg Gly Leu Lys Leu
   1 5 10
<210> 3
   <211> 21
   <212> PRT
   <213> Homo sapiens
<400> 3
   Val Lys Arg Gly Leu Lys Leu Val Lys Arg Gly Leu Lys Leu Val Lys
   1 5 10 15
   Arg Gly Leu Lys Leu
   20
<210> 4
   <211> 11
   <212> PRT
   <213> Mus musculus x Rattus norvegicus
<400> 4
   Arg Gln Lys Tyr Asn Lys Arg Ala Met Asp Tyr
   1 5 10
<210> 5
   <211> 18
   <212> PRT
   <213> Homo sapiens-Mus musculus-Rattus Norvegicus
<400> 5
   Val Lys Arg Gly Leu Lys Leu Arg Gln Lys Tyr Asn Lys Arg Ala Met
   1 5 10 15
   Asp Tyr
<210> 6
   <211> 20
   <212> PRT
   <213> Mus musculus x Rattus norvegicus
<400> 6
   Thr Tyr Tyr Ser Asp Thr Val Lys Gly Arg Phe Thr Arg Gln Lys Tyr
   1 5 10 15
   Asn Lys Arg Ala
   20
<210> 7
   <211> 27
   <212> PRT
   <213> Homo sapiens-Mus musculus-Rattus Norvegicus
<400> 7
   Val Lys Arg Gly Leu Lys Leu Thr Tyr Tyr Ser Asp Thr Val Lys Gly
   1 5 10 15
   Arg Phe Thr Arg Gln Lys Tyr Asn Lys Arg Ala
   20 25
<210> 8
   <211> 17
   <212> PRT
   <213> Homo sapiens
<400> 8
   Val Arg Arg Ser Gly Arg Val Val Val Pro Ala Ala Pro Arg Asn Arg
   1 5 10 15
   Asp
<210> 9
   <211> 24
   <212> PRT
   <213> Homo sapiens
<400> 9
   Val Lys Arg Gly Leu Lys Leu Val Arg Arg Ser Gly Arg Val Val Val
   1 5 10 15
   Pro Ala Ala Pro Arg Asn Arg Asp
   20
<210> 10
   <211> 19
   <212> PRT
   <213> Homo sapiens
<400> 10
   Val Lys Arg Gly Leu Lys Leu Arg His Val Arg Pro Arg Val Thr Arg
   1 5 10 15
   Met Asp Val
<210> 11
   <211> 19
   <212> PRT
   <213> Homo sapiens
<400> 11
   Val Lys Arg Gly Leu Lys Leu Gly Tyr Tyr Asp Phe Trp Ser Gly Pro
   1 5 10 15
   Gly Lys Asn
<210> 12
   <211> 27
   <212> PRT
   <213> Mus musculus x Rattus norvegicus
<400> 12
   Asn Val Lys Lys Pro Lys Leu Thr Tyr Tyr Ser Asp Thr Val Lys Gly
   1 5 10 15
   Arg Phe Thr Arg Gln Lys Tyr Asn Lys Arg Ala
   20 25
<210> 13
   <211> 31
   <212> PRT
   <213> Homo sapiens
<400> 13
   Val Lys Arg Gly Leu Lys Leu Ser Gly Ser Thr Asn Tyr Asn Pro Ser
   1 5 10 15
   Leu Lys Ser Arg His Val Arg Pro Arg Val Thr Arg Met Asp Val
   20 25 30
<210> 14
   <211> 28
   <212> PRT
   <213> Mus musculus x Rattus norvegicus
<400> 14
   Arg Gln Lys Tyr Asn Lys Arg Ala Thr Tyr Tyr Ser Asp Thr Val Lys
   1 5 10 15
   Gly Arg Phe Thr Arg Gln Lys Tyr Asn Lys Arg Ala
   20 25
<210> 15
   <211> 24
   <212> PRT
   <213> Homo sapiens
<400> 15
   Arg His Val Arg Pro Arg Val Thr Arg Met Asp Val Arg His Val Arg
   1 5 10 15
   Pro Arg Val Thr Arg Met Asp Val
   20
<210> 16
   <211> 31
   <212> PRT
   <213> Homo sapiens
<400> 16
   Arg His Val Arg Pro Arg Val Thr Arg Met Asp Val Val Lys Arg Gly
   1 5 10 15
   Leu Lys Leu Arg His Val Arg Pro Arg Val Thr Arg Met Asp Val
   20 25 30
<210> 17
   <211> 8
   <212> PRT
   <213> Homo sapiens
<400> 17
   Leu Arg Lys Arg Leu Leu Arg Asp
   1 5
<210> 18
   <211> 4
   <212> PRT
   <213> Homo sapiens
<400> 18
   Lys Ser Arg Lys
   1
<210> 19
   <211> 8
   <212> PRT
   <213> Homo sapiens
<400> 19
   Lys Ser Arg Lys Lys Ser Arg Lys
   1 5
<210> 20
   <211> 12
   <212> PRT
   <213> Homo sapiens
<400> 20
   Arg Lys Gly Phe Tyr Lys Arg Lys Gln Cys Lys Pro
   1 5 10
<210> 21
   <211> 17
   <212> PRT
   <213> Homo sapiens
<400> 21
   Gly Arg Pro Arg Glu Ser Gly Lys Lys Arg Lys Arg Lys Arg Leu Lys
   1 5 10 15
   Pro
<210> 22
   <211> 32
   <212> PRT
   <213> Mus musculus x Rattus norvegicus
<400> 22
   Lys Lys Lys Lys Lys Lys Lys Lys Lys Lys Lys Lys Thr Tyr Tyr Ser
   1 5 10 15
   Asp Thr Val Lys Gly Arg Phe Thr Arg Gln Lys Tyr Asn Lys Arg Ala
   20 25 30
<210> 23
   <211> 30
   <212> PRT
   <213> Mus musculus x Rattus norvegicus
<400> 23
   Lys Lys Lys Lys Lys Lys Lys Lys Lys Lys Lys Lys Val Lys Arg Gly
   1 5 10 15
   Leu Lys Leu Arg Gln Lys Tyr Asn Lys Arg Ala Met Asp Tyr
   20 25 30
<210> 24
   <211> 14
   <212> PRT
   <213> Peptide synthétique
<400> 24
   Lys Leu Ala Lys Leu Ala Lys Lys Leu Ala Lys Leu Ala Lys
   1 5 10
<210> 25
   <211> 18
   <212> PRT
   <213> Homo sapiens
<400> 25
   Arg Lys Gly Lys Phe Tyr Lys Arg Lys Gln Cys Lys Pro Ser Arg Gly
   1 5 10 15
   Arg Lys
<210> 26
   <211> 18
   <212> PRT
   <213> Homo sapiens
<400> 26
   Ser Glu Arg Lys Lys Arg Arg Arg Glu Ser Arg Lys Lys Arg Arg Arg
   1 5 10 15
   Glu Ser
<210> 27
   <211> 18
   <212> PRT
   <213> Homo sapiens
<220>
   <221> misc_feature
   <222> () .. ()
   <223> Tous les acides aminés sont en configuration D
<400> 27
   Ser Glu Arg Lys Lys Arg Arg Arg Glu Ser Arg Lys Lys Arg Arg Arg
   1 5 10 15
   Glu Ser
<210> 28
   <211> 16
   <212> PRT
   <213> Homo sapiens
<400> 28
   Arg Lys Lys Arg Arg Arg Gly Asp Arg Lys Lys Arg Arg Arg Gly Asp
   1 5 10 15
<210> 29
   <211> 15
   <212> PRT
   <213> Homo sapiens
<400> 29
   Val Lys Arg Gly Leu Lys Leu Leu Arg Lys Arg Leu Leu Arg Asp
   1 5 10 15
<210> 30
   <211> 15
   <212> PRT
   <213> Homo sapiens
<400> 30
   Gly Lys Arg Lys Lys Lys Gly Lys Leu Gly Lys Lys Arg Asp Pro
   1 5 10 15
<210> 31
   <211> 16
   <212> PRT
   <213> Homo sapiens
<220>
   <221> misc_feature
   <222> () .. ()
   <223> Les acides aminés sont en position recto verso
<400> 31
   Pro Asp Arg Lys Lys Gly Leu Lys Gly Lys Lys Lys Arg Lys Lys Gly
   1 5 10 15
<210> 32
   <211> 14
   <212> PRT
   <213> Homo sapiens
<220>
   <221> misc_feature
   <222> ( ) .. ( )
   <223> Tous les acides aminés sont en configuration D.
<400> 32
   Gly Lys Arg Lys Lys Lys Gly Lys Leu Gly Lys Lys Arg Asp
   1               5                   10
<210> 33
   <211> 13
   <212> PRT
   <213> Homo sapiens
<400> 33
   Gly Leu Lys Lys Asn Gly Ser Cys Lys Arg Gly Pro Arg
   1               5                   10
<210> 34
   <211> 15
   <212> PRT
   <213> Homo sapiens
<400> 34
   Tyr Arg Ser Arg Lys Tyr Ser Ser Trp Tyr Tyr Ala Leu Lys Arg
   1               5                   10                  15
<210> 35
   <211> 14
   <212> PRT
   <213> Homo sapiens
<400> 35
   Ser Arg Arg Ala Arg Arg Ser Pro Arg His Leu Gly Ser Gly
   1               5                   10
<210> 36
   <211> 10
   <212> PRT
   <213> Homo sapiens
<400> 36
   Ala Lys Thr Gly Lys Arg Lys Arg Ser Gly
   1               5                   10
<210> 37
   <211> 13
   <212> PRT
   <213> Homo sapiens
<400> 37
   Gln Gly Lys Ser Lys Arg Glu Lys Lys Asp Arg Val Phe
   1 5 10
<210> 38
   <211> 12
   <212> PRT
   <213> Homo sapiens
<400> 38
   Lys His Leu Lys Lys His Leu Lys Lys His Leu Lys
   1 5 10
<210> 39
   <211> 17
   <212> PRT
   <213> Homo sapiens
<400> 39
   Leu Arg Arg Glu Arg Gln Ser Arg Leu Arg Arg Glu Arg Ser Gln Ser
   1 5 10 15
   Arg
<210> 40
   <211> 32
   <212> PRT
   <213> Homo sapiens
<400> 40
   Lys Lys Lys Lys Lys Lys Lys Lys Lys Lys Lys Lys Lys Val Lys Arg
   1 5 10 15
   Gly Leu Lys Leu Arg His Val Arg Pro Arg Val Thr Arg Met Asp Val
   20 25 30
<210> 41
   <211> 31
   <212> PRT
   <213> Homo sapiens
<400> 41
   Lys Lys Lys Lys Lys Lys Lys Lys Lys Lys Lys Lys Lys Ser Glu Arg
   1 5 10 15
   Lys Lys Arg Arg Arg Glu Ser Arg Lys Lys Arg Arg Arg Glu Ser
   20 25 30
<210> 42
   <211> 27
   <212> PRT
   <213> Homo sapiens
<400> 42
   Lys Lys Lys Lys Lys Lys Lys Lys Lys Lys Lys Lys Lys Ser Arg Arg
   1 5 10 15
   Ala Arg Arg Ser Pro Arg His Leu Gly Ser Gly
   20 25
<210> 43
   <211> 21
   <212> PRT
   <213> peptide synthétique
<400> 43
   Val Lys Arg Gly Leu Lys Leu Lys Leu Ala Lys Leu Ala Lys Lys Leu
   1 5 10 15
   Ala Lys Leu Ala Lys
   20
<210> 44
   <211> 29
   <212> PRT
   <213> Peptide synthétique
<400> 44
   Lys Leu Ala Lys Leu Ala Lys Lys Leu Ala Lys Leu Ala Lys Gly Lys
   1 5 10 15
   Arg Lys Lys Lys Gly Lys Leu Gly Lys Lys Arg Asp Pro
   20 25
<210> 45
   <211> 26
   <212> PRT
   <213> Peptide synthétique
<400> 45
   Lys Leu Ala Lys Leu Ala Lys Lys Leu Ala Lys Leu Ala Lys Lys His
   1 5 10 15
   Leu Lys Lys His Leu Lys Lys His Leu Lys
   20 25
<210> 46
   <211> 18
   <212> PRT
   <213> Homo sapiens
<220>
   <221> misc_feature
   <222> () .. ()
   <223> Résidu de glycine-phtaloyl en position N-terminal (X).
<400> 46
   Xaa Gly Arg Lys Lys Arg Arg Arg Glu Ser Arg Lys Lys Arg Arg Arg
   1 5 10 15
   Glu Ser
<210> 47
   <211> 18
   <212> PRT
   <213> Homo sapiens
<220>
   <221> misc_feature
   <222 > ( ) .. ( )
   <223> Présente un motif salicylyl (nommé X) en position N-termin al.
<400> 47
   Xaa Gly Arg Pro Arg Glu Ser Gly Lys Lys Arg Lys Arg Lys Arg Leu
   1 5 10 15
   Lys Pro
<210> 48
   <211> 19
   <212> PRT
   <213> Homo sapiens
<220>
   <221> misc_feature
   <222> ( ) .. ( )
   <223> Présente un motif salicylyl (nommé X) en position C-termin al.
<400> 48
   Gly Arg Pro Arg Glu Ser Gly Lys Lys Arg Lys Arg Lys Arg Leu Lys
   1 5 10 15
   Xaa Pro Gly